(19) Europäisches Patentamt
European Patent Office
Office européen des brevets

(11) EP 4 252 843 A1

(12) **EUROPEAN PATENT APPLICATION**
published in accordance with Art. 153(4) EPC

(43) Date of publication:
**04.10.2023 Bulletin 2023/40**

(21) Application number: **21898159.5**

(22) Date of filing: **29.11.2021**

(51) International Patent Classification (IPC):
*A61P 1/04* (2006.01) *A61P 3/10* (2006.01)
*A61P 9/04* (2006.01) *A61P 21/00* (2006.01)
*A61P 43/00* (2006.01) *C07D 211/90* (2006.01)
*C07D 471/04* (2006.01) *C07D 213/80* (2006.01)
*C07D 213/82* (2006.01) *A61K 31/4418* (2006.01)
*A61K 31/4422* (2006.01) *A61K 31/4745* (2006.01)

(52) Cooperative Patent Classification (CPC):
**A61K 31/4418; A61K 31/4422; A61K 31/4745; A61P 1/04; A61P 3/10; A61P 9/04; A61P 21/00; A61P 43/00; C07D 211/90; C07D 213/80; C07D 213/82; C07D 471/04**

(86) International application number:
**PCT/JP2021/043654**

(87) International publication number:
**WO 2022/114187 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.11.2020 JP 2020198084**

(71) Applicant: **Kyushu University, National University Corporation**
**Nishi-ku**
**Fukuoka-shi**
**Fukuoka 819-0395 (JP)**

(72) Inventors:
- **KAWANISHI, Eiji**
  **Fukuoka-shi, Fukuoka 819-0395 (JP)**
- **OJIDA, Akio**
  **Fukuoka-shi, Fukuoka 819-0395 (JP)**
- **NISHIDA, Motohiro**
  **Fukuoka-shi, Fukuoka 819-0395 (JP)**
- **KATO, Yuri**
  **Fukuoka-shi, Fukuoka 819-0395 (JP)**

(74) Representative: **Vossius & Partner**
**Patentanwälte Rechtsanwälte mbB**
**Siebertstraße 3**
**81675 München (DE)**

(54) **COMPOUND HAVING INHIBITORY ACTION AGAINST EXCESSIVE MITOCHONDRIAL DIVISIONS**

(57) The present invention provides a compound for treating or preventing diseases caused by mitochondrial hyperfission.

The present invention provides a compound represented by formula (1):

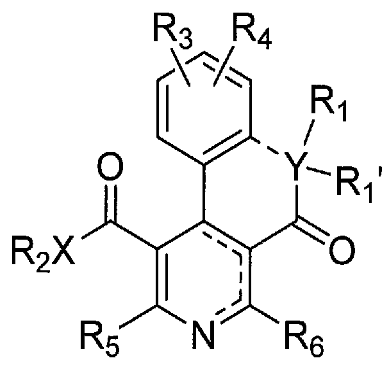

**(1)**

wherein
$R_1$ and $R_1$' are each independently hydrogen, an optionally substituted lower alkyl, an optionally substituted lower cycloalkyl, and the like; $R_2$ is an optionally substituted lower alkyl, an optionally substituted lower cycloalkyl, and the like; $R_3$ and $R_4$ are each independently hydrogen, halogen, hydroxy, nitro, cyano, an optionally substituted lower alkyl, and the like; $R_5$ and $R_6$ are each independently an optionally substituted lower alkyl; an optionally substituted lower cycloalkyl, and the like; X is nitrogen or oxygen; Y is carbon, nitrogen or oxygen; and a broken line represents the presence or absence of a bond;
or a pharmaceutically acceptable salt, a solvate, or a prodrug thereof.

## Description

TECHNICAL FIELD

In general, the present invention relates to novel compounds with an inhibitory effect on mitochondrial hyperfission. Specifically, the present invention relates to compounds that exhibit the inhibitory effect on mitochondrial hyperfission through inhibition of dynamin-related protein 1 (Drp1)-filamin complex formation, and pharmaceutical compositions comprising the same.

BACKGROUND TECHNOLOGY

Mitochondria are organelles found in almost all eukaryotic cells, and take principally part in ATP generation as a result of oxidative phosphorylation that occurs in electron transport system. Mitochondria are dynamic organelles that change their morphology and/or structure by repeating division and fusion (Non-Patent Document 1). Mitochondria in healthy mature cardiomyocytes are static, and rarely divide or fuse. However, once mitochondria are in morbid state, they undergo violent division or fusion. Similar to neurodegenerative diseases, dysfunction of mitochondrial dynamics is thought to cause the onset and progression of cardiovascular diseases (Non-Patent Document 2). In addition, abnormal mitochondrial division or fusion is known to be involved in onset of cancers, cardiovascular diseases, neurodegenerative diseases, and the like (Non-Patent Document 3). For example, it has been suggested that abnormal mitochondrial division or fusion induces abnormality of energy metabolism in the infarct focus after myocardial infarction, which causes chronic heart failure (Non-Patent Document 4).

Aberrant dynamics of mitochondria due to abnormal interaction between mitochondria and an actin cytoskeleton has attracted public attention as a critical determinant of cardiac vulnerability after myocardial infarction. Mitochondrial division is induced by activation of Dynamin-Related Protein 1 (Drp1), which is a GTP-binding protein. Upon hypoxic stimulation of cardiomyocytes to mimic myocardial infarction, Drp1 in the cells combines Filamin to form a Drp1-filamin complex, further forms a Drp1-filamin-actin complex to activate Drp1, and promotes mitochondrial division (Non-Patent Document 5).

Cilnidipine, a dihydropyridine-based calcium antagonist represented by formula (A1):

[Chem. 1]

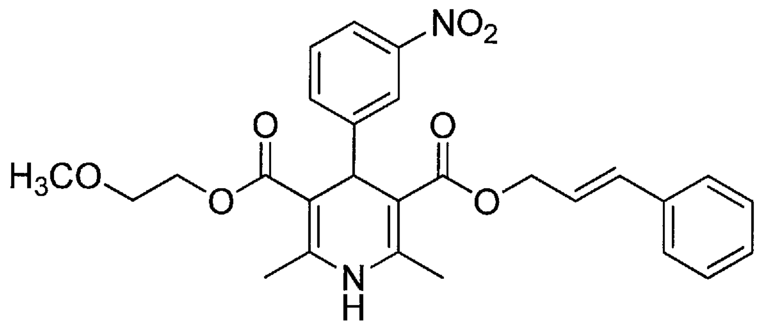

(A1)

, has been shown to inhibit formation of the Drp1-filamin complex as a mitochondria-related action (Non-Patent Document 6). Cilnidipine was demonstrated to inhibit the Drp1 complex formation, and a pharmaceutical composition comprising cilnidipine and so on, as an active ingredient, has been disclosed, which is useful for the treatment or prevention of diseases caused by mitochondrial hyperfission, such as heart failure and diabetic complications (Patent Document 1). Further, an inhibitor of a Drp1-filamin complex formation comprising a cilnidipine derivative as an active ingredient has been disclosed (Patent Document 2).

Currently, cilnidipine is widely prescribed as an antihypertension drug since it functions to block the L-type calcium channel and the N-type calcium channel. Cilnidipine is featured by its longer lastingness of the antihypertension activity as compared with other antihypertension drugs, and therefore, cilnidipine is valuable as a therapeutic agent for cardiovascular diseases including heart failure, arrhythmia, and the like (Patent Document 3, 4 and 5). It has also been reported that cilnidipine is useful for the treatment or prevention of cerebral infarction and cerebral hemorrhage in addition to the commonly used antihypertensive treatment (e.g., Patent Document 6) and for the treatment or prevention of renal dysfunction (Patent Document 7), aiming at its calcium antagonistic action. Furthermore, cilnidipine has also been reported to have an effect of reducing the side effects of anticancer drugs (Patent Document 8).

CITATION LIST

PATENT DOCUMENTS

Patent Document 1: International Publication No. WO2016/080516
Patent Document 2: International Publication No. WO2020/241638
Patent Document 3: Japanese Patent No. 4613496
Patent Document 4: Japanese Patent Publication (Kokai) JP-A-2008-044871
Patent Document 5: International Publication No. WO2013/147137
Patent Document 6: Japanese Patent Publication (Kokai) JP-A-2004-359675
Patent Document 7: International Publication No. WO2008/016171
Patent Document 8: Japanese Patent Publication (Kokai) JP-A-2013-014549

Non-PATENT DOCUMENTS

Non-Patent Document 1: Song, M. & Dorn, G.W. 2nd. (2015) Mitoconfusion: Noncanonical functioning of dynamism factors in static mitochondria of the heart. Cell Metab., 21, 195-205.
Non-Patent Document 2: Dorn, G.W. 2nd. (2016) Mitochondrial fission/fusion and cardiomyopathy. Curr. Opin. Genet. Dev., 38, 38-44.
Non-Patent Document 3: Hagir B Suliman, Claude A Piantadosi. Mitochondrial Quality Control as a Therapeutic Target. Pharmacol Rev. 2016 Jan; 68(1): 20-48. doi: 10.1124/pr.115.011502.
Non-Patent Document 4: Picca A, Mankowski RT, Burman JL, Donisi L, Kim JS, Marzetti E, Leeuwenburgh C. Mitochondrial quality control mechanisms as molecular targets in cardiac ageing. Nat Rev Cardiol. 2018 Sep; 15(9):543-554. doi: 10.1038/s41569-018-0059-z.
Non-Patent Document 5: Akiyuki Nishimura 1, Motohiro Nishida, Seikagaku, Vol. 91, No. 4, pp. 519-522 (2019)
Non-Patent Document 6: Nishimura A., et al., Hypoxia-induced interaction of filamin with Drp1 causes mitochondrial hyperfission-associated myocardial aging, Sci. Signal., 11 (556), eaat5185, 2018.

SUMMARY OF THE INVENTION

PROBLEMS TO BE SOLVED BY THE INVENTION

In general, it is important that a drug candidate compound has one main action and no other action, in view of the common process for drug development in which the efficacy of a drug candidate compound is confirmed and the adverse events thereof are identified. As described above, cilnidipine that has been found to exhibit the inhibitory effect on the formation of Drp1-filamin complex, has a calcium channel inhibitory effect, and this effect renders it approved as a drug agent for an antihypertension drug. Some of the derivatives of cilnidipine described in Patent Documents 1 and 2 have not only an inhibitory effect on the formation of Drp1-filamin complex, but also a calcium channel inhibitory effect.

MEANS TO SOLVE PROBLEMS

As a result of intensive studies on the cilnidipine derivatives, the present inventors succeeded in separating the inhibitory effect on the formation of the Drp1-filamin complex from the calcium channel inhibitory effect, so as to accomplish the present invention. Specifically, the present inventors synthesized various derivatives of cilnidipine by modifying the chemical structure thereof, and discovered specific compounds which attenuate the calcium channel inhibitory effect, and which have a potent inhibitory effect on mitochondrial hyperfission based on inhibitory effect on the formation of Drp1-filamin complex.

Accordingly, the present invention encompasses the following aspects:

<Compound>

[1] A compound represented by formula (1):

[Chem. 2]

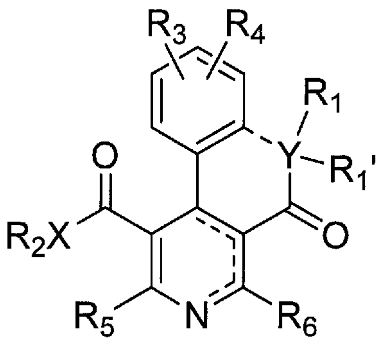

**(1)**

wherein

$R_1$ and $R_1$' are each independently hydrogen, an optionally substituted lower alkyl, an optionally substituted lower cycloalkyl, an optionally substituted lower heterocycloalkyl, an optionally substituted aryl, an optionally substituted arylalkyl, an optionally substituted heteroaryl, an optionally substituted heteroarylalkyl, an optionally substituted carbonyl, an optionally substituted carboxamide, or a covalent substituent;
$R_2$ is an optionally substituted lower alkyl, an optionally substituted lower cycloalkyl, an optionally substituted lower heterocycloalkyl, an optionally substituted aryl, an optionally substituted arylalkyl, an optionally substituted heteroaryl, an optionally substituted heteroarylalkyl, an optionally substituted carboxamide, an optionally substituted carbonyl, or a covalent substituent;
$R_3$ and $R_4$ are each independently hydrogen, halogen, hydroxy, nitro, cyano, an optionally substituted lower alkyl, an optionally substituted lower cycloalkyl, an optionally substituted lower heterocycloalkyl, an optionally substituted aryl, an optionally substituted arylalkyl, an optionally substituted heteroaryl, an optionally substituted heteroaryl, an optionally substituted heteroarylalkyl, an optionally substituted amino, an optionally substituted lower alkoxy, an optionally substituted lower alkylthio, an optionally substituted carbonyl, an optionally substituted carboxamide, deuterium, or a covalent substituent;
$R_5$ and $R_6$ are each independently an optionally substituted lower alkyl, an optionally substituted lower cycloalkyl, an optionally substituted lower heterocycloalkyl, an optionally substituted aryl, an optionally substituted arylalkyl, an optionally substituted heteroaryl, an optionally substituted heteroarylalkyl, an optionally substituted carbonyl, an an optionally substituted carboxamide, or a covalent substituent;
X is nitrogen or oxygen;
Y is carbon, nitrogen or oxygen, and
A broken line represents the presence or absence of a bond;

provided that:

a broken line attached to Y represents the presence or absence of a bond, if Y is carbon or nitrogen, or the broken line represents the absence of a bond, if X is oxygen; and provided that the covalent substituents are selected from a group consisting of acrylamide, alkynylamide, chloroacetamide, chlorofluoroacetamide, vinylsulfone, vinylsulfonamide, sulfonylfluoride, sulfonyltriazole, sulfonyloxyfluoride, bicyclobutaneketone, bicyclobutanamide, bicyclobutanesulfone, and bicyclobutanesulfonamide, and
that the covalent substituent can be substituted with only one of the substituents of $R_1$ through $R_6$, when the covalent substituents are substituted;

or a pharmaceutically acceptable salt, a solvate, or a prodrug thereof.

[2] A compound, or a pharmaceutically acceptable salt, a solvate, or a prodrug thereof, wherein the compound is represented by formula (2):

[Chem. 3]

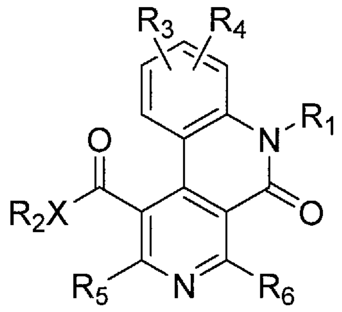

**(2)**

wherein $R_1$, $R_1$', $R_2$, $R_3$, $R_4$, $R_5$, $R_6$ and X are the same as defined in [1].

[3] The compound of [2], or a pharmaceutically acceptable salt, a solvate, or a prodrug thereof, wherein the compound is represented by formula (2-2):

[Chem. 4]

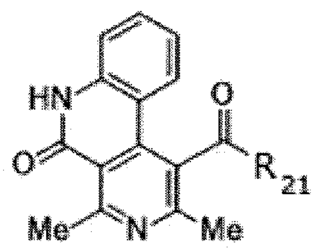

(2-2)

wherein $R_{21}$ is an optionally substituted lower alkyl, an optionally substituted arylalkyl, an optionally substituted heteroarylalkyl, an optionally substituted carboxamide, an optionally substituted carbonyl, or a covalent substituent; or

formula (2-3):

[Chem. 5]

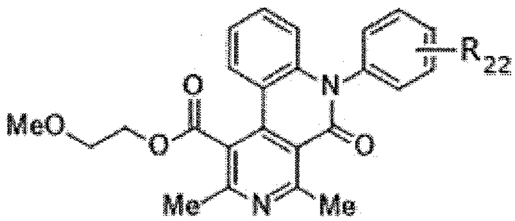

(2-3)

wherein $R_{22}$ is independently an optionally substituted carbonyl, preferably an optionally substituted carboxylic acid, an optionally substituted alkoxycarbonyl, in particular an optionally substituted methoxycarbonyl, or an optionally substituted tert-butoxycarbonyl, more preferably these substituents $R_{22}$ are located in the meta position.

[4] The compound, or a pharmaceutically acceptable salt, a solvate, or a prodrug thereof, wherein the compound is represented by formula (3):

[Chem. 6]

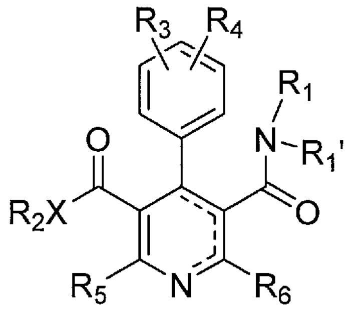

**(3)**

wherein $R_1$, $R_1$', $R_2$, $R_3$, $R_4$, $R_5$, $R_6$, X and a broken line are the same as defined in [1].

[5] The compound of [4], or a pharmaceutically acceptable salt, a solvate, or a prodrug thereof, wherein the compound is represented by formula (3-2):

[Chem. 7]

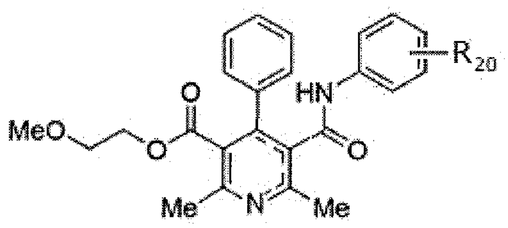

(3-2)

wherein $R_{20}$ is independently an optionally substituted carbonyl, preferably an optionally substituted carboxylic acid, an optionally substituted alkoxycarbonyl, in particular an optionally substituted methoxycarbonyl, or an optionally substituted tert-butoxycarbonyl, more preferably these substituents $R_{20}$ are located in the meta position.

[6] The compound of any one of [1] to [5], or a pharmaceutically acceptable salt, a solvate, or a prodrug thereof, wherein $R_1$ or $R_1$' is each independently hydrogen or an optionally substituted lower alkyl.

[7] The compound of any one of [1] to [6], or a pharmaceutically acceptable salt, a solvate, or a prodrug thereof, wherein $R_2$ is hydrogen or an optionally substituted lower alkyl.

[8] The compound of any one of [1] to [7], or a pharmaceutically acceptable salt, a solvate, or a prodrug thereof, wherein $R_3$ or $R_4$ is each independently selected from the group consisting of hydrogen, an optionally substituted lower alkyl, amino, and nitro.

[9] The compound of any one of [1] to [8], or a pharmaceutically acceptable salt, solvate, or a prodrug thereof, wherein $R_5$ or $R_6$ is each independently hydrogen, or an optionally substituted lower alkyl.

<Pharmaceutical composition>

[10] A pharmaceutical composition for treating or preventing a disease caused by mitochondrial hyperfission, which comprises the compound of any one of [1] to [9], or a pharmaceutically acceptable salt, a solvate, or a prodrug thereof.

[11] The pharmaceutical composition of [10], wherein the disease caused by mitochondrial hyperfission is chronic heart failure, amyotrophic lateral sclerosis, neurodegenerative diseases, inflammatory bowel diseases, or diabetes or diabetic complications.

EFFECT OF INVENTION

The compounds of the present invention have a superior Drp1-dependent inhibitory effect on mitochondrial hyperfission, compared to the approved drug, cilnidipine, and are expected to exert a higher therapeutic effect on diseases caused by mitochondrial hyperfission, such as chronic heart failure, amyotrophic lateral sclerosis, neurodegenerative diseases, inflammatory bowel disease, or diabetes or diabetic complications.

BRIEF DESCRIPTION OF DRAWINGS

Figure 1: Figure 1(a) is a fluorescence micrograph of a cell cultured under normoxia in Test Example 1, and Figure 1(b) is a fluorescence micrographof a cell cultured under hypoxia in Test Example 1.
Figure 2: Figure 2(a) is a representative fluorescence micrograph showing the general morphology of mitochondria. Figure 2(b) is an image that illustrates a shape of mitochondria extracted by filtering the photograph of Figure 2(a). Figure 2(c) is a graph that illustrates the results of measurement of length of mitochondria from the cardiomyocytes of a neonatal rat cultured under normoxia or hypoxia.
Figure 3: Figures 3(a) and (b) are graphs that illustrate the inhibitory effect on mitochondrial hyperfission of the compounds prepared in Examples, as a result of measurement of the ratio of cells having vesicle-shaped mitochondria.
Figure 4: Figures 4(a) and (b) are graphs that illustrate the results of measurement of increase in intracellular calcium ion concentration induced by potassium chloride stimulation.
Figure 5: Figure 5 is a set of photographs that illustrate suppressive effect on aging of the compounds of the present invention. Scale bar indicates 20 $\mu$m.
Figure 6: Figure 6 is graph that illustrates the cytoprotective effect against cytotoxicity induced by MeHg and hypotonic stimulation. In the figure, MeHg(+) indicates the presence of MeHg, and MeHg(-) indicates the absence of MeHg. Hypotonic stimulation (+) indicates condition in hypotonic solution, and hypotonic stimulation (-) indicates condition in isotonic solution.

EMBODIMENTS FOR CARRYING OUT INVENTION

<Compounds>

In one embodiment, the present invention provides a compound represented by formula (1):

[Chem. 8]

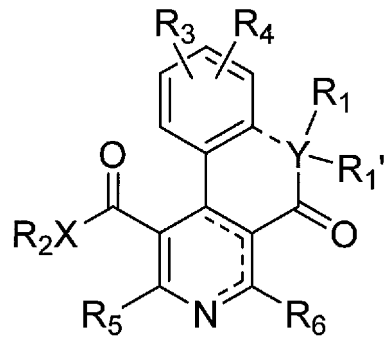

**(1)**

wherein

$R_1$ and $R_1$' are each independently hydrogen, an optionally substituted lower alkyl, an optionally substituted lower cycloalkyl, an optionally substituted lower heterocycloalkyl, an optionally substituted aryl, an optionally substituted arylalkyl, an optionally substituted heteroaryl, an optionally substituted heteroarylalkyl, an optionally substituted carbonyl, an optionally substituted carboxamide, or a covalent substituent;
$R_2$ is an optionally substituted lower alkyl, an optionally substituted lower cycloalkyl, an optionally substituted lower heterocycloalkyl, an optionally substituted aryl, an optionally substituted arylalkyl, an optionally substituted heteroaryl, an optionally substituted heteroarylalkyl, an optionally substituted carboxamide, an optionally substituted carbonyl, or a covalent substituent;
$R_3$ and $R_4$ are each independently hydrogen, halogen, hydroxy, nitro, cyano, an optionally substituted lower alkyl, an optionally substituted lower cycloalkyl, an optionally substituted lower heterocycloalkyl, an optionally substituted aryl, an optionally substituted arylalkyl, an optionally substituted heteroaryl, an optionally substituted heteroaryl, an optionally substituted heteroarylalkyl, an optionally substituted amino, an optionally substituted lower alkoxy, an optionally substituted lower alkylthio, an optionally substituted carbonyl, an optionally substituted carboxamide, deuterium, or a covalent substituent;
$R_5$ and $R_6$ are each independently an optionally substituted lower alkyl, an optionally substituted lower cycloalkyl, an optionally substituted lower heterocycloalkyl, an optionally substituted aryl, an optionally substituted arylalkyl, an optionally substituted heteroaryl, an optionally substituted heteroarylalkyl, an optionally substituted carbonyl, an optionally substituted carboxamide, or a covalent substituent;
X is nitrogen or oxygen;
Y is carbon, nitrogen or oxygen, and
A broken line represents the presence or absence of a bond;
provided that:

a broken line attached to Y represents the presence or absence of a bond, if Y is carbon or nitrogen, or the broken line represents the absence of a bond, if X is oxygen; and provided that the covalent substituents are selected from a group consisting of acrylamide, alkynylamide, chloroacetamide, chlorofluoroacetamide, vinylsulfone, vinylsulfonamide, sulfonylfluoride, sulfonyltriazole, sulfonyloxyfluoride, bicyclobutaneketone, bicyclobutanamide, bicyclobutanesulfone, and bicyclobutanesulfonamide, and
that the covalent substituent can be substituted with only one of the substituents of $R_1$ through $R_6$, when the covalent substituents are substituted;
or a pharmaceutically acceptable salt, a solvate, or a prodrug thereof.

As used herein, the term "lower alkyl" in "optionally substituted lower alkyl" means a straight or branched hydrocarbon chain group which consists of carbon atoms and hydrogen atoms, which does not contain any unsaturation, which contains 1 to 10, or 1 to 8, or 1 to 6, or 1 to 3 carbon atoms, and which connected to the remainder of the molecule by a single bond. Examples of lower alkyl groups include, but are not limited to, alkyl groups such as methyl, ethyl, n-propyl, isopropyl, isobutyl, 3-methyl-1-pentyl, 4-methyl-1-pentyl, 3,3-dimethyl-1-butyl, t-butyl, pentyl, isopentyl and hexyl. Preferred substituents are methyl, ethyl, n-propyl and isopropyl. Alkyl groups may be optionally substituted with one or more substituents, in which the substituents are as defined below.

As used herein, the term "lower cycloalkyl" in "optionally substituted lower cycloalkyl" means a nonaromatic monocyclic or polycyclic group containing carbon atoms and hydrogen atoms. A lower cycloalkyl group may contain one or more carbon-carbon double bonds in the ring, so long as the presence of the carbon-carbon double bonds does not render the ring aromatic. Examples of lower cycloalkyl groups include, but are not limited to, C3 to C7 fully saturated cycloalkyl groups such as cyclopropyl, cyclobutyl, cyclopentyl, cyclohexyl, cycloheptyl; saturated cyclic terpenes and bicyclic terpenes and C3 to C7 cycloalkenyl groups such as cyclopropenyl, cyclobutenyl, cyclopentenyl, cyclohexenyl, cycloheptenyl; and unsaturated cyclic terpenes and bicyclic terpenes. A lower cycloalkyl group may be unsubstituted or substituted with one or two suitable substituents, in which the substituents are as defined below. Preferably, cycloalkyl groups are monocyclic or bicyclic cyclic groups.

As used herein, the term "lower heterocycloalkyl" in "optionally substituted lower heterocycloalkyl" means a nonaromatic monocyclic or polycyclic group containing carbon atoms and hydrogen atoms as well as at least one heteroatom, preferably 1 to 4 heteroatoms selected from nitrogen, oxygen, and sulfur. A lower heterocycloalkyl group may contain one or more carbon-carbon double bonds or carbon-heteroatom double bonds in the ring, unless the presence of such carbon-carbon or carbon-heteroatom double bond renders the ring aromatic. Examples of heterocycloalkyl groups include, but are not limited to, aziridinyl, pyrrolidinyl, pyrrolidino, piperidinyl, piperazino, piperazinyl, piperazino, morpholinyl, morpholino, thiomorpholinyl, thiomorpholino, tetrahydrofuranyl, tetrahydrothiofuranyl, tetrahydropyranyl, and pyranyl. A lower heterocycloalkyl group may be unsubstituted or substituted with one or two suitable substituents. Preferably, a heterocycloalkyl group is a monocyclic or bicyclic group, and more preferably it is a monocyclic group that contains 2 to 6 carbon atoms and 1 to 3 heteroatoms. A lower heterocycloalkyl group may be optionally substituted with one or more substituents, in which the substituents are as defined below.

As used herein, the term "lower alkoxy" in "optionally substituted lower alkoxy" means an -O-alkyl group in alkyl as defined above. Examples of an optionally substituted lower alkoxy include, but are not limited to, a lower alkylalkoxy, a lower cycloalkylalkoxy, and a lower bicycloalkoxy.

As used herein, the term "halogen" means chloro, bromo, iodo or fluoro.

As used herein, the term "aryl" in "optionally substituted aryl" means an aromatic group, which contains 6 to 18, or 6 to 10, or 6, 7 or 8 carbon atoms, which contains 1, 2 or 3 aromatic nuclei, and which may be optionally fused. Examples of "aryl" include, but are not limited to, phenyl, naphthyl, diphenyl, indenyl, phenanthryl. An aryl group may be optionally substituted with one or more substituents, in which the substituents are as defined below.

As used herein, the term "arylalkyl" in "optionally substituted arylalkyl" means a lower alkyl group substituted with an aryl group as defined above. Preferably, the term "arylalkyl" means benzyl, diphenylmethyl, trityl, or phenethyl group.

As used herein, the term "heteroaryl" in "optionally substituted heteroaryl" means a stable 3- to 15-membered aromatic ring group which consists of a carbon atom and 1 to 5 heteroatoms selected from the group consisting of nitrogen, oxygen and sulfur. In one embodiment of the present invention, the heteroaryl group is a 3- to 10-membered, or a 5- or 6-membered aromatic ring group. In the present invention, a heteroaryl may be monocyclic, bicyclic or tricyclic ring systems, and may contain a fused ring system, in which the nitrogen, carbon or sulfur atom in the heteroaryl group may be optionally oxidized, or the nitrogen atom may be optionally quaternized. Examples of such heteroaryls include, but are not limited to, benzimidazolyl, benzothiazolyl, furanyl, pyronyl, thiophenyl, pyridinyl, bipyridinyl, pyrimidinyl, isothiazolyl, isoxazolyl, imidazolyl, indonyl, purinyl, quinolinyl, thiadiazolyl. Preferably, heteroaryl means pyridyl, pyrimidyl, furyl, thienyl, imidazolyl or thiazolyl. Preferred heteroaryls also include aziridin-1-yl, azetidin-1-yl, pyrrolidinyl, piperidinyl, piperazinyl, morpholino. A Heteroaryl group may be optionally substituted with one or more substituents, in which the substituents are as defined below.

The term "heteroarylalkyl" means a lower alkyl group substituted with a heteroaryl group as defined above. Examples of heteroarylalkyl include, but are not limited to, pyridylmethyl, 2-(pyridyl)ethyl, 3-(pyridyl)propyl, pyridin-4-ylmethyl, pyrimidylmethyl, 2-(pyrimidyl)ethyl, 3-(pyrimidyl)propyl, furylmethyl, 2-(furyl)ethyl, 3-(furyl)propyl, thienylmethyl, 2-(thienyl)ethyl, 3-(thienyl)propyl, imidazolylmethyl, 2-(imidazolyl)ethyl, 3-(imidazolyl)propyl, thiazolylmethyl, 2-(thiazolyl)ethyl, or 3-(thiazolyl)propyl).

As used herein, the term "carbonyl" in "optionally substituted carbonyl" means a group in which -C(O)- is attached with a substituent, and "optionally substituted carbonyl" includes, for example, a carboxy: -COOH in which OH is substituted, an alkylcarbonyl: -C(O)-alkyl group, or an "alkoxycarbonyl": -C(O)O-alkyl group. Preferred "optionally substituted carbonyls" include a lower alkylalkoxycarbonyl, a lower cycloalkylalkoxycarbonyl, a lower bicycloalkylalkoxycarbonyl, a lower alkylcarbonyl, a lower cycloalkylcarbonyl and a lower bicycloalkylcarbonyl.

As used herein, the term "carboxamide" in "optionally substituted carboxamide" means a group in which -C(O)N- may be attached with a substituent, and includes, for example, carboxamide: $-C(O)NH_2$, an alkylcarboxamide: -C(O)NH-alkyl group, and -C(O)N-(alkyl group)(alkyl group). Preferred "carboxamides" include, but are not limited to, carboxamide, methylcarboxamide, and dimethylcarboxamide.

As used herein, the term "optionally substituted amino" includes amino, a lower alkylamino, a lower cycloalkylamino, a lower bicycloalkylamino and the like. More specific examples include, but are not limited to, dimethylamino, ethanolamino.

As used herein, the term "covalent substituent" is synonymous with a covalent bond-forming group, and means a group which reacts with a target protein or the like to provide a compound irreversibly inhibiting or enhancing a function of the protein. Covalent substituents have the advantage in exerting potent and sustained pharmaceutical effects by irreversibly binding to target proteins. Reviews of covalent substituents have been described in in vivo targeting endogenous proteins with reactive small molecules, Naoya Shindo, Akio Ojida, Handbook of In Vivo Chemistry in Mice. From Lab to Living System, Tanaka, K., Vong, K. Eds. Wiley-VCH,; 281-307 (2020), and Covalent inhibitors: a rational approach to drug discovery, Fandi Sutanto, Markella Konstantinidou and Alexander Doemling, RSC Medicinal Chemistry, 11, 876-884 (2020). In the present invention, examples of covalent substituents include, but are not limited to, acrylamides, alkynylamides, chloroacetamides, chlorofluoroacetamides, vinylsulfones, vinylsulfonamides, sulfonylfluorides, sulfonyltriazoles, sulfonyloxyfluorides, bicyclobutaneketones, bicyclobutanamides, bicyclobutanesulfone, and bicyclobutanesulfonamide.

In the present invention, the substitution of covalent substituents is optional and not required. A compound substituted with any covalent substituent has only one covalent substituent. Specifically, in a compound substituted with any covalent substituent, the covalent substituent may be substituted with only one substituent of $R_1$ to $R_6$ substituents.

The "optionally substituted" in each of the above definitions means that a substituent may be substituted at an arbitrary, substitutable position (involved in carbon atoms and heteroatoms), and the substituent may be further substituted with one or more substituents selected from the group consisting of a halogen (fluoro, chloro, bromo); an alkoxy, preferably methoxy; an alkylcarbonyl, preferably methylcarbonyl; an alkoxycarbonyl, preferably methoxycarbonyl and ethoxycarbonyl; hydroxycarbonyl; an optionally substituted methyl such as trifluoromethyl; cyano; nitro, methanesulfonyl; isobutyl; tert-butyl; ethanone; acetamidomethyl (-CH2-NH-CO-CH3); oxazolyl or isoxazolyl substituted optionally with a C1 to C3 alkyl (such as methyl); pyridyl; hydroxy; phenyl; an alkylamide, preferably isobutylamide (-NH-CO-CH-(CH3)2); an O-alkylamino-alkyl, preferably O-(CH2)2-N-(CH2-CH3)2; N-acetamide; [1,2,3]thiadiazolyl; dialkylamine, preferably dimethylamine; ethyl; ethyl substituted by amine (such as dialkylamine), or a dialkylcarboxamide, preferably dimethylamine and diethylamine), preferably a group in which the dialkylamine of the dialkylcarboxamide forms a 4-, 5-, 6- or 7-membered ring; isopropyl; N-propionamide, haloacetyl, cyano, formyl and ketones.

Preferably, the above substituents are selected from the group consisting of fluoro, chloro, bromo, methoxy, methylcarbonyl, methoxycarbonyl, hydroxycarbonyl, ethoxycarbonyl, an optionally substituted methyl such as trifluoromethyl, cyano, nitro, methanesulfonyl, pyridine, tert-butyl, acetamidomethyl (-CH2-NH-CO-CH3), oxazolyl or isoxazolyl substituted optionally with a C1 to C3 alkyl (such as methyl), phenyl, O-(CH2)2-N-(CH2-CH3)2, N-acetamide, dimethylamine, isopropyl, N-propionamide, haloacetyl, cyano, formyl and a ketone.

The compounds of the present invention may be in the form of salts, preferably pharmaceutically acceptable salts, solvates or prodrugs.

In yet another preferred embodiment, the present invention provides a compound, or a pharmaceutically acceptable salt, a solvate, or a prodrug thereof, wherein the compound is represented by formula (2):

[Chem. 9]

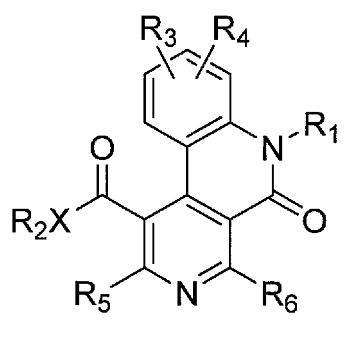

**(2)**

wherein $R_1$, $R_1'$, $R_2$, $R_3$, $R_4$, $R_5$, $R_6$ and X are the same as defined in formula (1).

In yet another preferred embodiment, the present invention provides a compound, or a pharmaceutically acceptable salt, a solvate, or a prodrug thereof, wherein the compound is represented by formula (3):

[Chem. 10]

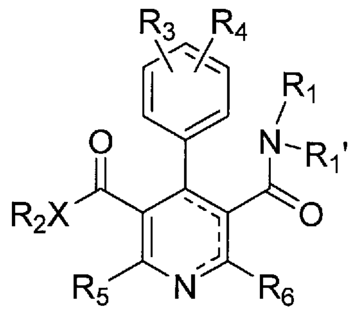

**(3)**

wherein $R_1$, $R_1$', $R_2$, $R_3$, $R_4$, $R_5$, $R_6$, X and a broken line are the same as defined in formula (1).

In yet another preferred embodiment, the present invention provides a compound represented by formula (1):

[Chem. 11]

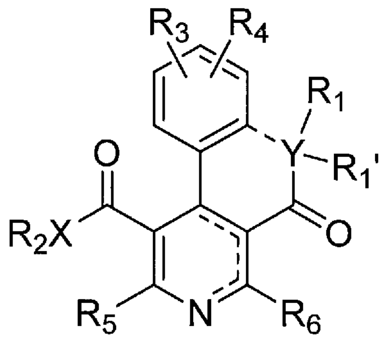

**(1)**

wherein

X is oxygen;
Y is nitrogen or oxygen;
A broken line represents the presence or absence of a bond;
One of $R_1$ and $R_1$' is hydrogen, and the other is an optionally substituted lower alkyl, an optionally substituted aryl, an optionally substituted arylalkyl, an optionally substituted heteroarylalkyl, an optionally substituted carbonyl, an optionally substituted carboxamide, or a covalent substituent;
$R_2$ is an optionally substituted lower alkyl, an optionally substituted arylalkyl, an optionally substituted heteroarylalkyl, an optionally substituted carboxamide, an optionally substituted carbonyl, or a covalent substituent;
$R_3$ and $R_4$ are each independently hydrogen, halogen, hydroxy, nitro, cyano, an optionally substituted lower alkyl, an optionally substituted amino, an optionally substituted lower alkoxy, an optionally substituted lower alkylthio, an optionally substituted carbonyl, an optionally substituted carboxamide, deuterium, or a covalent substituent;
$R_5$ and $R_6$ are each independently an optionally substituted lower alkyl, an optionally substituted carbonyl, an optionally substituted carboxamide, or a covalent substituent;
provided that:

a broken line attached to Y represents the presence or absence of a bond, if Y is carbon or nitrogen, or the broken line represents the absence of a bond, if X is oxygen; and provided that the covalent substituents are selected from a group consisting of acrylamide, alkynylamide, chloroacetamide, chlorofluoroacetamide, vinylsulfone, vinylsulfonamide, sulfonylfluoride, sulfonyltriazole, sulfonyloxyfluoride, bicyclobutaneketone, bicyclobutanamide, bicyclobutanesulfone, and bicyclobutanesulfonamide, and

that the covalent substituent can be substituted with only one of the substituents of $R_1$ through $R_6$, when the covalent substituents are substituted;
or a pharmaceutically acceptable salt, a solvate, or a prodrug thereof.

In yet another preferred embodiment, the present invention provides a compound represented by formula (2):

[Chem. 12]

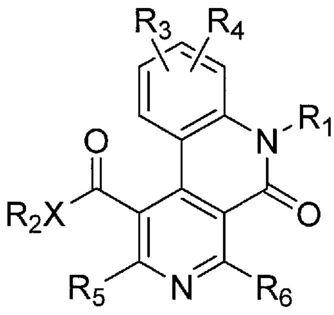

**(2)**

wherein

X is oxygen;
$R_1$ is an optionally substituted lower alkyl, an optionally substituted aryl, an optionally substituted arylalkyl, an optionally substituted heteroarylalkyl, an optionally substituted carbonyl, an optionally substituted carboxamide, or is a covalent substituent;
$R_2$ is an optionally substituted lower alkyl, an optionally substituted arylalkyl, an optionally substituted heteroarylalkyl, an optionally substituted carboxamide, an optionally substituted carbonyl, or a covalent substituent;
$R_3$ and $R_4$ are each independently hydrogen, halogen, hydroxy, nitro, cyano, an optionally substituted lower alkyl, an optionally substituted amino, an optionally substituted lower alkoxy, an optionally substituted lower alkylthio, an optionally substituted carbonyl, an optionally substituted carboxamide, deuterium, or covalent substituent;
$R_5$ and $R_6$ are each independently an optionally substituted lower alkyl, an optionally substituted carbonyl, an optionally substituted carboxamide, or a covalent substituent;

provided that:

the covalent substituents are acrylamide, alkynylamide, chloroacetamide, chlorofluoroacetamide, vinylsulfone, vinylsulfonamide, sulfonylfluoride, sulfonyltriazole, sulfonyloxyfluoride, bicyclobutaneketone, bicyclobutanamide, bicyclobutanesulfone, and bicyclobutanesulfonamide; and
that the covalent substituents can be substituted with only one of the substituents of $R_1$ through $R_6$, when the covalent substituents are substituted;

or a pharmaceutically acceptable salt, a solvate, or a prodrug thereof.

In such embodiments, preferred compounds are represented by formula (2-2):

[Chem. 13]

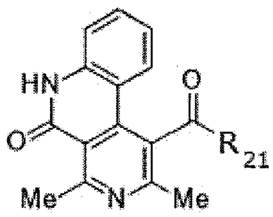

(2-2)

wherein $R_{21}$ is an optionally substituted lower alkyl, an optionally substituted arylalkyl, an optionally substituted heteroarylalkyl, an optionally substituted carboxamide, an optionally substituted carbonyl, or a covalent substituent; or a pharmaceutically acceptable salt, a solvate, or a prodrug thereof; or

formula (2-3):

[Chem. 14]

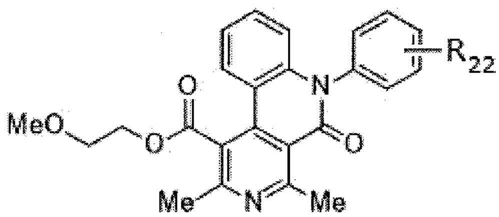

(2-3)

wherein $R_{22}$ is independently an optionally substituted carbonyl, preferably an optionally substituted carboxylic acid, an optionally substituted alkoxycarbonyl, in particular an optionally substituted methoxycarbonyl, or an optionally substituted tert-butoxycarbonyl, more preferably these substituents $R_{22}$ are located in the meta position; or a pharmaceutically acceptable salt, a solvate, or a prodrug thereof.

In yet another preferred embodiment, the present invention provides a compound represented by formula (3):

[Chem. 15]

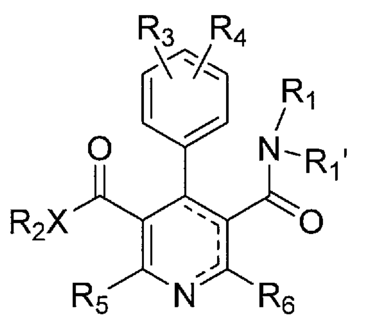

**(3)**

wherein

X is oxygen;
a broken line represents the presence or absence of a bond;
one of $R_1$ and $R_1$ is hydrogen, and the other is an optionally substituted lower alkyl, an optionally substituted aryl, an optionally substituted arylalkyl, an optionally substituted heteroarylalkyl, substituted optionally a carbonyl, an optionally substituted carboxamide, or a covalent substituent;
$R_2$ is an optionally substituted lower alkyl, an optionally substituted arylalkyl, an optionally substituted heteroarylalkyl, an optionally substituted carboxamide, an optionally substituted carbonyl, or a covalent substituent;
$R_3$ and $R_4$ are each independently hydrogen, halogen, hydroxy, nitro, cyano, an optionally substituted lower alkyl, an optionally substituted amino, an optionally substituted lower alkoxy, an optionally substituted lower alkylthio, an optionally substituted carbonyl, an optionally substituted carboxamide, deuterium, or covalent substituent;
$R_5$ and $R_6$ are each independently an optionally substituted lower alkyl, an optionally substituted carbonyl, an optionally substituted carboxamide, or a covalent substituent;

provided that:

the covalent substituents are acrylamide, alkynylamide, chloroacetamide, chlorofluoroacetamide, vinylsulfone, vinylsulfonamide, sulfonylfluoride, sulfonyltriazole, sulfonyloxyfluoride, bicyclobutaneketone, bicyclobutanamide, bicyclobutanesulfone, and bicyclobutanesulfonamide; and
that the covalent substituents can be substituted with only one of the substituents of $R_1$ through $R_6$, when the covalent substituents are substituted;

or a pharmaceutically acceptable salt, a solvate, or a prodrug thereof.

In such embodiments, preferred compounds are represented by formula (3-2):

[Chem. 16]

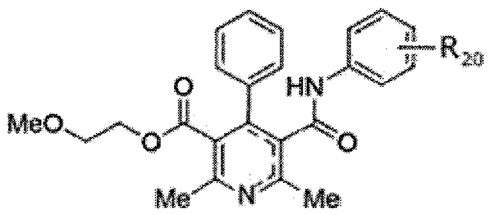

(3-2)

further preferred compounds are represented by formula (3-2'):

[Chem. 17]

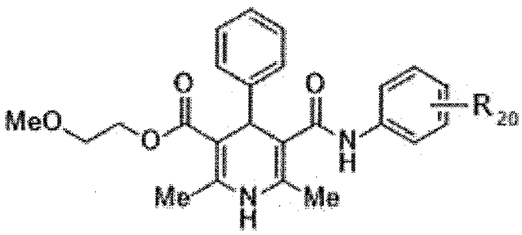

(3-2')

or formula (3-2"):

[Chem. 18]

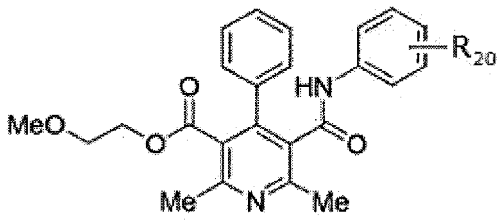

(3-2")

wherein $R_{20}$ is independently an optionally substituted carbonyl, preferably an optionally substituted carboxylic acid, an optionally substituted alkoxycarbonyl, in particular an optionally substituted methoxycarbonyl, or an optionally substituted tert-butoxycarbonyl, more preferably these substituents $R_{20}$ are located in the meta position; or a pharmaceutically acceptable salt, a solvate, or a prodrug thereof.

The compounds of the present invention are preferably those wherein $R_1$ and $R_1$' are each independently selected from the group consisting of hydrogen or an optionally substituted lower alkyl, and more preferably those wherein $R_1$ and $R_1$' are each independently selected from the group consisting of hydrogen, methyl, benzyl, and 4-pyridylmethyl, or pharmaceutically acceptable salts, solvates, or prodrugs thereof.

In another embodiment, the compounds of the present invention are those wherein $R_2$ is preferably selected from the group consisting of hydrogen and an optionally substituted lower alkyl, and more preferably selected from the group consisting of methyl, ethyl, benzyl, pyridin-4-ylmethyl, 2-(pyridin-4-yl)ethyl, and 3-(pyridin-4-yl)propyl, or pharmaceutically acceptable salts, solvates, or prodrugs thereof.

In another embodiment, the compounds of the present invention are preferably those wherein $R_3$ or $R_4$ is each independently selected from the group consisting of hydrogen, an optionally substituted lower alkyl, amino, and nitro, and more preferably those wherein $R_3$ or $R_4$ is each independently selected from the group consisting of hydrogen, methyl, ethyl, amino, and nitro, or pharmaceutically acceptable salts, solvates, or prodrugs thereof. Even more preferably, $R_3$ or $R_4$ is a group that can serve as the basis for covalent substituents.

In another embodiment, the compounds of the present invention are preferably those in which $R_5$ or $R_6$ is each independently selected from the group consisting of hydrogen and an optionally substituted lower alkyl, and more preferably those in which $R_5$ or $R_6$ is each independently selected from the group consisting of hydrogen, methyl, ethyl, hydroxymethyl, aminomethyl, 2-hydroxyethyl, and 2-aminoethyl, or pharmaceutically acceptable salts, solvates, or prodrugs thereof. Even more preferably, $R_5$ or $R_6$ is a group that can serve as the basis for covalent substituents.

In one preferred embodiment of the present invention, a compound of the present invention wherein $R_1$, $R_1$', $R_2$, $R_3$, $R_4$, $R_5$ and $R_6$ are each of the preferred groups, or a pharmaceutically acceptable salt, a solvate, or a prodrug thereof is provided.

The term "pharmaceutically acceptable salt" means a salt that, when administered to a subject, affords (directly or indirectly) a compound described herein. The preparation of the salt may be carried out by methods known in the art. Preferably, when administered to humans, the "pharmaceutically acceptable salt" is physiologically tolerable, and typically affords a molecular part that does not create any allergic reactions or any similar untoward reactions (such as upset stomach, dizziness, and the like).

For example, pharmaceutically acceptable salts of the compounds provided herein are synthesized from the parent compounds, which contain a basic or acidic moiety, according to conventional chemical methods. Generally, such salts are prepared by reacting the free acid or free base forms of these compounds with appropriate bases or acids in stoichiometric amounts, for example, in water or an organic solvent, or a mixture thereof. Generally, non-aqueous media such as ether, ethyl acetate, ethanol, isopropanol or acetonitrile are preferred. Examples of acid addition salts include mineral acid addition salts (such as hydrochloride, hydrobromide, hydroiodide, sulfate, nitrate, phosphate), and organic acid addition salts such as acetate, maleate, fumarate, citrate, oxalate, succinate, tartrate, malate, mandelate, methanesulfonate and p-toluenesulfonate. Examples of alkali addition salts include inorganic salts (such as salts of sodium, potassium, calcium, ammonium, magnesium, aluminum, and lithium), and organic alkali salts (such as salts of ethylenediamine, ethanolamine, N,N-dialkyleneethanolamine, triethanolamine, glucamine, and a basic amino acid).

According to the present invention, it should be understood that the term "solvate" means any form of the active

compound of the present invention to which another molecule (most likely it would be a polar solvent) is attached via a non-covalent bond. Examples of such solvates include hydrates and alcoholates such as methanolates.

Whether the compounds of the present invention are either free compounds or solvates (such as hydrates), they may be in crystalline forms, and both forms should be within the scope of the present invention. In general, methods of solvation are known in the art. Suitable solvates are pharmaceutically acceptable solvates. In specific embodiments, the solvate is a hydrate.

The term "prodrug" as used herein means a chemical compound that is subjected to chemical derivatization such as substitution or addition of a further chemical group so as to alter any of physicochemical properties thereof, for example, solubility or bioavailability toward pharmaceutical use. Examples of the prodrug include ester and ether derivatives of an active compound which, when administered to a subject, afford the active compound itself. Examples of the well-known methods for preparing prodrugs of a given active compound are known to those skilled in the art, and may be found in, for example, Krogsgaard-Larsen et al., Textbook of Drug design and Discovery, Taylor & Francis (April 2002).

Preparation of salts, solvates and prodrugs can be carried out by methods known in the art. It should be understood that non-pharmaceutically acceptable salts, solvates or prodrugs are also within the scope of the present invention as they may be useful in the preparation of pharmaceutically acceptable salts, solvates or prodrugs.

Methods for preparing the compounds of the present invention are generally described below.

Reaction Scheme I

[Chem. 19]

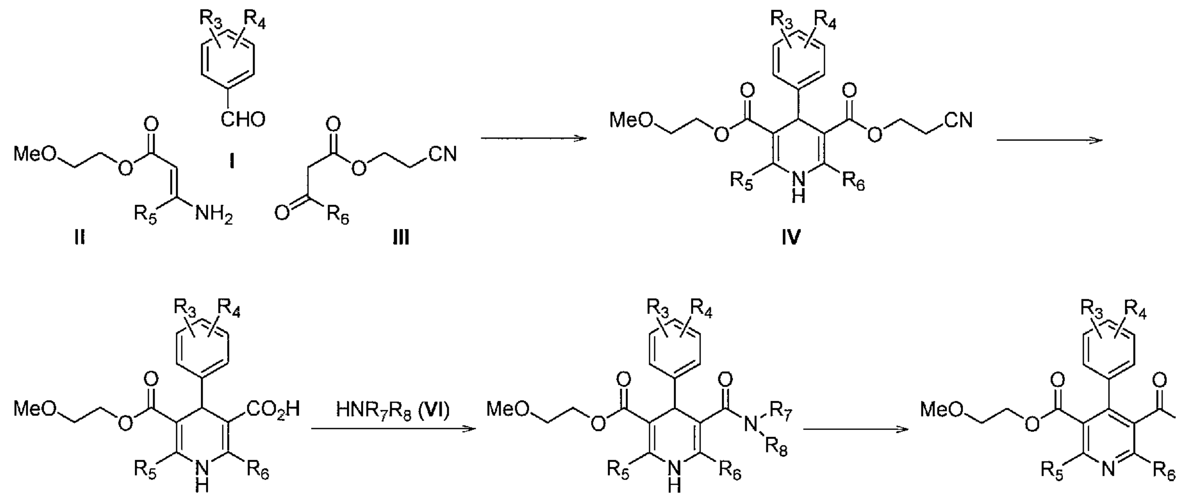


I II III IV

V VII VIII

wherein

R$_3$ to R$_6$ are the same as defined in formula (1); and

R$_7$ and R$_8$ are each independently an optionally substituted lower alkyl, an optionally substituted lower cycloalkyl, an optionally substituted lower heterobicycloalkyl, an optionally substituted aryl, an optionally substituted arylalkyl, an optionally substituted heteroaryl, an optionally substituted heteroarylalkyl, an optionally substituted carbonyl, an optionally substituted carboxamide, or a covalent substituent.

The compound represented by general formula (IV) can be obtained by subjecting three components of a benzaldehyde of general formula (I), a compound of general formula (II), and a compound of general formula (III) to a condensation reaction.

This reaction can be carried out in a suitable solvent at a suitable temperature. Any solvent may be used as long as it does not adversely affect the reaction. Examples include acetonitrile, methanol, ethanol, n-propyl alcohol, 2-propyl alcohol, t-butyl alcohol, acetone, N,N'-dimethylformamide, dimethyl sulfoxide, tetrahydrofuran, diethyl ether, dioxane, ethyl acetate, toluene, methylene chloride, dichloroethane, chloroform, N,N'-dimethylacetamide, 1,3-dimethyl-2-imidazolidinone, 1-methyl-2-pyrrolidinone, 1,2-dimethoxyethane, xylene, and a mixed solvent thereof.

This reaction preferably proceeds at 0 to 100°C, in particular at room temperature to 80°C.

Then, the resulting compound of general formula (IV) can be subjected to a hydrolysis reaction to obtain a

compound of general formula (V).

This reaction can be carried out in a suitable solvent in the presence or absence of a suitable base at a suitable temperature. Examples of the Base include sodium hydroxide, potassium hydroxide, ammonia, triethylamine, diisopropylethylamine, tetra-n-butylammonium fluoride and the like.

This reaction preferably proceeds at -20 to 100°C, in particular at 0 to 80°C.

Then, the compound of general formula (V) and a disubstituted amine of general formula (VI) can be subjected to a condensation reaction to obtain a compound of general formula (VII).

This reaction can be carried out in a suitable solvent in the presence of a suitable condensing agent, in the presence or absence of a suitable additive, in the presence or absence of a suitable base, at a suitable temperature.

Examples of the condensing agent include N,N'-dicyclohexylcarbodiimide, N,N'-diisopropylcarbodiimide, 1-ethyl-3-(3-dimethylaminopropyl)carbodiimide, N,N'-carbonyldiimidazole, 1,1'-carbonyldi(1,2,4-triazole), 4-(4,6-dimethoxy-1,3,5-triazin-2-yl)-4-methylmorpholinium chloride n-hydrate (where n is any number), O-(7-azabenzotriazol-1-yl)-N,N,N',N'-tetramethyluronium hexafluorophosphate, {{(1-cyano-2-ethoxy-2-oxoethylidene)amino)oxy}-4-morpholinomethylene}dimethylammonium hexafluorophosphate, and the like.

Examples of the additive include 1-hydroxybenzotriazole, 1-hydroxy-7-azabenzotriazole, N-hydroxysuccinimide and the like.

This reaction preferably proceeds at -20 to 100°C, in particular at 0 to 80°C.

Then, the compound of general formula (VII) can be subjected to an oxidation reaction to obtain a compound of general formula (VIII).

This reaction can be carried out in a suitable solvent in the presence of a suitable oxidizing agent at a suitable temperature.

Examples of the oxidizing agent include 2,3-dichloro-5,6-dicyano-p-benzoquinone, diammonium cerium (IV) nitrate, potassium peroxodisulfate, 2,2,6,6-tetramethylpiperidine 1-oxyl, and the like.

This reaction preferably proceeds at 0 to 100°C, in particular at room temperature to 80°C.

Alternatively, the compounds of the present invention can be prepared according to Reaction Scheme II below.

Reaction Scheme II

[Chem. 20]

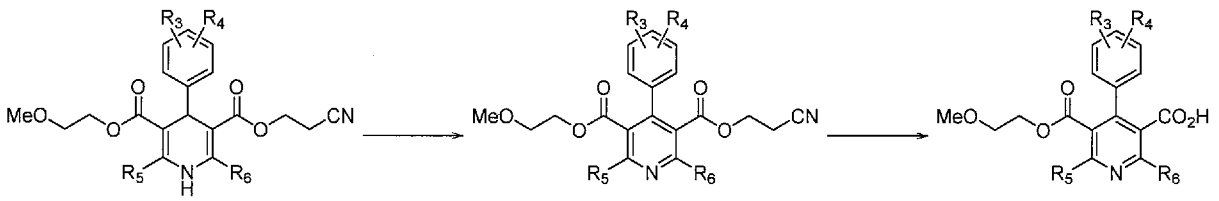

IV IX X

wherein $R_3$ to $R_6$ are the same as defined in Reaction Scheme (1).

A compound of general formula (IV) can be subjected to an oxidation reaction to convert to a compound of general formula (IX).

This reaction can be carried out in a suitable solvent in the presence of a suitable oxidizing agent at a suitable temperature.

Examples of the oxidizing agent include 2,3-dichloro-5,6-dicyano-p-benzoquinone, diammonium cerium (IV) nitrate, potassium peroxodisulfate, 2,2,6,6-tetramethylpiperidine 1-oxyl, and the like.

This reaction preferably proceeds at 0 to 100°C, in particular at room temperature to 80°C.

Then, the compound of general formula (IX) can be subjected to a hydrolysis reaction to obtain a compound of general formula (X).

This reaction can be carried out in a suitable solvent in the presence of a suitable base at a suitable temperature.

Examples of the base include sodium hydroxide, potassium hydroxide, ammonia, triethylamine, diisopropylethylamine, tetra-n-butylammonium fluoride and the like.

This reaction preferably proceeds at -20 to 100°C, in particular at 0 to 80°C.

Additionally, the compounds of the present invention can be prepared according to Reaction Scheme III below.

Reaction Scheme III

[Chem. 21]

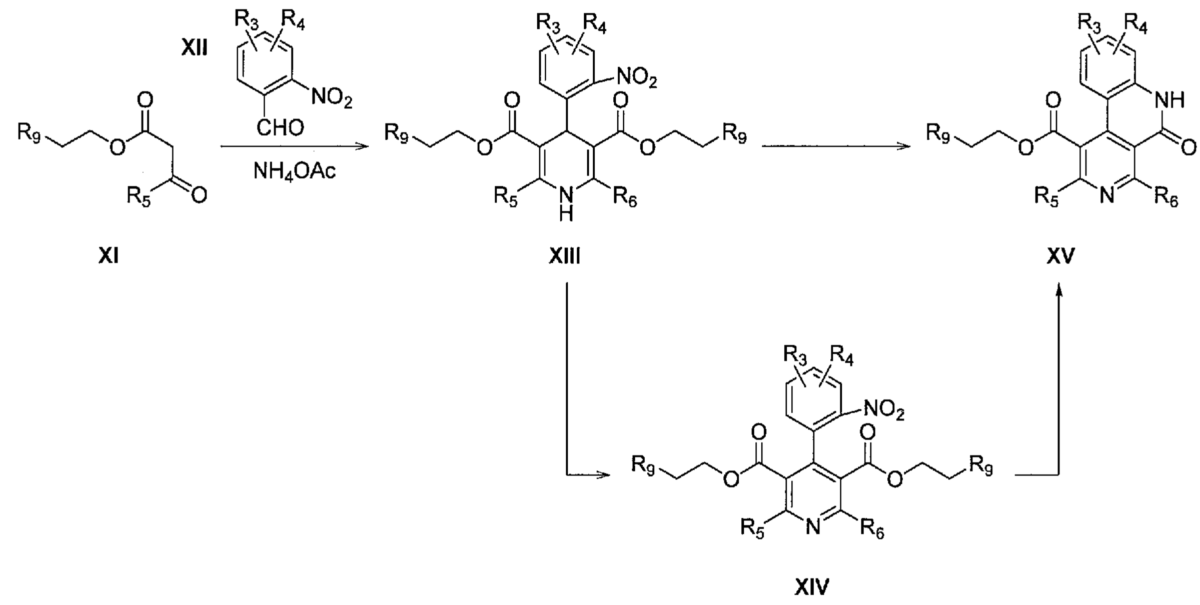


wherein

$R_5$ to $R_6$ are the same as defined in Reaction Scheme (1);
$R_9$ is an optionally substituted alkoxy, preferably methoxy, cyano, or the like; and
Provided that $R_5$ and $R_6$ represent the same group in Reaction Formula III.

The compound of general formula (XIII) can be obtained by subjecting three components of an acetoacetate of general formula (XI), a benzaldehyde with a nitro group of general formula (XII), and ammonium acetate to a condensation reaction.
This reaction can be carried out in a suitable solvent at a suitable temperature.
This reaction preferably proceeds at 0 to 100°C, in particular at room temperature to 80°C.
The compound of general formula (XIII) can be subjected to an oxidation reaction to obtain a compound of general formula (XIV).
This reaction can be carried out in a suitable solvent in the presence of a suitable oxidizing agent at a suitable temperature.
Examples of the oxidizing agent include 2,3-dichloro-5,6-dicyano-p-benzoquinone, diammonium cerium (IV) nitrate, potassium peroxodisulfate, 2,2,6,6-tetramethylpiperidine 1-oxyl, and the like.
This reaction preferably proceeds at 0 to 100°C, in particular at room temperature to 80°C.
A compound of general formula (XV) can be obtained by subjecting the nitro group of the compound of general formula (XIV) to a reductive reaction, as well as cyclizing intramolecularly.
This reaction can be carried out in a suitable solvent in the presence or absence of a suitable reducing agent, in the presence or absence of a suitable catalyst, in the presence or absence of hydrogen, under a suitable pressure, at a suitable temperature.
As the reducing agent, iron, tin(II) chloride, palladium carbon, platinum carbon, noble metal catalysts, and the like can be used.
This reaction proceeds preferably at normal pressure to 10 atm, in particular at normal pressure to 3 atm.
This reaction preferably proceeds at 0 to 100°C, in particular at room temperature to 80°C.
Further, the compound of general formula (XV) can be also obtained by subjecting the nitro group of the compound of general formula (XIII) to a reductive reaction, as well as cyclizing intramolecularly and aromatizing at the same time.
This reaction can be carried out in a suitable solvent in the presence or absence of a suitable reducing agent, in the presence or absence of a suitable catalyst, in the presence or absence of hydrogen, under a suitable pressure, at a suitable temperature.
As the reducing agent, iron, tin(II) chloride, palladium carbon, platinum carbon, noble metal catalysts, and the like can be used.
This reaction proceeds favorably at normal pressure to 10 atm, in particular at normal pressure to 3 atm.
This reaction preferably proceeds at 0 to 100°C, in particular room temperature to 80°C.
Additionally, the compounds of the present invention can be prepared according to Reaction Scheme IV below.

Reaction Scheme IV

[Chem. 22]

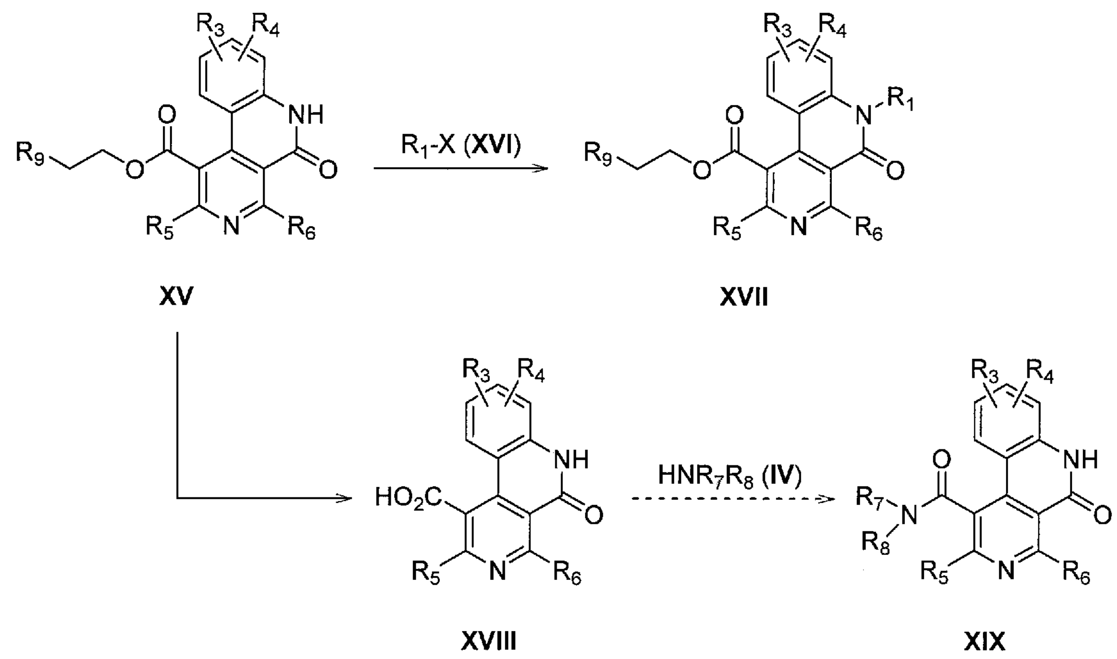


**XV** **XVII** **XVIII** **XIX**

wherein $R_3$ to $R_8$ are the same as defined in Reaction Scheme (1) .

A compound of general formula (XVII) can be obtained by subjecting the compound of general formula (XV) to a condensation reaction using a halogenated compound of general formula (XVI) wherein X is chlorine, bromine, iodine, or the like.

This reaction can be carried out in a suitable solvent in the presence of a suitable base at a suitable temperature.

Examples of the base include sodium hydride, potassium hydride, triethylamine, diisopropylethylamine, dimethylaminopyridine, sodium alkoxide and the like.

This reaction preferably proceeds at -20 to 100°C, in particular at 0 to 80°C.

Alternatively, a compound of general formula (XVIII) can be obtained by subjecting the compound of general formula (XV), wherein $R_9$ = CN, to a hydrolysis reaction.

This reaction can be carried out in a suitable solvent in the presence of a suitable base at a suitable temperature.

Examples of the base include sodium hydroxide, potassium hydroxide, ammonia, triethylamine, diisopropylethylamine, tetra-n-butylammonium fluoride and the like.

This reaction preferably proceeds at -20 to 100°C, in particular at 0 to 80°C.

Then, the compound of general formula (XVIII) and the disubstituted amine of general formula (IV) can be subjected to a condensation reaction to obtain a compound of general formula (XIX).

This reaction can be carried out in a suitable solvent in the presence of a suitable condensing agent, in the presence or absence of a suitable additive, in the presence or absence of a suitable base, at a suitable temperature.

Examples of the condensing agent include N,N'-dicyclohexylcarbodiimide, N,N'-diisopropylcarbodiimide, 1-ethyl-3-(3-dimethylaminopropyl)carbodiimide, N,N'-carbonyldiimidazole, 1,1'-carbonyldi(1,2,4-triazole), 4-(4,6-dimethoxy-1,3,5-triazin-2-yl)-4-methylmorpholinium chloride n-hydrate, O-(7-azabenzotriazol-1-yl)-N,N,N',N'-tetramethyluronium hexafluorophosphate, {{((1-cyano-2-ethoxy-2-oxoethylidene) amino)oxy}-4-morpholinomethylene}dimethylammonium hexafluorophosphate and the like.

Examples of the additives include 1-hydroxybenzotriazole, 1-hydroxy-7-azabenzotriazole, N-hydroxysuccinimide and the like.

This reaction preferably proceeds at -20 to 100°C, in particular at 0 to 80°C.

Alternatively, the compounds of the present invention can be prepared according to Reaction Scheme V below.

Reaction Scheme V

[Chem. 23]

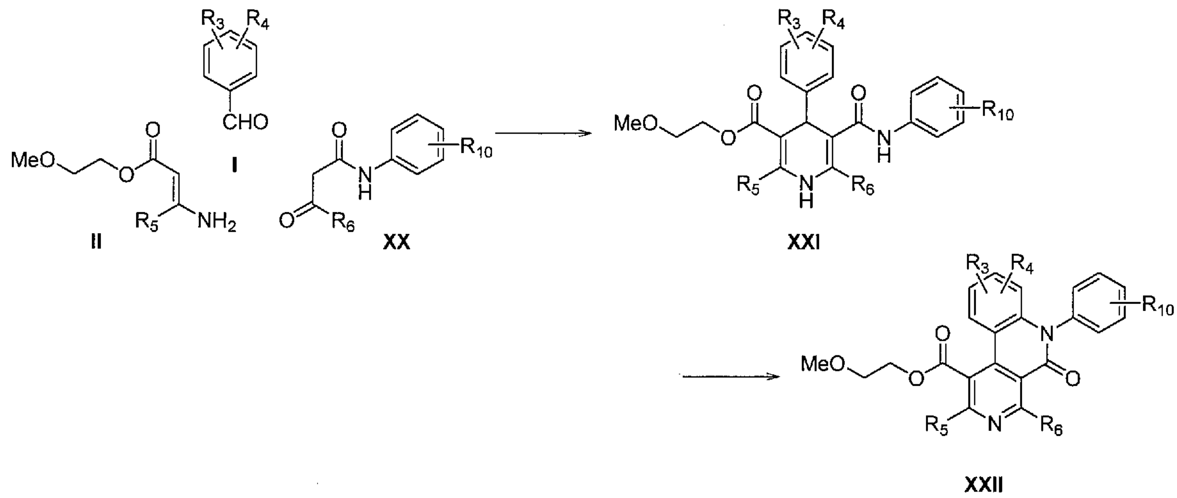

II I XX XXI XXII

wherein

$R_3$ to $R_8$ are the same as defined in Reaction Scheme (1); and
$R_{10}$ is hydrogen, hydroxy, an optionally substituted lower alkyl, an optionally substituted lower alkoxy, an optionally substituted carbonyl, cyano, formyl, nitro, or an optionally substituted amino.

A compound of general formula (XXI) can be obtained by subjecting three components of the benzaldehyde of general formula (I), the compound of general formula (II), and the acetoacetamide of general formula (XX) to a condensation reaction. Then, the compound of general formula (XXI) can be subjected to an oxidative cyclization reaction to obtain a compound of general formula (XXII).

This reaction can be carried out in a suitable solvent at a suitable temperature.

This reaction preferably proceeds at 0 to 100°C, in particular at room temperature to 80°C.

Alternatively, the compounds of the present invention can be prepared according to Reaction Scheme VI below.
Reaction Scheme VI

[Chem. 24]

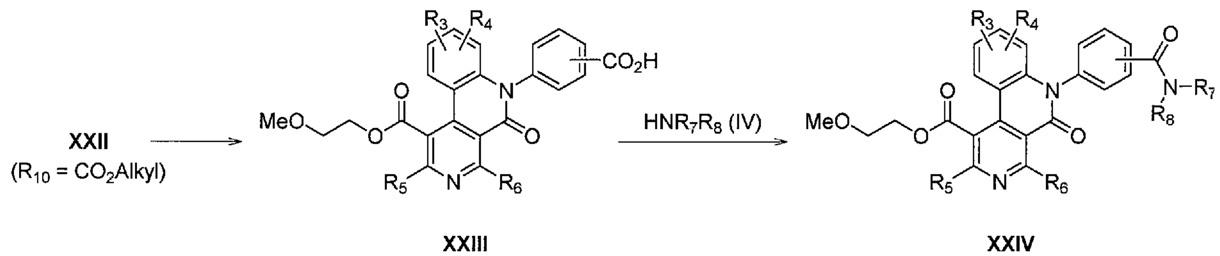


XXIII XXIV

wherein $R_3$ to $R_8$ are the same as defined in Reaction Scheme (1) .

A compound of general formula (XXIII) can be obtained by subjecting the compound of the general formula (XXII) wherein $R_{10}$ is an alkoxycarbonyl group, preferably methoxycarbonyl group, ethoxycarbonyl group, tert-butoxycarbonyl group or the like) to a hydrolysis reaction. Then, the compound represented by general formula (XXIII) and the disubstituted amine represented by general formula (IV) can be subjected to a condensation reaction to obtain the compound represented by general formula (XXIV).

Alternatively, the compounds of the present invention of general formula (XIX) can be prepared according to Reaction Scheme VII below.

Reaction Scheme VII

[Chem. 25]

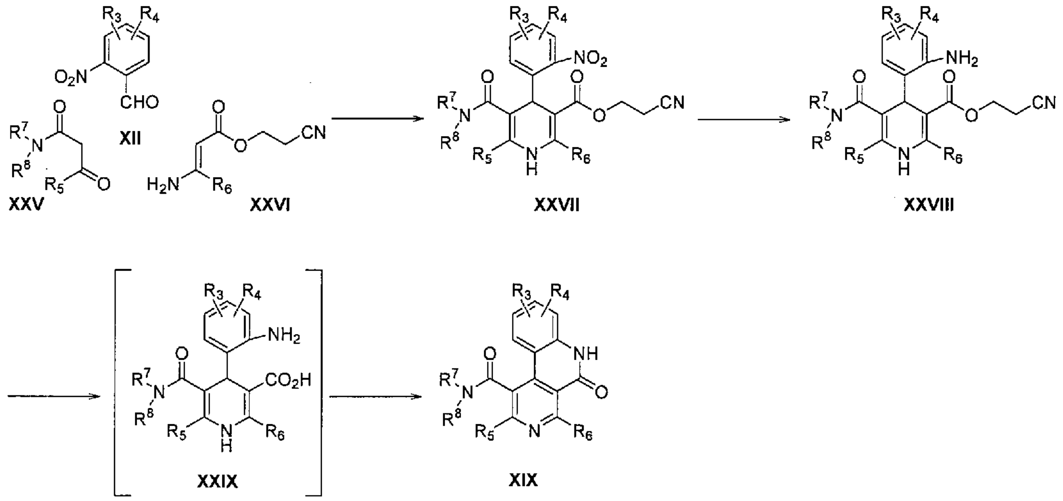

XII, XXV, XXVI, XXVII, XXVIII, XXIX, XIX

wherein $R_3$ to $R_8$ are the same as defined in Reaction Scheme (1) .

A compound of general formula (XXVII) can be obtained by subjecting a benzaldehyde with a nitro group of general formula (XII), a compound of general formula (XXVI) and an acetoacetamide of general formula (XXV) to a condensation reaction. Then, the nitro group of general formula (XXVII) can be subjected to a reduction reaction to obtain a compound of general formula (XXVIII). Next, the compound of general formula (XXVIII) can be subjected to a hydrolysis reaction, and the resulting compound of (XXIX) can be intramolecularly cyclized and aromatized to obtain a compound of general formula (XIX). Alternatively, the compound of general formula [XIX] can be obtained by subjecting the nitro group of the compound of general formula [XXVII] to a reduction reaction, followed by cyclizing intramolecularly and aromatizing, which process dose or do not go through the compound of [XXIX].

Alternatively, the compounds of general formula (XIX) of the present invention can be prepared according to Reaction Scheme VIII below.

Reaction Scheme VIII

[Chem. 26]

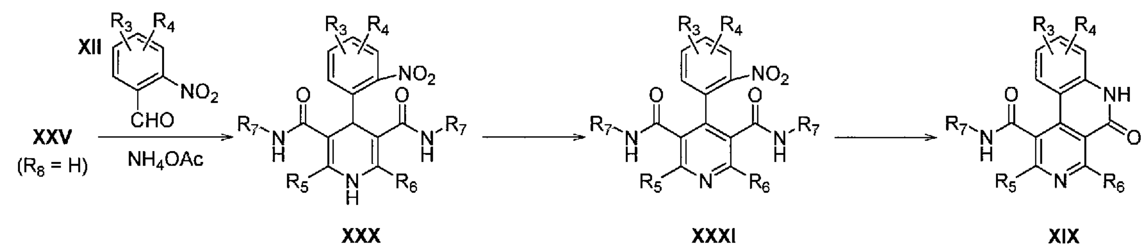


XII, XXV, XXX, XXXI, XIX

wherein

$R_3$ to $R_8$ are the same as defined in Reaction Scheme (1);
provided that $R_5$ and $R_6$ mean the same group in Reaction Scheme VIII.

A compound of general formula (XXX) can be obtained by subjecting three components of an acetoacetamide of general formula (XXV) wherein $R_8$ is hydrogen, a benzaldehyde with a nitro group of general formula (XII), and ammonium acetate to a condensation reaction. Then, the compound of general formula (XXX) can be subjected to an oxidation reaction to obtain a compound of general formula (XXXI). Next, the nitro group of the compound of general formula (XXXI) can be subjected to a reduction reaction, and simultaneously to an intramolecular cyclization and an

aromatization, to obtain a compound of general formula (XIX).

<Pharmaceutical composition>

As another aspect, the present invention relates to a pharmaceutical composition for treating or preventing a disease caused by mitochondrial hyperfission, which comprises the compound of the present invention, or a pharmaceutically acceptable salt, a solvate, or a prodrug thereof. As demonstrated in the working examples herein, the compounds of the present invention and the like are specific those which have a potent inhibitory effect on mitochondrial hyperfission based on inhibitory effect on the formation of Drp1-filamin complex, and which, unlike cilnidipine, attenuate the calcium channel inhibitory effect that is the main action of cilnidipine. It can be expected that inhibition of mitochondrial hyperfission exert a strong therapeutic effect on chronic heart failure, amyotrophic lateral sclerosis, neurodegenerative diseases, inflammatory bowel diseases, or diabetes or diabetic complications, for example.

As used herein, "treatment" means a method or process aimed at (1) delaying a disease caused by mitochondrial hyperfission; (2) slowing down or halting the progression, aggravation or exacerbation of the symptoms of a disease caused by mitochondrial hyperfission; (3) inducing remission of the symptoms of a disease caused by mitochondrial hyperfission; or (4) facilitating the cure of a disease caused by mitochondrial hyperfission. Treatment may be given as a prophylactic measure before the onset of the disease or the condition, or treatment may be given after the onset of the disease.

As used herein, "prevention" means a prophylactic action on a disease caused by mitochondrial hyperfission.

Examples of the disease caused by mitochondrial hyperfission include, for example, chronic heart failure, amyotrophic lateral sclerosis, neurodegenerative diseases, inflammatory bowel diseases, or diabetes or diabetic complications.

According to the present invention, a pharmaceutical composition usually means a drug agent for treatment or prevention of diseases, or for examination/diagnosis.

The pharmaceutical compositions of the present invention may be formulated by methods well-known to those skilled in the art. For example, the pharmaceutical compositions may be used parenterally in the form of injections of sterile solutions or suspensions with water or other pharmaceutically acceptable liquids. For example, the pharmaceutical compositions may be formulated by combining appropriately with pharmacologically acceptable carriers or vehicles, in particular sterile water, physiological saline, vegetable oils, emulsifiers, suspending agents, surfactants, stabilizers, flavoring agents, excipients, vehicles, preservatives, binders, and the like, and admixing them in a unit dosage form required for generally accepted pharmaceutical practice. The amount of an active ingredient in these formulations is set such that a suitable dosage within the indicated range is obtained.

Sterile compositions for injection may be formulated according to conventional prescriptions using a vehicle such as distilled water for injection.

Examples of the aqueous solution for injection include, for example, physiological saline, and isotonic solutions containing glucose and other adjuvants such as D-sorbitol, D-mannose, D-mannitol, sodium chloride, and the like. Appropriate solubilizing agents such as alcohols (such as ethanol), polyalcohols (such as propylene glycol, polyethylene glycol), nonionic surfactants (such as polysorbate 80 (TM), HCO-50) may be used in combination.

Examples of oily liquids include sesame oil and soybean oil, and benzyl benzoate and/or benzyl alcohol may be used as a solubilizing agent in combination with them. Further, a buffer (such as phosphate buffer and sodium acetate buffer), an analgesic (such as procaine hydrochloride), a stabilizer (such as benzyl alcohol and phenol), and an antioxidant may be blended. The prepared injection solution is usually filled into a suitable ampoule.

The pharmaceutical compositions of the present invention are preferably administered by parenteral administration. For example, the compositions may be in the form of an injection type, nasal administration type, pulmonary administration type, or transdermal administration type. For example, the compositions may be administered systemically or locally by intravenous injection, intramuscular injection, intraperitoneal injection, subcutaneous injection and the like.

The administration method may be appropriately selected depending on the patient's age and symptoms. The dose of a pharmaceutical composition comprising a polypeptide may be set, for example, into the range of 0.0001 mg to 1000 mg per kg body weight per dose. Alternatively, for example, the dose may be set to 0.001 to 100000 mg per patient, although the present invention is not necessarily limited to these figures. The dose and the administration method vary depending on the patient's body weight, age, symptoms, and the like, and those skilled in the art can determine an appropriate dose and an administration method taking account of those conditions.

Hereinafter, the present invention will be described in detail by way of the working examples, but it should be noted that these would not limit the scope of the present invention and merely illustrate the invention.

Abbreviations used herein have the following meanings:

MeOH: methanol
EtOH: ethanol

IPrOH: isopropanol or 2-propanol
DMF: N,N-dimethylformamide
MeCN: acetonitrile
THF: tetrahydrofuran
$CH_2Cl_2$: dichloromethane
NMR: nuclear magnetic resonance
MS: mass spectrometry
DMSO: dimethyl sulfoxide
$CDCl_3$: deuterated chloroform
Fe: iron
AcOH: acetic acid
$NH_3$: ammonia
TBAF: tetra-n-butylammonium fluoride
EDC/HCl: 1-(3-dimethyl-aminopropyl)-3-ethylcarbodiimide hydrochloride
HOBt•$H_2O$: 1-hydroxybenzotriazole hydrate
TEA: triethylamine
DMAP: 4-dimethylaminopyridine
$K_2S_2O_8$: potassium persulfate or potassium peroxodisulfate
$NH_4OAc$: ammonium acetate
NaH: sodium hydride
Pd/C: Palladium on carbon
TFA: trifluoroacetic acid
SNAP: disposable silica gel column
sfar silica HC D: disposable silica gel column
Presep: Disposable silica gel column

EXAMPLES

Example 1

5-((2-methoxyethoxy)carbonyl)-2,6-dimethyl-4-(2-nitrophenyl)-1,4-dihydropyridine-3-carboxylic acid (compound 2)

The title compound was prepared according to the synthetic steps in Scheme 1.

Scheme 1

[Chem. 27]

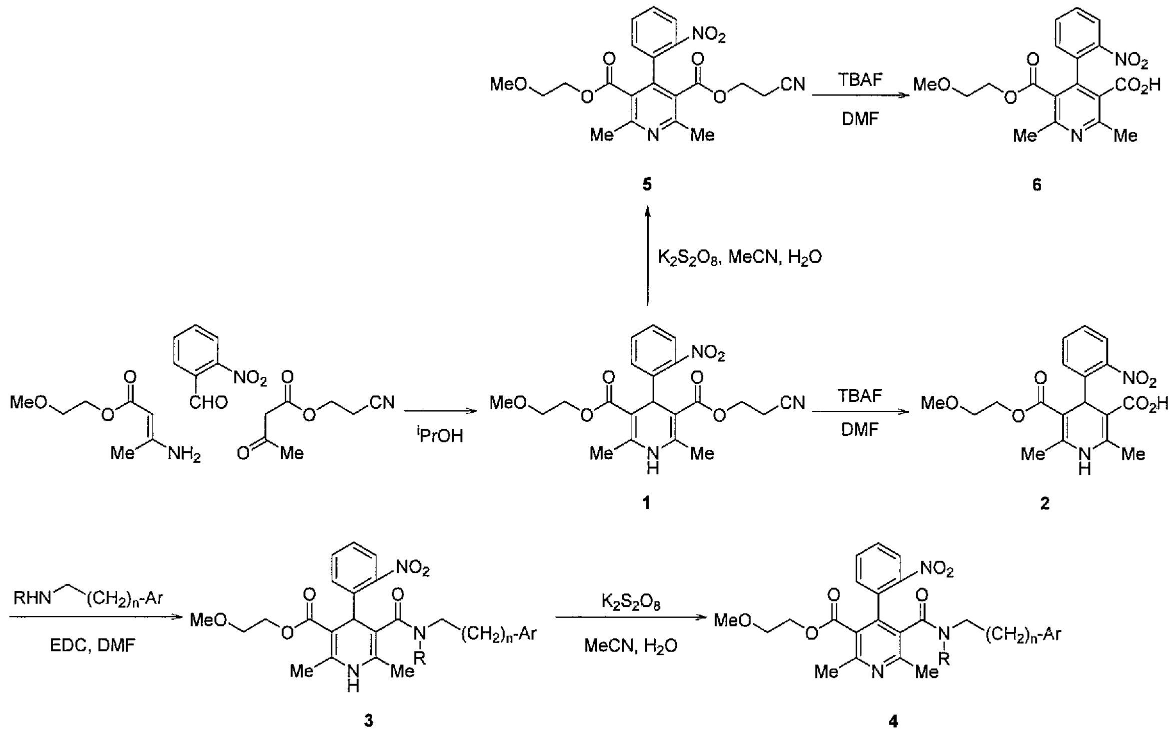


1-1: 2-Methoxyethyl-3-aminocrotonate

[Chem. 28]

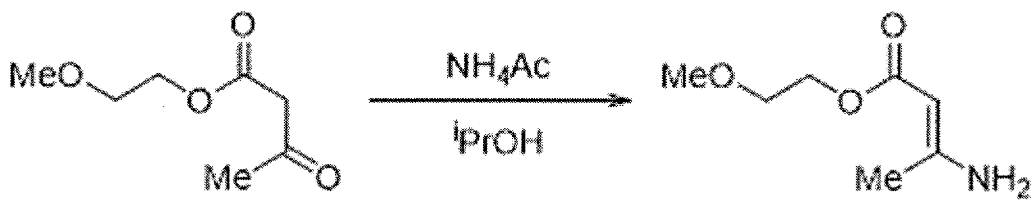


A solution of 2-methoxyethyl acetoacetate (TCI, 8 g, 50 mmol) and ammonium acetate (WAKO, 19.25 g, 250 mmol) in 2-propanol (150 mL) was stirred at 60°C for 4 hours under an argon atmosphere. After concentrating the reaction solution under reduced pressure, ethyl acetate (150 mL) was added thereto and the mixture was stirred. The mixture was dried over magnesium sulfate, filtered, and concentrated under reduced pressure, to provide 2-methoxyethyl-3-aminocrotonate (8.59 g, quant.) as a pale yellow oil.

$^1H$ NMR (500 MHz, $CDCl_3$): δ 1.87 (3H, s), 3.36 (3H, s), 3.57 (2H, t, J = 4.8 Hz), 4.18 (2H, dt, J = 5.0, 0.5 Hz), 4.55 (1H, s). MS (ESI, pos): m/z 160 $(M+H)^+$, 182 $(M+Na)^+$.

1-2: 2-Cyanoethyl 3-oxobutanoate

[Chem. 29]

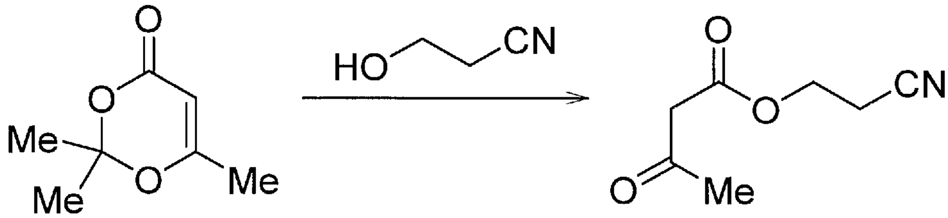

A solution of 3-hydroxypropionitrile (WAKO, 7.11 g, 100 mmol) and 2,2,6-trimethyl-1,3-dioxin-4-one (TCI, 15.6 g, 110 mmol) in toluene (100 mL) was reflux for 2 hours under an argon atmosphere. The reaction solution was cooled to room temperature, and then concentrated under reduced pressure. The residue was purified by medium-pressure column chromatography (Presep Si, 30 μm, 90 g, hexane:ethyl acetate = 88:12 → 22:78, 50 mL/min) to provide 2-cyanoethyl 3-oxobutanoate (15.4 g, 99%) as a yellow oil.

$^1$H NMR (500 MHz, $CDCl_3$): δ 2.26 (3H, s), 2.72 (2H, t, J = 6.3 Hz), 3.51 (3H, s), 4.33 (2H, t, J = 6.3 Hz). MS (ESI, pos): m/z 178 $(M + Na)^+$.

1-3: 3-(2-cyanoethyl) 5-(2-methoxyethyl) 2,6-dimethyl-4-(2-nitrophenyl)-1,4-dihydropyridine-3,5-dicarboxylate

(compound 1)

A solution of 2-nitrobenzaldehyde (TCI, 1.51 g, 10 mmol), 2-methoxyethyl-3-aminocrotonate (1.59 g, 10 mmol), and 2-cyanoethyl 3-oxobutanoate (1.55 g, 10 mmol) in 2-propanol (40 mL) was stirred for 3 hours at 70°C under an argon atmosphere. The precipitated solid was collected by filtration to provide compound 1 (2.74 g, 64%) as a yellow solid.

$^1$H NMR (500 MHz, DMF-$d_7$): δ 2.36 and 2.39 (3H, s), 2.92-2.94 (2H, m), 3.25 (3H, s), 3.50-3.58 (2H, m), 4.00-4.23 (2H, m), 4.13-4.32 (2H, m), 5.82 (1H, s), 7.41-7.43 (1H, m), 7.61-7.66 (2H, m), 7.80 (1H, dd, J = 8.5, 1.0 Hz), 9.14 (1H, m). MS (ESI, pos): m/z 430 $(M + H)^+$, 452 $(M + Na)^+$.

1-4: 5-((2-Methoxyethoxy)carbonyl)-2,6-dimethyl-4-(2-nitrophenyl)-1,4-dihydropyridine-3-carboxylic acid (compound 2)

1.0 M TBAF in THF (TCI, 12 mL, 12 mmol) was added to a solution of compound 1 (4.3 g, 10 mmol) in DMF (20 mL), and the mixture was stirred at room temperature for 4 hours under an argon atmosphere, followed by adding 1.0 M TBAF in THF (TCI, 10 mL, 10 mmol) thereto, and stirring the resulting mixture overnight at room temperature. After cooling to 0°C, 1N HCl (32 mL) was added, ethyl acetate (100 mL) and water (100 mL) were added, and the mixture was stirred. After separation, the organic layer was washed with water (100 mL) and saturated brine (100 mL). The aqueous layer was extracted with ethyl acetate (100 mL), and the organic layers were combined, dried over magnesium sulfate, and concentrated under reduced pressure. The concentrated residue was crystallized with diethyl ether, and the crystal was collected by filtration and dried to provide compound 2 (3.0 g, 80%) as a yellow solid.

$^1$H NMR (500 MHz, DMSO-$d_6$): δ 2.11 and 2.26 (3H, s), 3.14 (3H, s), 3.38-3.46 (2H, m), 3.91-4.09 (2H, m), 5.57 (1H, s), 7.33 (1H, dt, J = 7.7, 1.5 Hz), 7.46 (1H, dd, J = 8.0, 1.0 Hz), 7.57 (1H, dt, J = 7.7, 1.5 Hz), 7.68 (1H, dd, J = 8.0, 1.0 Hz), 8.82 (1H, s), 11.7 (1H, brd). MS (ESI, pos): m/z 399 $(M + Na)^+$.

In the following Examples 2-6, each example compound was prepared according to the following scheme using compound 2 prepared in Example 1 as a starting material.

[Chem. 30]

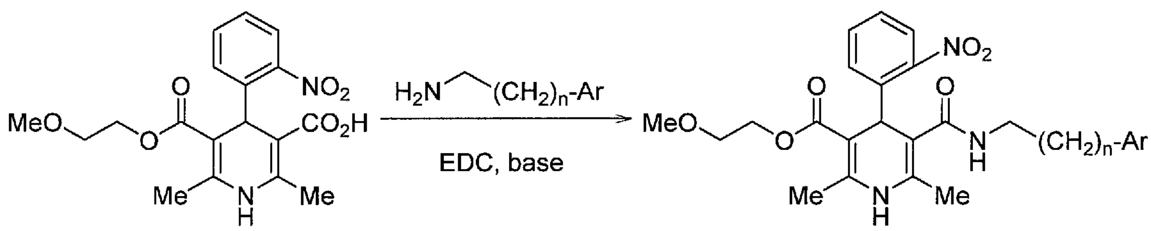


**2** **3a, 3b, 3c, 3d, 3f**

Example 2

2-Methoxyethyl 2,6-dimethyl-5-(methylcarbamoyl)-4-(2-nitrophenyl)-1,4-dihydropyridine-3-carboxylate (compound 3a)

A solution of compound 2 (377 mg, 1.0 mmol), DMAP (WAKO, 134 mg, 1.1 mmol), and EDC/HCl (WATANABE, 288 mg, 1.5 mmol) in dichloromethane (22 mL) was stirred at room temperature under an argon atmosphere. After 1 hour, methylamine in THF (2 mol/L, 1 mL) was added dropwise. After 23 hours, the stirring was stopped, ethyl acetate and water were added thereto, and the layers were separated. The aqueous layer was extracted with ethyl acetate. The organic layers were combined, washed with saturated brine, and dried over magnesium sulfate. After the filtrate was concentrated under reduced pressure, the residue was purified by medium pressure column chromatography (SNAP 25 μm, 25 g, chloroform:methanol = 100:0 → 99:1) to provide compound 3a (224 mg, 58%) as an orange solid.

$^1$H NMR (500 MHz, $CDCl_3$): δ 2.26 (3H, s), 2.25 (3H, s), 2.76 (3H, d), 3.33 (3H, s), 3.52 (2H, m), 3.97 (1H, m), 4.32 (1H, m), 5.54 (2H, s), 7.24 (1H, s), 7.29 (1H, t), 7.51 (1H, t), 7.58 (1H, d), 7.68 (1H, d) . MS (ESI, pos) : m/z not detected.

Example 3

2-Methoxyethyl 5-(dimethylcarbamoyl)-2,6-dimethyl-4-(2-nitrophenyl)-1,4-dihydropyridine-3-carboxylate (compound 3b)

A solution of compound 2 (377 mg, 1.0 mmol), dimethylamine hydrochloride (TCI, 82 mg, 1.0 mmol), and triethylamine (320 mg, 3.2 mmol) in DMF (10 mL) was stirred at 0°C under an argon atmosphere. EDC/HCl (WATANABE, 211 mg, 1.1 mmol) and HOBt/$H_2O$ (WATANABE, 184 mg, 1.2 mmol) were added thereto, and after stirring for 2 hours, the mixture was returned to room temperature and stirred overnight. Ethyl acetate (50 mL) and water (50 mL) were used to separate the layers, the aqueous layer was further extracted with ethyl acetate (80 mL), and then the organic layers were combined and washed with saturated brine (50 mL). After drying over magnesium sulfate and concentrating under reduced pressure, the resultant residue was purified by medium pressure column chromatography (SNAP 25 μm, 25 g, chloroform:methanol = 100:0 → 97:3) to provide compound 3b (274 mg, 68%) as a yellow solid.

$^1$H NMR (500 MHz, $CDCl_3$): δ 1.84 (3H, s), 2.37 (3H, s), 2.94 (3H, s), 3.17-3.29 (6H, m), 3.37 (2H, t), 3.99 (1H, m), 4.07 (1H, m), 5.33 (1H, s), 5.37 (1H, s), 7.23 (1H, d), 7.51 (1H, t), 7.60 and 7.62 (1H, d), 7.67 and 7.68 (1H, d). MS (ESI, pos): m/z 487 $(M + Na)^+$.

Example 4

2-Methoxyethyl 5-(benzylcarbamoyl)-2,6-dimethyl-4-(2-nitrophenyl)-1,4-dihydropyridine-3-carboxylate (compound 3c)

A solution of compound 2 (377 mg, 1.0 mmol), benzylamine (TCI, 110 μL, 1.0 mmol), and triethylamine (TCI, 202 mg, 2.0 mmol) in DMF (10 mL) was cooled to 0°C under an argon atmosphere, then EDC/HCl (WATANABE, 211 mg, 1.1 mmol) and HOBt/$H_2O$ (WATANABE, 184 mg, 1.2 mmol) were added thereto, and the mixture was stirred for 2 hours. The mixture was returned to room temperature, and further stirred overnight. Water (80 mL) and ethyl acetate (80 mL) were used to separate the layers, and the organic layer was washed with water (80 mL) and saturated brine (80 mL). The aqueous layer was extracted with ethyl acetate (80 mL), and the organic layers were combined, dried over magnesium sulfate, and concentrated under reduced pressure. The residue was purified by medium pressure column chromatography (SNAP 25 μm, 10 g, hexane:ethyl acetate = 84:16 → 20:80) to provide compound 3c (288 mg, 62%) as a yellow solid.

$^1$H NMR (500 MHz, DMSO-$d_6$): δ 2.01 (3H, s), 2.21 (3H, s), 3.14 (3H, s), 3.30-3.40 (2H, m), 3.82-4.05 (2H, m), 4.15 (1H, dd, J = 15, 6.0 Hz), 4.30 (1H, dd, J = 15, 6.0 Hz), 5.43 (1H, s), 7.01-7.02 (2H, m), 7.14-7.21 (3H, m), 7.35-7.38 (1H, m), 7.54 (1H, dd, J = 8.0, 1.5 Hz), 7.61-7.65 (1H, m), 7.70 (1H, dd, J = 8.0, 1.5 Hz), 7.99 (1H, brd), 8.52 (1H, brd). MS (ESI, pos): m/z 488 $(M + Na)^+$.

Example 5

2-Methoxyethyl 2,6-dimethyl-4-(2-nitrophenyl)-5-((pyridin-4-ylmethyl)carbamoyl)-1,4-dihydropyridine-3-carboxylate

(compound 3d)

A solution of compound 2 (377 mg, 1.0 mmol), 4-(aminomethyl)pyridine (sigma-Aldrich, 108 mg, 1.0 mmol) and triethylamine (200 mg, 2.0 mmol) in DMF (10 mL) was stirred at 0°C under an argon atmosphere. EDC/HCl (WATANABE, 211 mg, 1.1 mmol) and HOBt/$H_2O$ (WATANABE, 184 mg, 1.2 mmol) were added thereto and the mixture was

stirred for 2 hours. After returning to room temperature, the mixture was stirred overnight. Ethyl acetate (80 mL) and water (80 mL) were used to separate the layers, the aqueous layer was further extracted with ethyl acetate (80 mL), and then the organic layers were combined and washed with saturated brine (50 mL) . After drying over magnesium sulfate and concentrating under reduced pressure, the concentrated residue was purified by medium pressure column chromatography (SNAP 25 μm, 25 g, chloroform:methanol = 98:2→95:5). The resultant material was involved with a spot corresponding to any impurity, and therefore the material was again purified by medium pressure column chromatography (SNAP 25 μm, 35 g, chloroform:methanol = 98:2 → 95:5) to provide compound 3d (147 mg, 31%) as a yellow oil.

$^1H$ NMR (500 MHz, $CDCl_3$): δ 2.27 (3H, s), 2.51 (3H, s), 3.32 (3H, s), 3.45-3.58 (2H, m), 3.94-3.99 (1H, m), 4.29-4.34 (2H, m), 4.53-4.58 (1H, dd), 5.64 (1H, s), 5.66 (1H,s), 6.89 and 6.91 (2H, d), 7.34 (1H, t), 7.58 (2H, t), 7.63-7.65 (2H, d), 7.91 (1H, t), 8.35 and 8.36 (2H, d). MS (ESI, pos): m/z 467 $(M + H)^+$, 489 $(M + Na)^+$.

Example 6

2-Methoxyethyl 2,6-dimethyl-4-(2-nitrophenyl)-5-((3-(pyridin-4-yl)propyl)carbamoyl)-1,4-dihydropyridine-3-carboxylate (compound 3f)

A solution of compound 2 (377 mg, 1 mmol), 3-(pyridin-4-yl)propan-1-amine (BLD pharm, 136 mg, 1.0 mmol) and triethylamine (TCI, 200 mg, 2.0 mmol) in DMF (10 mL) were cooled to 0°C under an argon atmosphere, then EDC/HCl (WATANABE, 211 mg, 1.1 mmol), HOBt/$H_2O$ (WATANABE, 184 mg, 1.2 mmol) were added thereto, and the mixture was stirred for 2 hours. The mixture was returned to room temperature and stirred overnight. Water (20 mL) and ethyl acetate (20 mL) were used to separate the layers, and the organic layer was washed with water (20 mL) and saturated brine (20 mL). The aqueous layer was extracted with ethyl acetate (20 mL), and the organic layers were combined, dried over magnesium sulfate, and concentrated under reduced pressure. The residue was purified by medium pressure column chromatography (SNAP 25 μm, 10 g, chloroform:methanol = 100:0 → 98:2), so that the desired fractions were collected and concentrated under reduced pressure. Further the concentrated residue was purified by medium pressure column chromatography (SNAP 25 μm, 25 g, chloroform:methanol = 100:0 → 96:4), so that the desired fractions were collected and concentrated under reduced pressure to provide compound 3f (164 mg, 33%) as a yellow solid.

$^1H$ NMR (500 MHz, $CDCl_3$): δ 1.71-7.80 (2H, m), 2.30 (3H, s), 2.43-2.49 (2H, m), 2.51 (3H, s), 3.19-3.24 (2H, m), 3.33(3H, s), 3.47-3.62 (2H, m), 3.96-4.02 (2H, m), 4.30-4.35 (2H, m), 5.57 (1H, s), 5.78 (1H, brd), 7.03 (2H, dd, J = 4.5, 1.5 Hz), 7.27-7.30 (1H, m), 7.34 (1H, brd), 7.53 (1H, dt, J = 8, 1.5 Hz), 7.60 (1H, dd, J = 8, 1.5 Hz), 7.68 (1H, dd, J = 8, 1.5 Hz), 8.43 (1H, dd, J = 4.5, 1.5 Hz). MS (ESI, pos) : m/z 495 $(M + H)^+$, 517 $(M + Na)^+$.

In the following Examples 7 to 10, each example compound was prepared according to the following scheme using the example compounds prepared above as a starting material.

[Chem. 31]

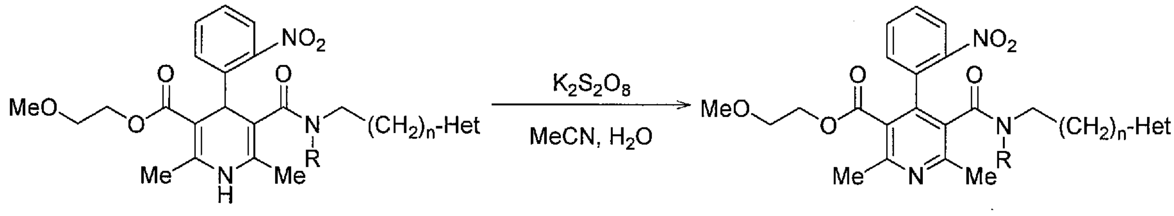


**3a, 3c, 3d, 3f** **4a, 4c, 4d, 4f**

Example 7

2-Methoxyethyl 2,6-dimethyl-5-(methylcarbamoyl)-4-(2-nitrophenyl)nicotinate (compound 4a)

A solution of compound 3a prepared in Example 2 (164 mg, 0.42 mmol) and potassium peroxodisulfate (Nacalai, 340 mg, 1.26 mmol) in acetonitrile/water (2:1, 12.6 mL) was stirred at room temperature under an argon atmosphere. After 1 hour, the solution was heated at 100°C, after 5 hours, the stirring was stopped, and ethyl acetate and water were added thereto to separate the layers. The aqueous layer was extracted with ethyl acetate. The organic layers were combined, washed with saturated brine, and dried over magnesium sulfate. After concentration under reduced pressure, the residue was purified by medium pressure column chromatography (SNAP 25 μm, 10 g, chloroform:methanol = 100:0 → 98.9:1.1) to provide compound 4a (30 mg, 18%) as a colorless solid.

$^1H$ NMR (500 MHz, $CDCl_3$): δ 2.59 (3H, d), 2.62 (3H, s), 3.25 (3H, s), 3.34 (2H, m), 4.04 (1H, m), 4.10 (1H, m),

5.98 (1H, s), 7.33 (1H, d), 7.56 (1H, t), 7.65 (1H, t), 8.07 (1H, d). MS (ESI, pos): m/z 410 $(M + Na)^+$.

Example 8

2-Methoxyethyl 5-(benzylcarbamoyl)-2,6-dimethyl-4-(2-nitrophenyl)nicotinate (compound 4c)

A solution of compound 3c prepared in Example 4 (220 mg, 0.47 mmol) and potassium peroxodisulfate (nacalai, 384 mg, 1.42 mmol) in acetonitrile/water (2:1, 15 mL) was stirred at 100°C for 2 hours under an argon atmosphere. After returning to room temperature, the solution was separated with water (30 mL) and ethyl acetate (30 mL), and the organic layer was washed with water (30 mL) and saturated brine (30 mL). The aqueous layer was extracted with ethyl acetate (30 mL), and the organic layers were combined, dried over magnesium sulfate, and concentrated under reduced pressure. The residue was purified by medium pressure column chromatography (SNAP 25 $\mu$m, 10 g, chloroform:methanol = 99.9:0.1 $\rightarrow$ 98:2) to provide compound 4c (64 mg, 29%) as a yellow solid.

$^1$H NMR (500 MHz, $CDCl_3$): $\delta$ 2.61 (3H, s), 2.67 (3H, s), 3.24 (3H, s), 3.28-3.38 (2H, m), 3.99-4.11 (3H, m), 4.47 (1H, dd, J = 14.5, 7.5 Hz), 6.42 (1H, brd), 6.78-6.80 (2H, m), 7.13-7.21 (3H, m), 7.30 (1H, dd, J = 7.5, 1.0 Hz), 7.45-7.49 (1H, m), 7.60 (1H, dt, J = 7.5, 1.0 Hz), 7.82 (1H, dd, J = 7.5, 1.0 Hz). MS (ESI, pos): m/z 464 $(M + H)^+$, 486 $(M + Na)^+$.

Example 9

2-Methoxyethyl 2,6-dimethyl-4-(2-nitrophenyl)-5-((pyridin-4-ylmethyl)carbamoyl)nicotinate (compound 4d)

A solution of compound 3d prepared in Example 5 (117 mg, 0.25 mmol) and potassium peroxodisulfate (Nacalai, 203 mg, 0.75 mmol) in acetonitrile/water (2:1, 7.5 mL) was refluxed at 80°C for 1 hour. The stirring was stopped, 1N NaOH was added thereto until the pH reached 7, and the mixture was separated with ethyl acetate (50 mL) and water (50 mL). The aqueous layer was extracted with ethyl acetate (50 mL), and the organic layers were combined and washed with saturated brine (50 mL). After drying over magnesium sulfate, the combination was concentrated under reduced pressure, and the residue was purified by medium pressure column chromatography (SNAP 10 $\mu$m, 10 g, chloroform:methanol = 100:0 $\rightarrow$ 96:4) to provide compound 4d (50 mg, 43%) as a white solid.

$^1$H NMR (500 MHz, $CDCl_3$): $\delta$ 2.62 (3H, s), 2.66 (3H, s), 3.24 (3H, s), 3.29-3.38 (2H, m), 4.00-4.04 (2H, m), 4.08-4.12 (1H, m), 4.59-4.63 (1H, dd), 6.64 (1H, t), 6.76 and 6.77 (2H, d), 7.30 and 7.31 (1H, d), 7.54 (1H, t), 7.62 (1H, t), 7.87 and 7.89 (1H, d), 8.37 and 8.38 (2H, d) . MS (ESI, pos): m/z 487 $(M + Na)^+$.

Example 10

2-Methoxyethyl 2,6-dimethyl-4-(2-nitrophenyl)-5-((3-(pyridin-4-yl)propyl)carbamoyl)nicotinate (compound 4f)

A solution of compound 3f prepared in Example 6 (126 mg, 0.25 mmol) and potassium peroxodisulfate (Nacalai, 206 mg, 0.76 mmol) in acetonitrile/water (2:1, 7 mL) was stirred at 80°C for 1.5 hours under an argon atmosphere. After returning to room temperature, 1N NaOH (350 uL) was added thereto, the mixture was separated with water (40 mL) and ethyl acetate (40 mL), and the organic layer was washed with water (40 mL) and saturated brine (40 mL). The aqueous layer was extracted with ethyl acetate (40 mL), and the organic layers were combined, dried over magnesium sulfate, and concentrated under reduced pressure. The residue was purified by medium pressure column chromatography (SNAP 25 $\mu$m, 10 g, chloroform:methanol = 100:0 $\rightarrow$ 96:4) to provide compound 4f (43 mg, 34%) as pale yellowish white oil.

$^1$H NMR (500 MHz, $CDCl_3$): $\delta$ 1.37 (2H, m), 2.31 (2H, t, J = 7.5 Hz), 2.63 (3H, s), 2.64 (3H, s), 2.95-3.02 (1H, m), 3.21-3.26 (4H, m), 3.31-3.39 (2H, m), 4.02-4.14 (2H, m), 6.18 (1H, brd), 6.97 (2H, d, J = 5.0 Hz), 7.35 (1H, dd, J = 8.0, 1.0 Hz), 7.46-7.49 (1H, m), 7.63 (1H, dt, J = 8.0, 1.0 Hz), 8.00 (1H, dd, J = 8.0, 1.0 Hz), 8.48 (2H, d, J = 5.0 Hz). MS (ESI, pos): m/z 493 $(M + H)^+$, 515 $(M + Na)^+$.

Example 11

2-Methoxyethyl 2,6-dimethyl-4-(2-nitrophenyl)-5-((2-(pyridin-4-yl)ethyl)carbamoyl)nicotinate (compound 4e)

[Chem. 32]

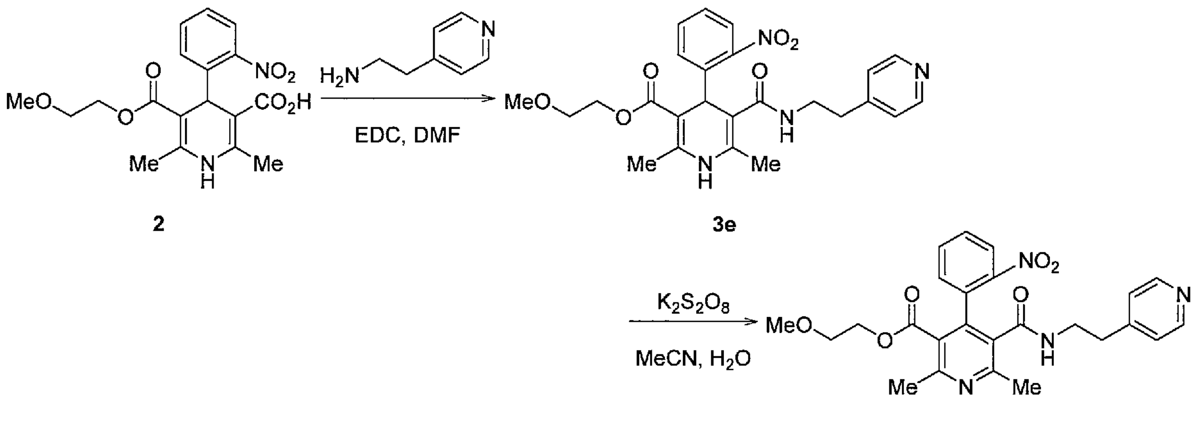


2 3e 4e

A solution of compound 2 prepared in Example 1 (377 mg, 1.0 mmol), 4-(2-aminoethyl)pyridine (TCI, 122 mg, 1.0 mmol), and triethylamine (WAKO, 200 mg, 2.0 mmol) in DMF (10 mL) was cooled to 0°C under an argon atmosphere. HOBt/$H_2O$ (WATANABE, 184 mg, 1.2 mmol) and EDC/HCl (WATANABE, 211 mg, 1.1 mmol) were added thereto, and the mixture was stirred at 0°C for 2 hours and stirred at room temperature overnight. Water (80 mL) and ethyl acetate (80 mL) were added to the reaction solution, and after separating the layers, the organic layer was washed with saturated brine. The aqueous layer was extracted with ethyl acetate, and the organic layers were combined and dried over magnesium sulfate. After concentration under reduced pressure, the residue was purified by medium pressure column chromatography (SNAP 25 μm, 25 g, chloroform:methanol = 100:0 → 93.1:6.9) to provide a brown amorphous (358 mg) . MS (ESI, pos) confirmed compound 3e: m/z 481 $(M + H)^+$, and compound 4e: m/z 479 $(M + Na)^+$, and therefore the crude product was used as it is in the next reaction. A solution of the crude product (358 mg) and potassium peroxodisulfate (Nacalai, 608 mg, 2.25 mmol) in acetonitrile/water (2:1, 12.6 mL) was stirred at 80°C for 1.5 hours under an argon atmosphere. Water and ethyl acetate (80 mL) were added to the reaction mixture, and after separating the layers, the organic layer was washed with saturated brine. The aqueous layer was extracted with ethyl acetate. The organic layers were combined, dried over magnesium sulfate, and concentrated under reduced pressure. The residue was purified by medium pressure column chromatography (SNAP 25 μm, 10 g, chloroform:methanol = 100:0 → 96:4) to provide compound 4e (135 mg, 38%) as a brown amorphous.

$^1H$ NMR (500 MHz, $CDCl_3$): δ 2.45 (2H, m), 2.58 (3H, m), 2.60 (3H, s), 3.24 (3H, s), 3.30 (4H, m), 4.09 (2H, m), 6.19 (1H, t), 6.98 (2H, d), 7.30 (1H, d), 7.52 (1H, t), 7.62 (1H, t), 7.92 (1H, d), 8.45 (2H, d). MS (ESI, pos): m/z 479 $(M + H)^+$, 501 $(M + Na)^+$.

Example 12

5-((2-methoxyethoxy)carbonyl)-2,6-dimethyl-4-(2-nitrophenyl)nicotinic acid (compound 6)

[Chem. 33]

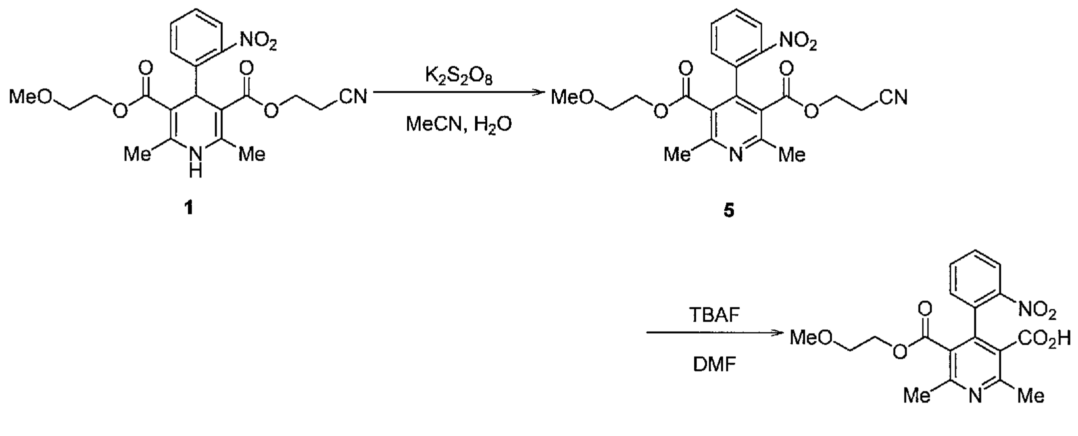


1

5

6

12-1: 3-(2-cyanoethyl) 5-(2-methoxyethyl) 2,6-dimethyl-4-(2-nitrophenyl)pyridine-3,5-dicarboxylate (compound 5)

A solution of compound 1 prepared in Example 1-3 (858 mg, 2.0 mmol) and potassium peroxodisulfate (Nacalai, 1.62 g, 6.0 mmol) in acetonitrile/water (2:1, 60 mL) was stirred at 80°C for 1 hour under an argon atmosphere. After returning to room temperature, 1N NaOH (8.5 mL) was added to the solution, the mixture was separated with water (80 mL) and ethyl acetate (80 mL), and the organic layer was washed with water (80 mL) and saturated brine (80 mL). The aqueous layer was extracted with ethyl acetate (80 mL), and the organic layers were combined, dried over magnesium sulfate, and concentrated under reduced pressure. The residue was purified by medium pressure column chromatography (SNAP 25 um, chloroform:methanol = 100:0 → 99.8:0.2), so that the desired fractions were collected and the concentrated under reduced pressure to provide compound 5 (570 mg, 67%) as a yellow oil.

$^1H$ NMR (500 MHz, $CDCl_3$): δ 2.35-2.49 (2H, m), 2.68 (6H, s), 3.24 (3H, s), 3.28-3.13 (2H, m), 3.97-4.03 (1H, m), 4.07-4.14 (2H, m), 4.16-4.20 (1H, m), 7.25 (1H, dd, J = 7.5, 1.5 Hz), 7.60 (1H, dt, J = 7.5, 1.5 Hz), 7.67 (1H, dt, J = 7.5, 1.5 Hz), 8.22 (1H, dd, J = 7.5, 1.5 Hz). MS (ESI, pos): m/z 450 $(M + H)^+$.

12-2: 5-((2-methoxyethoxy)carbonyl)-2,6-dimethyl-4-(2-nitrophenyl)nicotinic acid (compound 6)

To a solution of compound 5 prepared in Example 12-1 (520 mg, 1.21 mmol) in THF (2 mL), 1.0 M TBAF in THF (TCI, 1.45 mL, 1.45 mmol) was added and the mixture was stirred overnight at room temperature under an argon atmosphere, followed by adding more 1.0 M TBAF in THF (TCI, 1.21 mL, 1.21 mmol) thereto, and stirring the mixture for 2 hours. After adding saturated aqueous sodium bicarbonate, the mixture was separated with ethyl acetate (40 mL) and water (40 mL), and the organic layer was washed with water (40 mL) and saturated brine (40 mL) . The aqueous layer was extracted with ethyl acetate (40 mL), and the organic layers were combined, dried over magnesium sulfate, and concentrated under reduced pressure. The residue was purified by medium pressure column chromatography (SNAP 25 μm, 10 g, hexane:ethyl acetate = 90:10 → 0:100) to provide compound 6 (164 mg, 36%) as a colorless solid.

$^1H$ NMR (500 MHz, DMSO-$d_6$): δ 2.56 (6H, s), 3.10 (3H, s), 3.31-3.24 (2H, m), 3.92-4.06 (2H, m), 7.27 (1H, dd, J = 8.0, 1.5 Hz), 7.71 (1H, dt, J = 7.8, 1.5 Hz), 7.79 (1H, dt, J = 7.78, 1.5 Hz), 8.26 (1H, dd, J = 8.0, 1.5 Hz), 13.3 (1H, brd). MS (ESI, pos): m/z 375 $(M + H)^+$, 397 $(M + Na)^+$.

Example 13

2-Methoxyethyl 2,4-dimethyl-5-oxo-5,6-dihydrobenzo[c][2,7]naphthyridine-1-carboxylate (compound 9)

The title compound was prepared according to the synthetic steps in Scheme 2.

Scheme 2

[Chem. 34]

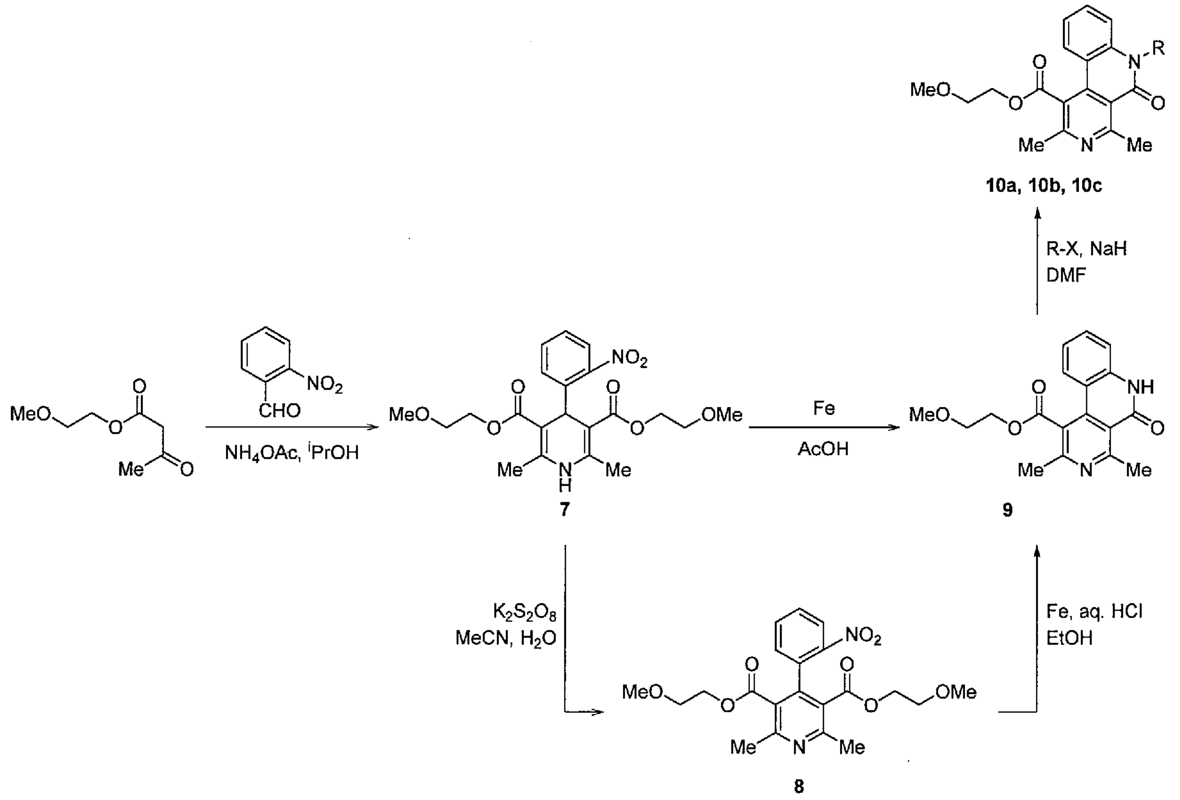


13-1: Bis(2-methoxyethyl) 2,6-dimethyl-4-(2-nitrophenyl)-1,4-dihydropyridine-3,5-dicarboxylate (compound 7)

A solution of 2-nitrobenzaldehyde (TCI, 3.03 g, 20 mmol), 2-methoxyethyl 3-oxobutanoate (TCI, 6.0 mL, 41 mmol), and ammonium acetate (WAKO, 2.42 g, 31 mmol) in 2-propanol (100 mL) was heated with stirring at 80°C for 24 hours under an argon atmosphere. The mixture was cooled to room temperature, diluted with ethyl acetate (200 mL), and the dilution was washed with water (50 mL × 2) and saturated brine (50 mL ×2) . The organic layer was dried over sodium sulfate, filtration, and concentrated under reduced pressure. The residue was washed with a diisopropyl ether (30 mL)/dichloromethane (3 mL) solution to provide compound 7 (5.42 g, 62%) as a yellow solid.

$^1$H NMR (500 MHz, $CDCl_3$): δ 2.32 (6H, s), 3.28 (6H, s), 3.48-3.58 (4H, m), 4.02-4.07 (2H, m), 4.22-4.27 (2H, m), 5.64 (1H, s), 5.88 (1H, s), 7.23-7.26 (1H, m), 7.43-7.47 (1H, m), 7.52 (1H, dd, J = 8.0, 1.4 Hz), 7.73 (1H, dd, J = 8.0, 1.4 Hz). MS (ESI, pos): m/z 435 $(M + H)^+$, 457 $(M + Na)^+$.

13-2: Bis(2-methoxyethyl) 2,6-dimethyl-4-(2-nitrophenyl)pyridine-3,5-dicarboxylate (compound 8)

A solution of compound 7 prepared in Example 13-1 (3.91 g, 9.0 mmol) and potassium peroxodisulfate (Nacalai, 7.30 g, 27.0 mmol) in acetonitrile/water (2:1, 30 mL) was reflux at 80°C. After 20 minutes, the mixture was cooled to room temperature, acetonitrile was distilled off under reduced pressure, diluted with water, and the dilution was extracted with ethyl acetate (50 mL × 3). The extract was washed with water (50 mL × 2) and saturated brine (50 mL), and dried over sodium sulfate. After filtration and concentration under reduced pressure, the residue was purified by medium pressure column chromatography (SNAP 25 μm, 35 g, hexane/ethyl acetate = 83/17 → 50/50 → 30/70) to provide compound 8 (2.42 g, 62%) as a yellow solid.

$^1$H NMR (500 MHz, $CDCl_3$): δ 2.67 (6H, s), 3.24 (6H, s), 3.28-3.31 (4H, m), 3.97-4.02 (2H, m), 4.08-4.13 (2H, m), 5.64 (1H, s), 5.88 (1H, s), 7.22 (1H, d, J = 7.7 Hz), 7.55-7. 64 (2H, m), 8.24 (1H, d, J = 8.1 Hz). MS (ESI, pos): m/z 433 $(M + H)^+$, 455 $(M + Na)^+$.

13-3: 2-Methoxyethyl 2,4-dimethyl-5-oxo-5,6-dihydrobenzo[c][2,7]naphthyridine-1-carboxylate (compound 9)

13-3A:

A solution of compound 8 prepared in Example 13-2 (2.92 g, 6.8 mmol), iron powder (WAKO, 1.44 g, 25.8 mmol), and 36% hydrochloric acid (2.0 mL) in ethanol (50 mL) was refluxed for 14 hours. After cooling to room temperature, the mixture was neutralized with 1N NaOH, and filtered through celite with chloroform. The filtrate was washed with water (50 mL × 2) and saturated brine (50 mL), and dried over sodium sulfate. After filtration and concentration under reduced pressure, the residue was purified by medium pressure column chromatography (SNAP 25 μm, 10 g, chloroform/methanol = 100/0 → 99/1), followed by washing the resultant solid with diisopropyl ether to provide compound 9 (851 mg, 38%) as a white solid.

$^1H$ NMR (500 MHz, $CDCl_3$): δ 2.70 (3H, s), 3.20 (3H, s), 3.36 (3H, s), 3.70-3.72 (2H, m), 4.61-4.63 (2H, m), 7.17-7.21 (1H, m), 7.24 (1H, dd, J = 8.1, 0.9 Hz), 7.52-7.56 (1H, m), 7.97 (1H, d, J = 8.3 Hz). MS (ESI, pos): m/z 327 $(M + H)^+$.

13-3B:

Iron powder (WAKO, 1.54 g, 27.5 mmol) was added to a solution of compound 7 (217mg, 0.5 mmol) prepared in Example 13-1 in acetic acid (8 mL), and the mixture was stirred at 100°C. After 3 hours, ethyl acetate was added, and the mixture was stirred for 1 hour. The mixture was filtered through celite with ethyl acetate, and 10N NaOH (15 mL) was added under an ice bath to separate the layers. The aqueous layer was extracted with ethyl acetate. The organic layers were combined, washed with saturated brine, and dried over magnesium sulfate, and concentrated under reduced pressure. The residue was purified by medium pressure column chromatography (SNAP 25 μm, 10 g, hexane/ethyl acetate = 88/12 → 0/100) to provide compound 9 (224.1 mg, 20%) as a yellow solid.

$^1H$ NMR (500 MHz, $CDCl_3$): δ 2.79 (3H, s), 3.21 (3H, s), 3.36 (3H, s), 3.71(2H, m), 4.62 (2H, m), 7.18 (1H, m), 7.28 (1H, m), 7.53 (1H, m), 7.98 (1H, m), 10.7 (1H, s). MS (ESI, pos): m/z 327 $(M + H)^+$.

In Examples 14 to 16 below, each example compound was prepared according to the following scheme using compound 9 prepared in Example 13 as a starting material.

[Chem. 35]

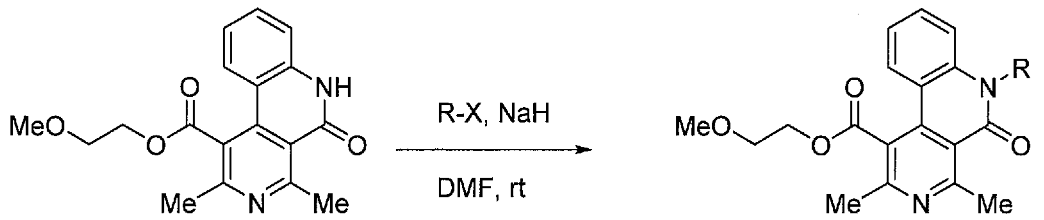


9

10a, 10b, 10c

Example 14

2-Methoxyethyl 2,4,6-trimethyl-5-oxo-5,6-dihydrobenzo[c][2,7]naphthyridine-1-carboxylate (compound 10a)

After sodium hydride (WAKO, 60% in oil, 10.3 mg, 0.43 mmol) was added to an ice-cooled solution of compound 9 (98.9 mg, 0.30 mmol) in DMF (5.0 mL) under an argon atmosphere, the mixture was stirred at room temperature for 30 minutes. The mixture was ice-cooled again, methyl iodide (TCI, 40 μL, 0.64 mmol) was added, and the mixture was stirred at room temperature for 30 minutes. After further addition of methyl iodide (40 μL, 0.64 mmol), the disappearance of the starting material was confirmed, water (10 mL) was added, and the mixture was extracted with ethyl acetate (10 mL × 3). The organic layer was washed with water (10 mL × 3) and saturated brine (10 mL), and dried over sodium sulfate. After filtration and concentration under reduced pressure, the residue was purified by medium pressure column chromatography (SNAP 25 μm, 10 g, hexane/ethyl acetate = 100/0 → 50/50) to provide compound 10a (110 mg, quant.) as a white solid.

$^1H$ NMR (500 MHz, $CDCl_3$): δ 2.68 (3H, s), 3.14 (3H, s), 3.34 (3H, s), 3.675 (2H, m), 3.73 (3H, s), 4.57 (2H, m), 7.21 (1H, m), 7.39 (1H, dd, J = 8.5, 0.8 Hz), 7.60 (1H, m), 8.01 (1H, dd, J = 8.3, 1.3 Hz). MS (ESI, pos): m/z 341 $(M + H)^+$, 363 $(M + Na)^+$.

Example 15

2-Methoxyethyl 6-benzyl-2,4-dimethyl-5-oxo-5,6-dihydrobenzo[c][2,7]naphthyridine-1-carboxylate (compound 10b)

To an ice-cooled solution of compound 9 (98.3 mg, 0.30 mmol) in DMF (5.0 mL) was added sodium hydride (WAKO, 60% in oil, 10.5 mg, 0.43 mmol) under an argon atmosphere, the mixture was stirred at room temperature for 30 minutes. After the mixture was ice-cooled again, benzyl bromide (WAKO, 72 μL, 0.60 mmol) was added thereto, and the mixture was stirred at room temperature for 30 minutes. After 1 hour, the disappearance of the starting materials was confirmed, water (10 mL) was added thereto, and the mixture was extracted with ethyl acetate (10 mL × 3). The organic layer was washed with water (10 mL × 3) and saturated brine (10 mL), and dried over sodium sulfate. After filtration and concentration under reduced pressure, the residue was purified by medium pressure column chromatography (SNAP 25 μm, 10 g, hexane/ethyl acetate = 100/0 → 65/35) to provide compound 10b (111 mg, 89%) as a yellow oil.

$^1$H NMR (500 MHz, $CDCl_3$): δ 2.70 (3H, s), 3.17 (3H, s), 3.34 (3H, s), 3.69 (2H, m), 4.59 (2H, m), 5.58 (2H, s), 7.16 (1H, m), 7.23 (2H, m), 7.26 (2H, m), 7.31 (2H. m), 7.44 (1H, m), 8.02 (1H, dd, J = 8.3, 1.3 Hz), 8.55 (2H, d, J = 5.0 Hz). MS (ESI, pos): m/z 417 $(M + H)^+$, 439 $(M + Na)^+$.

Example 16

2-Methoxyethyl 2,4-dimethyl-5-oxo-6-(pyridin-4-ylmethyl)-5,6-dihydrobenzo[c] [2,7]naphthyridine-1-carboxylate (compound 10c)

16A:

To an ice-cooled solution of compound 9 (98.5 mg, 0.30 mmol) and 4-pyridylmethyl chloride (WAKO, 62.3 mg, 0.37 mmol) in DMF (5.0 mL) was added sodium hydride (WAKO, 60% in oil, 19.3 mg, 0.80 mmol) under an argon atmosphere, and then the mixture was stirred at room temperature for 11 hours. Water (10 mL) was added thereto, and the mixture was extracted with ethyl acetate (10 mL × 3). The organic layer was washed with water (10 mL × 3) and saturated brine (10 mL), and dried over sodium sulfate. After filtration and concentration under reduced pressure, the residue was purified by medium pressure column chromatography (SNAP 25 μm, 10 g, hexane/ethyl acetate = 75/25 → 0/100) to provide compound 10c (35.1 mg, 28%) as a white solid.

$^1$H NMR (500 MHz, $CDCl_3$): δ 2.71 (3H, s), 3.15 (3H, s), 3.35 (3H, s), 3.67-3.71 (2H, m), 4.58-4.62 (2H, m), 5.56 (2H, brd), 7.09 (1H, d, J = 8.5 Hz), 7.14 (2H, d, J = 5.7 Hz), 7.17-7.23 (1H, m), 7.43-7.48 (1H, m), 8.06 (1H, dd, J = 8.3, 1.2 Hz). MS (ESI, pos): m/z 418 $(M + H)^+$, 440 $(M + Na)^+$.

16B:

To an ice-cooled solution of compound 9 (61.2 mg, 0.19 mmol) and 4-pyridylmethyl chloride (WAKO, 37.5 mg, 0.22 mmol) in DMF (3.0 mL), triethylamine (67 μL) was added under ice-cooling under an argon atmosphere, and the mixture was stirred at room temperature for 3 hours. Then, sodium hydride (WAKO, 60% in oil, 12.2 mg, 0.51 mmol) was added thereto under ice-cooling, and the mixture was stirred at room temperature for 1 hour. Furthermore, under ice-cooling, sodium hydride (WAKO, 60% in oil, 12.5 mg, 0.52 mmol) and 4-pyridylmethyl chloride (WAKO, 38.3 mg, 0.22 mmol) were added thereto and the mixture was stirred at room temperature for 2 hours. Furthermore, under ice-cooling, sodium hydride (WAKO, 60% in oil, 14.2 mg, 0.59 mmol) and 4-pyridylmethyl chloride (WAKO, 39.2 mg, 0.24 mmol) were added thereto and the mixture was stirred overnight. Water (10 mL) was added thereto, and the mixture was extracted with ethyl acetate (10 mL × 3). The organic layer was washed with water (10 mL × 3) and saturated brine (10 mL), and dried over sodium sulfate, filtration, concentration under reduced pressure. The residue was purified by medium pressure column chromatography (SNAP 25 μm, 10 g, hexane/ethyl acetate = 75/25 → 0/100) to provide compound 10c (41.9 mg, 53%) as a white solid.

$^1$H NMR (500 MHz, $CDCl_3$): δ 2.71 (3H, s), 3.15 (3H, s), 3.35 (3H, s), 3.67-3.71 (2H, m), 4.58-4.62 (2H, m), 5.55 (2H, brd), 7.09 (1H, d, J = 8.4 Hz), 7.15 (2H, d, J = 4.7 Hz), 7.17-7.23 (1H, m), 7.42-7.49 (1H, m), 8.06 (1H, dd, J = 8.2, 1.1 Hz), 8.56 (2H, s). MS (ESI, pos): m/z 418 $(M + H)^+$, 440 $(M + Na)^+$.

Example 17

2-Cyanoethyl 2,4-dimethyl-5-oxo-5,6-dihydrobenzo[c][2,7]naphthyridine-1-carboxylate (compound 13) And

Example 18

2,4-dimethyl-5-oxo-5,6-dihydrobenzo[c][2,7] naphthyridine-1-carboxylic acid (compound 14)

The title compounds were prepared according to the synthetic steps in Scheme 3.

Scheme 3

[Chem. 36]

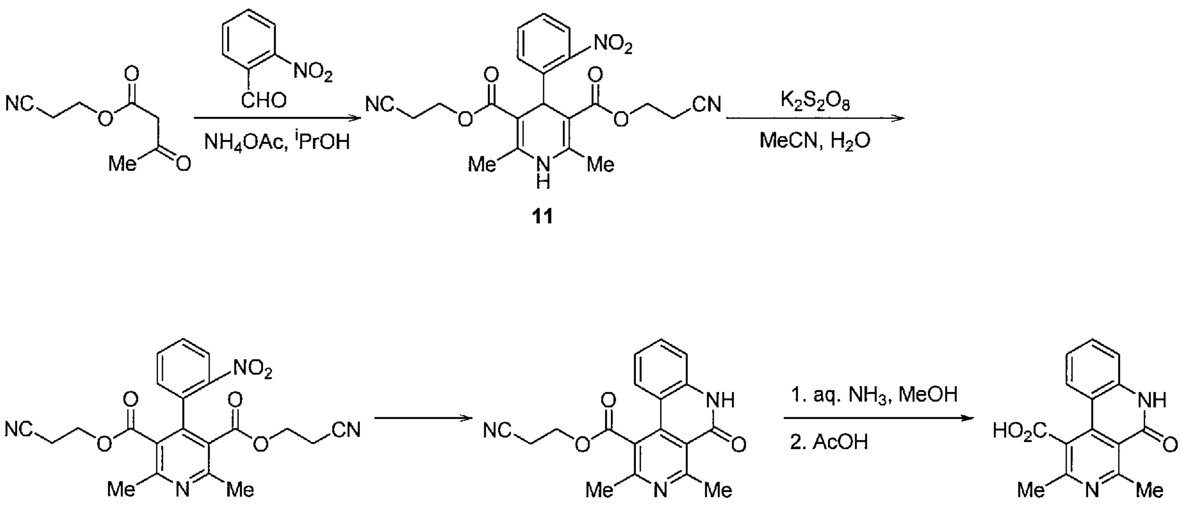


11

12

13

14

17-1: Bis(2-cyanoethyl) 2,6-dimethyl-4-(2-nitrophenyl)-1,4-dihydropyridine-3,5-dicarboxylate (compound 11)

A solution of 2-nitrobenzaldehyde (TCI, 1.52 g, 10.1 mmol), 2-cyanoethoxyethyl 3-oxobutanoate (3.09 g, 20.0 mmol) and ammonium acetate (WAKO, 1.17 g, 15.2 mmol) in 2-propanol (50 mL) was reflux at 80°C for 15 hours under an argon atmosphere. After cooling to room temperature, the mixture was diluted with ethyl acetate and water, and the dilution was extracted with ethyl acetate (50 mL × 4), washed with water (50 mL × 2) and saturated brine (50 mL), and dried over sodium sulfate. After filtration and concentration under reduced pressure to give a crude product (4.92 g) as a solid. Dichloromethane was added thereto and the solid was collected by filtration to provide compound 11 (1.70 g, 40%) as a yellow solid.

$^1H$ NMR (500 MHz, $CDCl_3$): δ 2.36 (6H, s), 2.68-2.72 (4H, m), 4.13-4.18 (2H, m), 4.28-4.33 (2H, m), 5.77 (1H, s), 6.00 (1H, s), 7.28-7.31 (1H, m), 7.48-7.51 (2H, m), 7.71 (1H, d, J = 7.9 Hz). MS (ESI, pos): m/z 447 $(M + Na)^+$.

17-2: Bis(2-cyanoethyl) 2,6-dimethyl-4-(2-nitrophenyl)pyridine-3,5-dicarboxylate (compound 12)

Compound 11 (1.70 g, 4.0 mmol) prepared in Example 17-2 and potassium peroxodisulfate (Nacalai, 3.27 g, 12.1 mmol) were added to acetonitrile (15 mL)/water (8 mL), and the mixture was reflux. As any solid was not completely dissolved during the reflux, additional acetonitrile (10 mL)/water (5 mL) was added thereto. After 2.5 hours, the mixture was cooled to room temperature, acetonitrile was distilled off under reduced pressure, the resultant material was diluted with ethyl acetate and water, and the dilution was extracted with ethyl acetate (50 mL × 3), followed by washing the extract with water (50 mL × 2) and saturated brine (50 mL), and then drying over sodium sulfate. After filtration and concentration under reduced pressure, the residue was purified by medium pressure column chromatography (SNAP 25 μm, 25 g, chloroform/methanol = 100/0 → 95/5) to provide compound 12 (0.98 g, 58%) as a white solid.

$^1H$ NMR (500 MHz, $CDCl_3$): δ 2.40-2.49 (4H, m), 2.70 (6H, s), 4.08-4.13 (2H, m), 4.17-4.22 (2H, m), 7.28 (1H, dd, J = 7.6, 1.4 Hz), 7.62-7.66 (2H, m), 7.69-7.73 (1H, m), 8.21 (1H, dd, J = 4.1, 1.4 Hz). MS (ESI, pos): m/z 423 (M +

H)$^+$, 445 (M + Na)$^+$.

17-3: 2-Cyanoethyl 2,4-dimethyl-5-oxo-5,6-dihydrobenzo[c][2,7]naphthyridine-1-carboxylate (compound 13)

10% Pd/C (WAKO, 22.5 mg) was added to a solution of compound 12 prepared in Example 17-2 (212 mg, 0.50 mmol) in ethanol (10 mL), and the mixture was stirred overnight at room temperature under a hydrogen atmosphere. 10% Pd/C (WAKO, 22.5 mg) was added again, and the mixture was stirred at room temperature for 19 hours under a hydrogen atmosphere. After filtering the reaction mixture and washing with ethanol, the filtrate was concentrated under reduced pressure, and the residue was purified by medium pressure column chromatography (SNAP 25 $\mu$m, 10 g, chloroform/methanol = 100/0 $\rightarrow$ 95/5) to provide compound 13 (135 mg, 84%).
$^1$H NMR (500 MHz, $CDCl_3$): $\delta$ 2.73 (3H, s), 2.81 (2H, t, J = 6.3 Hz), 3.22 (3H, s), 4.63 (2H, t, J = 6.3 Hz), 7.32 (1H, m), 7.73 (1H, m), 7.85 (1H, dd, J = 8.4, 1.1 Hz), 7.96 (1H, dd, J = 8.4, 0.8 Hz). MS (ESI, pos): None detected.

18: 2,4-dimethyl-5-oxo-5,6-dihydrobenzo[c][2,7] naphthyridine-1-carboxylic acid (compound 14)

25% Aqueous ammonia (10 mL) was added to a solution of compound 13 prepared in Example 17-3 (426 mg, 1.33 mmol) in methanol (10 mL), and the mixture was stirred at room temperature for 2 hours. The reaction solution was neutralized by adding acetic acid thereto, and the precipitated solid was washed with chloroform, followed by heating the solid at 70°C to remove the residual acetic acid and drying the same under reduced pressure, thereby providing compound 14 (257 mg, 72%) as a white solid.
$^1$H NMR (500 MHz, DMSO-$d_6$): $\delta$ 2.52 (3H, s), 2.94 (3H, s), 6.98-7.03 (1H, m), 7.20-7.30 (2H, m), 8.73 (1H, d, J = 8.2 Hz). MS (ESI, neg): m/z 267 (M-H)$^+$.

Example 19

2-Methoxyethyl 5-((4-methoxyphenyl)carbamoyl)-2,6-dimethyl-4-phenyl-1,4-dihydropyridine-3-carboxylate (compound 16b)

Example 20

2-Methoxyethyl 6-(3-methoxyphenyl)-2,4-dimethyl-5-oxo-5,6-dihydrobenzo[c][2,7]naphthyridine-1-carboxylate (compound 17a)

Example 21

2-Methoxyethyl 6-(3-(tert-butoxycarbonyl)phenyl)-2,4-dimethyl-5-oxo-5,6-dihydrobenzo[c] [2,7]naphthyridine-1-carboxylate (compound 17c)

The title compounds were prepared according to the synthetic steps in Scheme 4.

Scheme 4

[Chem. 37]

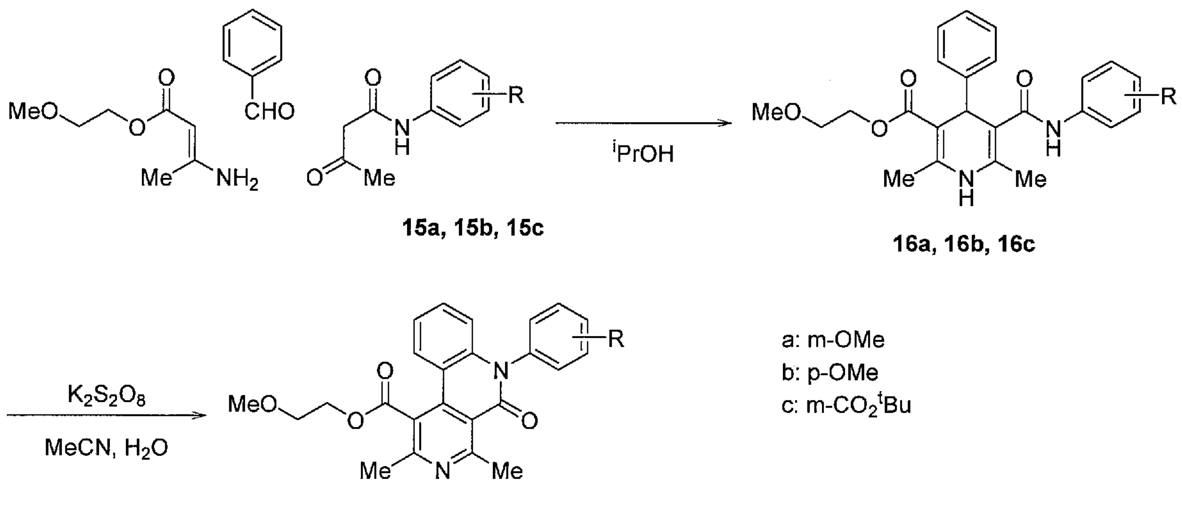


15a, 15b, 15c

16a, 16b, 16c

17a, 17c

a: m-OMe
b: p-OMe
c: m-$CO_2{}^tBu$

19-1: N-(3-Methoxyphenyl)-3-oxobutanamide (Compound 15a)

A solution of m-anisidine (TCI, 615 mg, 5.0 mmol) and 2,2,6-trimethyl-1,3-dioxin-4-one (TCI, 781 μL, 5.5 mmol) in toluene (13 mL) was reflux for 1 hour under an argon atmosphere. After cooling the reaction solution to room temperature, then concentrated under reduced pressure. The residue was purified by medium pressure column chromatography (SNAP 25 μm, 25 g, hexane:ethyl acetate = 88:12 → 0:100) to provide compound 15a (896 mg, 86%) as a yellow solid.

$^1H$ NMR (500 MHz, $CDCl_3$): δ 2.33 (3H, s), 3.58 (2H, s), 3.80 (3H, s), 6.68 (1H, dd, J = 8.0, 2.0 Hz), 7.04 (1H, dd, J = 8.0, 2.0 Hz), 7.22 (1H, t, J = 8.0 Hz), 7.28 (1H, t, J = 2.0 Hz), 9.07 (1H, brd). MS (ESI, pos): m/z 230 $(M + Na)^+$.

P-Acetoacetanisidide (compound 15b) was purchased from TCI.

19-2: tert-butyl 3-(3-oxobutanamido)benzoate (compound 15c)

A solution of tert-butyl 3-aminobenzoate (combi-Blocks, 1.0 g, 5.2 mmol) and 2,2,6-trimethyl-1,3-dioxin-4-one (TCI, 809 mg, 5.7 mmol) in toluene (20 mL) was reflux under an argon atmosphere at 110°C for 0.5 hours. After cooling to room temperature and concentrating under reduced pressure, the residue was purified by medium pressure column chromatography (SNAP 25 μm, 25 g, hexane:ethyl acetate = 88:12 → 0:100) to provide compound 15c (1.12 g, 78%) as a yellow oil.

$^1H$ NMR (500 MHz, $CDCl_3$): δ 1.59 (9H, s), 2.33 (3H, s), 3.60 (2H, s), 7.37 (1H, t), 7.88 (1H, d), 7.97 (1H, s), 9.23 (1H, s). MS (ESI, pos): m/z 300 $(M + Na)^+$.

19-3: 2-Methoxyethyl 5-((3-methoxyphenyl)carbamoyl)-2,6-dimethyl-4-phenyl-1,4-dihydropyridine-3-carboxylate (compound 16a)

A solution of benzaldehyde (aldrich, 455 mg, 4.29 mmol), compound 15a (890 mg, 4.29 mmol), and 2-methoxyethyl-3-aminocrotonate (713 mg, 4.48 mmol) in 2-propanol (22 mL) was stirred overnight at 70°C under an argon atmosphere. The reaction solution was cooled to room temperature, and concentrated, followed by purifying the concentrated residue by medium pressure column chromatography (SNAP 25 μm, 50 g, hexane:ethyl acetate = 88:12 → 0:100) to provide compound 16a (680 mg, 36%) as a yellow solid.

$^1H$ NMR (500 MHz, $CDCl_3$): δ 2.31 (3H, s), 2.33 (3H, s), 3.36 (3H, s), 3.54-3.61 (2H, m), 3.76 (3H, s), 4.16-4.21 (1H, m), 4.23-4.27 (1H, m), 4.84 (1H, s), 5.66 (1H, brd), 6.58 (1H, ddd, J = 8.0, 2.5, 0.75 Hz), 6.66 (1H, ddd, J = 8.0, 2.5, 0.75 Hz), 7.05 (1H, t, J = 2.5 Hz), 7.11 (1H, t, J = 8.0 Hz), 7.15 (1H, brd), 7.24-7.27 (1H, m), 7.32-7.35 (2H, m), 7.44-7.46 (2H, m). MS (ESI, pos): m/z 437 $(M + H)^+$, 459 $(M + Na)^+$.

19-4: 2-Methoxyethyl 5-((4-methoxyphenyl)carbamoyl)-2,6-dimethyl-4-phenyl-1,4-dihydropyridine-3-carboxylate (compound 16b)

A solution of benzaldehyde (aldrich, 106 mg, 1.0 mmol), p-acetoacetanisidide 15b (TCI, 207 mg, 1.0 mmol), and 2-methoxyethyl-3-aminocrotonate (175 mg, 1.1 mmol) in 2-propanol (5 mL) was stirred overnight at 70°C under an argon atmosphere. After cooling the reaction solution to room temperature and concentrating, the mixture was separated with water (20 mL) and chloroform (20 mL), and the organic layer was washed with water (20 mL) and saturated brine (20 mL). The aqueous layer was extracted with chloroform (20 mL), and the organic layers were combined, dried over magnesium sulfate, filtered, and concentrated under reduced pressure. The residue was purified by medium pressure column chromatography (SNAP 25 $\mu$m, 10 g, chloroform:methanol = 99:1 → 98:2), and the desired fractions were collected and concentrated under reduced pressure. Further purification by medium pressure column chromatography (SNAP 25 $\mu$m, 10 g, hexane:ethyl acetate = 84:16 → 0:100) gave compound 16b (135 mg, 31%) as a yellow oil.

$^1$H NMR (500 MHz, $CDCl_3$): $\delta$ 2.30 (3H, s), 2.32 (3H, s), 3.36 (3H, s), 3.54-3.61 (2H, m), 3.45 (3H, s), 4.16-4.27 (2H, m), 4.85 (1H, s), 5.66 (1H, brd), 6.77 (2H, td, J = 10.5, 2.8 Hz), 7.06 (1H, brd), 7.15 (2H, td, J = 10.5, 2.8 Hz), 7.23-7.26 (1H, m), 7.31-7.34 (2H, m), 7.43-7.45 (2H, m). MS (ESI, pos): m/z 437 $(M + H)^+$, 459 $(M + Na)^+$.

20-1: 2-Methoxyethyl 5-((3-(tert-butoxycarbonyl)phenyl) carbamoyl)-2,6-dimethyl-4-phenyl-1,4-dihydropyridine-3-carboxylate (compound 16c)

A solution of compound 15c (318 mg, 2.0 mmol), benzaldehyde (sigma-Aldrich, 212 mg, 2.0 mmol), and 2-methoxyethyl-3-aminocrotonate (555 mg, 2.0 mmol) in 2-propanol (10 mL) was stirred overnight at 70°C under an argon atmosphere. After returning to room temperature and concentrating under reduced pressure, the residue was purified by medium pressure column chromatography (SNAP 25 $\mu$m, 25 g, hexane:ethyl acetate = 88:12 → 0:100) to provide compound 16c (402 mg, 40%) as a yellow oil.

$^1$H NMR (500 MHz, $CDCl_3$): $\delta$ 1.57 (9H, s), 2.28 (3H, s), 2.32 (1H, s), 3.37 (3H, s), 3.59 (2H, m), 4.20 (2H, m), 4.86 (1H, s), 5.57 (1H, s), 7.21 (1H, s), 7.36 (3H, m), 7.46 (2H, d), 7.53 (1H, s), 7.63 (1H, dd), 7.77 (1H, d) . MS (ESI, pos): None detected

20-2: 2-Methoxyethyl 6-(3-methoxyphenyl)-2,4-dimethyl-5-oxo-5,6-dihydrobenzo[c] [2,7]naphthyridine-1-carboxylate (Compound 17a)

A solution of compound 16a prepared in Example 19-3 (446 mg, 1.02 mmol) and potassium peroxodisulfate (Nacalai, 828 mg, 3.06 mmol) in acetonitrile/water (2:1, 30 mL) was stirred at 80°C for 2 hours under an argon atmosphere. After returning to room temperature, the liquidity was adjusted to pH 7. The mixture was separated with ethyl acetate (80 mL), and then the organic layer was washed with water (80 mL) and saturated brine (80 mL). The aqueous layer was extracted with ethyl acetate (80 mL), and the organic layers were combined, dried over magnesium sulfate, and concentrated under reduced pressure. The residue was purified by medium pressure column chromatography (SNAP 25 $\mu$m, 10 g, hexane:ethyl acetate = 88:12 → 50:50) to provide compound 17a (266 mg, 60%) as a pale yellow solid.

$^1$H NMR (500 MHz, $CDCl_3$): $\delta$ 2.71 (3H, s), 3.11 (3H, s), 3.37 (3H, s), 3.70-3.72 (2H, m), 3.84 (3H, s), 4.61-4.63 (2H, m), 6.71 (1H, dd, J = 8.0, 1.0 Hz), 6.83 (1H, t, J = 2.5 Hz), 6.90 (1H, ddd, J = 8.0, 2.5, 1.0 Hz), 7.08 (1H, ddd, J = 8.0, 2.5, 1.0 Hz), 7.15-7.18 (1H, m), 7.34-7.38 (1H, m), 7.53 (1H, t, J = 8.0 Hz), 8.03 (1H, dd, J = 8.0, 1.0 Hz). MS (ESI, pos): m/z 433 $(M + H)^+$, 455 $(M + Na)^+$.

Example 21-1: 2-Methoxyethyl 6-(3-(tert-butoxycarbonyl) phenyl)-2,4-dimethyl-5-oxo-5,6-dihydrobenzo[c] [2,7] naphthyridine- 1-carboxylate (compound 17c)

A solution of compound 16c (200 mg, 0.29 mmol) and potassium peroxodisulfate (Nacalai, 319 mg, 1.18 mmol) in acetonitrile/water (2:1, 15 mL) was reflux at 80°C for 1.5 hours under an argon atmosphere. After returning to room temperature, the pH was adjusted to about 7, and water (50 mL) and ethyl acetate (50 mL) were added thereto. After separation, the aqueous layer was extracted with ethyl acetate (2 × 50 mL). The organic layers were combined, washed with water (2 × 50 mL) and saturated brine (2 × 50 mL), dried over magnesium sulfate, and concentrated under reduced pressure. The residue was purified by medium pressure column chromatography (SNAP 25 $\mu$m, 10 g, hexane:ethyl acetate = 88:12 → 0:100). Since the resultant material was contaminated with impurities, it was concentrated again and purified by medium pressure column chromatography (SNAP 25 $\mu$m, 10 g, hexane:ethyl acetate = 95:5 → 65:35). From this operation compound 17c (86.4 mg, 44%) was obtained as a pale yellow solid.

$^1$H NMR (500 MHz, $CDCl_3$): $\delta$ 1.59 (9H, s), 2.72 (3H, s), 3.10 (3H, s), 3.38 (3H, s), 3.72 (2H, t), 4.62 (2H, t), 6.60 (1H, d), 7.18 (1H, t), 7.36 (1H, t), 7.49 (1H, d), 7.69 (1H, t), 7.91 (1H, s), 8.05 (1H, d), 8.18 (1H, d). MS (ESI, pos): m/z 503 $(M + H)^+$, 525 $(M + Na)^+$.

In Examples 22-26 below, each example compound was prepared according to Scheme 5 below.

Scheme 5

[Chem. 38]

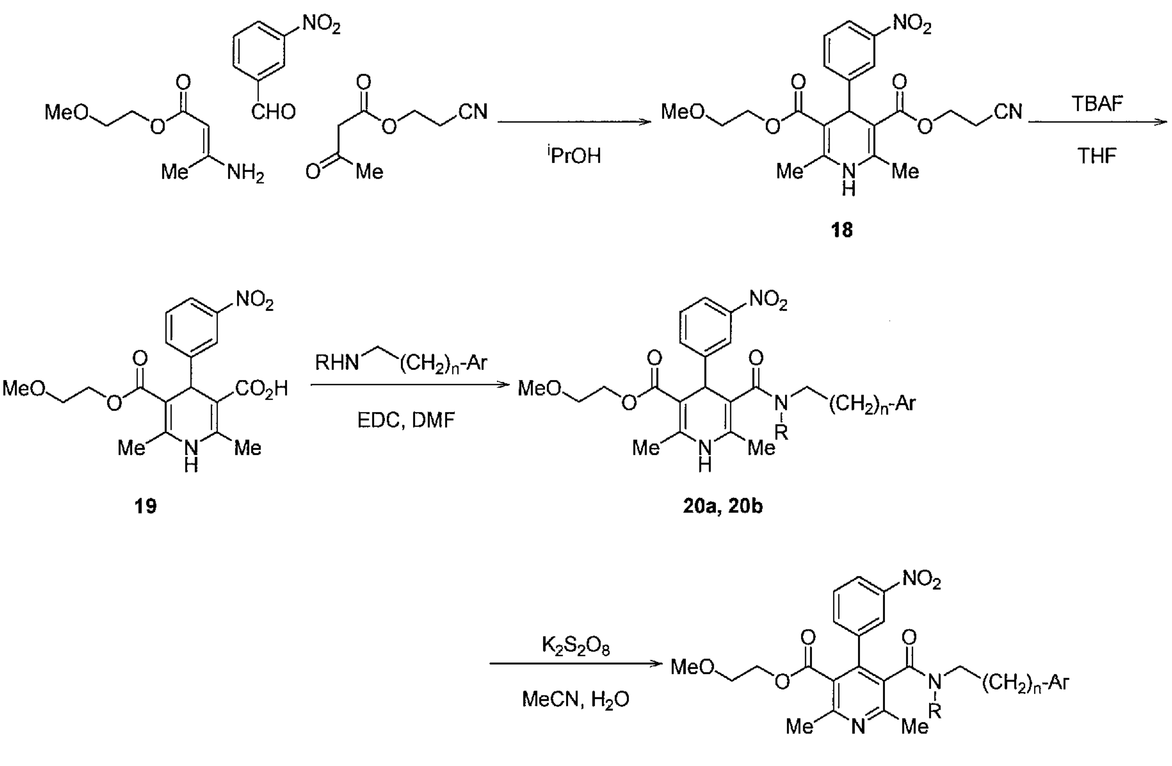


21b

Example 22

3-(2-cyanoethyl) 5-(2-methoxyethyl) 2,6-dimethyl-4-(3-nitrophenyl)-1,4-dihydropyridine-3,5-dicarboxylate (compound 18)

A solution of m-nitrobenzaldehyde (WAKO, 453 mg, 3.0 mmol), 2-methoxyethyl-3-aminocrotonate (525 mg, 3.0 mmol), and 2-cyanoethyl 3-oxobutanoate (465 mg, 3.0 mmol) in 2-propanol (12 mL) was stirred at 100°C for 2 hours under an argon atmosphere. After overnight, the mixture was cooled to room temperature and concentrated, and the residue was purified by medium pressure column chromatography (SNAP 25 μm, 25 g, hexane:ethyl acetate = 88:12 → 20:80), so that the desired fractions were collected and concentrated under reduced pressure. After that, the residue was crystallized with diisopropyl ether to provide compound 18 (1.0 g, 77%) as a yellow solid.

$^1$H NMR (500 MHz, $CDCl_3$): δ 2.37 (3H, s), 2.40 (3H, s), 2.65 (2H, dt, J = 7.6, 1.5 Hz), 3.35 (3H, s), 3.51-3.59 (2H, m), 4.15-4.28 (4H, m), 5.12 (1H, s), 5.86 (1H, brd), 7.40 (1H, t, J = 7.8 Hz), 7.71 (1H, td, J = 7.8, 1.3 Hz), 8.01 (1H, dd, J = 7.8, 1.3 Hz), 8.11 (1H, t, J = 2.0 Hz). MS (ESI, pos): m/z 430 $(M + H)^+$, 452 $(M + Na)^+$.

Example 23

5-((2-methoxyethoxy)carbonyl)-2,6-dimethyl-4-(3-nitrophenyl)-1,4-dihydropyridine-3-carboxylic acid (compound 19)

To a solution of compound 18 prepared in Example 22 (215 mg, 0.50 mmol) in THF (1 mL), 1.0 M TBAF in THF (TCI, 600 μL, 0.6 mmol) was added, the mixture was stirred at room temperature for 4 hours under an argon atmosphere, followed by adding 1.0 M TBAF in THF (TCI, 500 μL, 0.5 mmol) thereto and stirring the mixture overnight at room temperature. After cooling to 0°C, the mixture was added with 1N HCl (1.5 mL), and with ethyl acetate (20 mL) and water (20 mL), and the mixture was stirred. After separation, the organic layer was washed with water (20 mL) and

saturated brine (20 mL). The aqueous layer was extracted with ethyl acetate (20 mL), and the organic layers were combined, dried over magnesium sulfate, and concentrated under reduced pressure. The residue was crystallized with diisopropyl ether to provide compound 19 (136 mg, 72%) as a yellow solid.

$^1$H NMR (500 MHz, DMSO-$d_6$): δ 2.28 (3H, s), 2.29 (3H, s), 3.21 (3H, s), 3.43-3.52 (2H, m), 4.01-4.13 (2H, m), 4.97 (1H, s), 7.55 (1H, t, J = 7.3 Hz), 7.62 (1H, dt, J = 7.3, 1.3 Hz), 7.97-8.01 (2H, m), 8.94 (1H, brd), 10.8 (1H, brd). MS (ESI, pos): m/z 377 $(M + H)^+$, 399 $(M + Na)^+$.

Example 24

2-Methoxyethyl 5-(benzylcarbamoyl)-2,6-dimethyl-4-(3-nitrophenyl)-1,4-dihydropyridine-3-carboxylate (compound 20a)

A solution of compound 19 prepared in Example 23 (102 mg, 0.27 mmol), benzylamine (TCI, 29.6 μL, 0.27 mmol), and triethylamine (TCI, 54mg, 0.54 mmol) in DMF (2.7 mL) was brought to 0°C under an argon atmosphere, then EDC/HCl (WATANABE, 57 mg, 0.297 mmol) and HOBt/$H_2O$ (WATANABE, 496 mg, 0.324 mmol) were added thereto, and the mixture was stirred for 2 hours. The mixture was returned to room temperature, and stirred overnight. The mixture was separated with water (20 mL) and ethyl acetate (20 mL), and the organic layer was washed with water (20 mL) and saturated brine (20 mL). The aqueous layer was extracted with ethyl acetate (20 mL), and the organic layers were combined, dried over magnesium sulfate, and concentrated under reduced pressure. The residue was purified by medium pressure column chromatography (SNAP 25 μm, 10 g, hexane:ethyl acetate = 84:16 → 20:80) to provide compound 20a (85 mg, 68%) as a yellow solid.

$^1$H NMR (500 MHz, DMSO-$d_6$): δ 2.04 (3H, s), 2.26 (3H, s), 3.17 (3H, s), 3.39-3.47 (2H, m), 3.95-4.07 (2H, m), 4.16 (1H, dd, J=15, 5.5 Hz), 4.27 (1H, dd, J=15, 5.5 Hz), 5.00 (1H, s), 6.92-6.95 (2H, m), 7.13-7.18 (3H, m), 7.53 (1H, t, J = 16 Hz), 7.61 (1H, td, J = 8.0, 2.5 Hz), 7.97 (1H, t, J = 4.0 Hz), 8.02 (1H, ddd, J = 8.0, 2.5, 1.0 Hz), 8.12(1H, brd), 8.51 (1H, brd). MS (ESI, pos) : m/z 466 $(M + H)^+$, 488 $(M + Na)^+$.

Example 25

2-Methoxyethyl 2,6-dimethyl-4-(3-nitrophenyl)-5-((3-(pyridin-4-yl)propyl)carbamoyl)-1,4-dihydropyridine-3-carboxylate (Compound 20b)

A solution of compound 19 prepared in Example 24 (180 mg, 0.477 mmol), 3-(pyridin-4-yl)propan-1-amine (BLD pharm, 65 mg, 0.477 mmol) and triethylamine (TCI, 96mg, 0.954 mmol) in DMF (5 mL) was brought to 0°C under an argon atmosphere, then EDC/HCl (WATANABE, 101 mg, 0.525 mmol) and HOBt/$H_2O$ (WATANABE, 88 mg, 0.572 mmol) were added thereto, and the mixture was stirred for 2 hours. The mixture was returned to room temperature and stirred for another 2 days. The mixture was separated with water (30 mL) and ethyl acetate (30 mL), and the organic layer was washed with water (30 mL) and saturated brine (30 mL). The aqueous layer was extracted with ethyl acetate (30 mL), and the organic layers were combined, dried over magnesium sulfate, and concentrated under reduced pressure. The residue was purified by medium pressure column chromatography (SNAP 25 μm, 10 g, chloroform: methanol = 100:0 → 96:4) to provide compound 20b (144 mg, 61%) as a yellow solid.

$^1$H NMR (500 MHz, $CDCl_3$) : δ 1.68-1.76 (2H, m), 2.27 (3H, s), 2.33 (3H, s), 2.48 (2H, t, J = 8.0 Hz), 3.18-3.28 (2H, m), 3.34 (3H, s), 3.52-3.59 (2H, m), 4.15-4.18 (1H, m), 4.22-4.28 (1H, m), 4.93 (1H, s), 5.41 (1H, brd), 5.65 (1H, brd), 7.02 (2H, dd, J = 4.5, 1.5 Hz), 7.41 (1H, t, J =8.0 Hz), 7.69 (1H, dt, J=8.0, 1.0 Hz), 8.03 (1H, ddd, J = 8.0, 2.0, 1.0 Hz), 8.15 (1H, t, J = 2.0 Hz), 8.47 (2H, dd, J = 4.5, 1.5 Hz). MS (ESI, pos): m/z 517 $(M + Na)^+$.

Example 26

2-Methoxyethyl 2,6-dimethyl-4-(3-nitrophenyl)-5-((3-(pyridin-4-yl)propyl)carbamoyl)nicotinate (compound 21b)

A solution of compound 20b prepared in Example 25 (114 mg, 0.23 mmol) and potassium peroxodisulfate (Nacalai, 186 mg, 0.69 mmol) in acetonitrile/water (2:1, 7 mL) was stirred at 80°C for 1 hour under an argon atmosphere. After returning to room temperature, the liquidity was adjusted to pH 7-8. The mixture was separated with ethyl acetate (40 mL), washed with water (40 mL) and saturated brine (40 mL). The aqueous layer was extracted with ethyl acetate (40 mL), and the organic layers were combined, dried over magnesium sulfate, and concentrated under reduced pressure. The residue was purified by medium pressure column chromatography (SNAP 25 μm, 10 g, chloroform:methanol = 100:0 → 90:10) to provide compound 21b (59 mg, 52%) as a pale yellow oil.

$^1$H NMR (500 MHz, $CDCl_3$): δ 1.49-1.55 (2H, m), 2.36 (2H, t, J = 8.0 Hz), 2.62 (6H, s), 3.15-3.19 (2H, m), 3.22 (3H, s), 3.32-3.36 (2H, m), 4.10-4.14 (2H, m), 5.76 (1H, brd), 6.96-6.97 (2H, m), 7.55 (1H, t, J = 8.0 Hz), 7.68-7.70 (1H, m), 8.15-8.20 (2H, m), 8.45-8.46 (2H, m). MS (ESI, pos): m/z 493 $(M + H)^+$, 515 $(M + Na)^+$.

In Examples 27-29 below, each example compound was prepared according to Scheme 6.

Scheme 6

[Chem. 39]

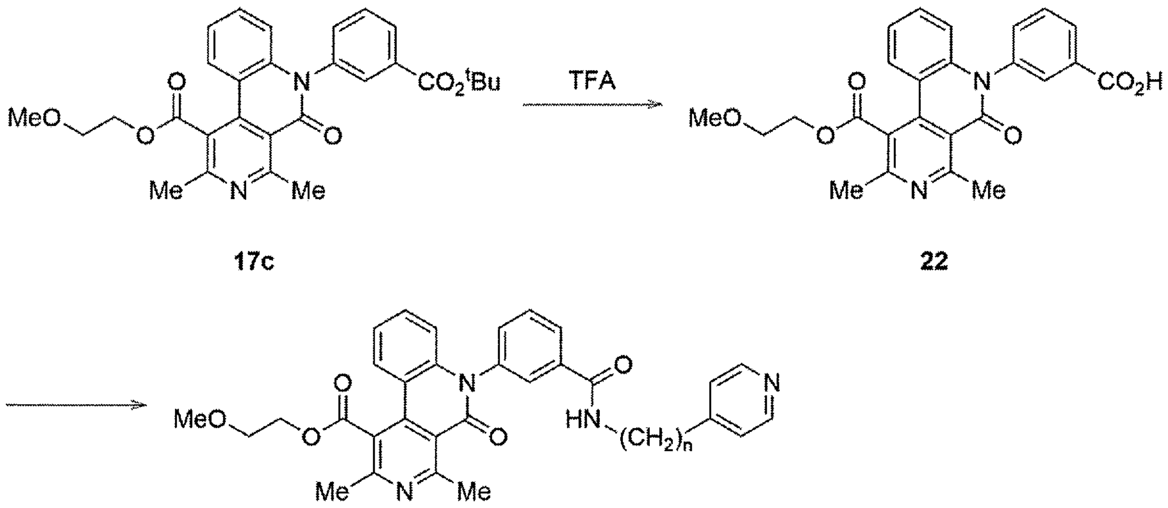


17c

22

23a (n = 1)
23b (n = 2)
23c (n = 3)

Example 27

2-Methoxyethyl 2,4-dimethyl-5-oxo-6-(3-((pyridin-4-ylmethyl)carbamoyl)phenyl)-5,6-dihydrobenzo[c] [2,7] naphthyridine-1- Carboxylate (compound 23a)

27-1: 3-(1-((2-methoxyethoxy)carbonyl)-2,4-dimethyl-5-oxobenzo[c][2,7]naphthyridine-6(5H)-yl)benzoic acid (compound 22)

[Chem. 40]

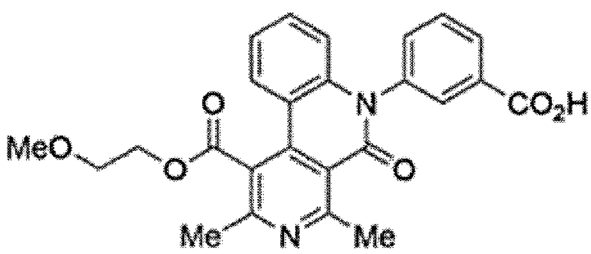

To a solution of 17c (213 mg, 0.42 mmol) synthesized in Example 21-1 in dichloromethane (4 mL) was added trifluoroacetic acid (2 mL) under ice cooling. After stirring at room temperature for 1 hour, the reaction mixture was boiled as an azeotrope with toluene. The residue was diluted with ethyl acetate (30 mL), and the dilution was dried over magnesium sulfate, filtered, and concentrated under reduced pressure. The residue was purified by medium pressure column chromatography (SNAP 25 $\mu$m, 25 g, chloroform: methanol = 100:0 $\rightarrow$ 98:2) to provide compound 22 (181 mg, 97%) as a yellow solid.

$^1$H NMR (500 MHz, $CDCl_3$): $\delta$ 2.79 (3H, s), 3.16 (3H, s), 3.37 (3H, s), 3.71 (2H, t), 4.63 (2H, t), 6.64 (1H, d), 7.24 (1H, t), 7.42 (1H, t), 7.57 (1H, d), 7.76 (1H, t), 8.04 (1H, s), 8.09 (1H, d), 8.28 (1H, d). MS (ESI, pos): No m/z detected.

Example 27-2: 2-Methoxyethyl 2,4-dimethyl-5-oxo-6-(3-((pyridin-4-ylmethyl)carbamoyl)phenyl)-5,6-dihydrobenzo[c][2,7]naphthyridine-1-carboxylate (compound 23a)

[Chem. 41]

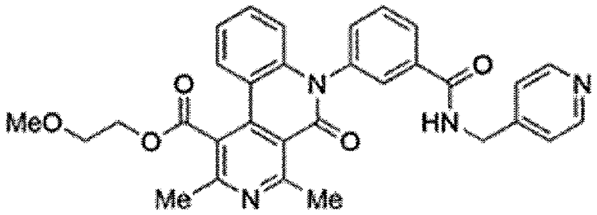

A reaction similar to that of Example 24 was conducted using compound 22 prepared in Example 27 (100 mg, 0.22 mmol), 4-(aminomethyl)pyridine (24 mg, 0.22 mmol), EDC/HCl (46mg, 0.24 mmol), HOBt/$H_2O$ (40 mg, 0.26 mmol), TEA (44 mg, 0.44 mmol), and DMF (10 mL) to provide compound 23a (68.8 mg, 58%) .

$^1H$ NMR (500 MHz, $CDCl_3$): δ 2.12 (3H, s), 3.07 (3H, s), 3.37 (3H, s), 3.71 (2H, t), 4.62 (2H, t), 4.66 (2H, s), 6.60 (1H, s), 6.65 (1H, brd), 7.19 (1H, t), 7.28 (1H, m), 7.36 (1H, t), 7.50 (1H, d), 7.75 (2H, m), 8.02 (1H, d), 8.05 (1H, d), 8.58 (2H, brd). MS (ESI, pos): m/z 559 $(M + Na)^+$.

Example 28

2-Methoxyethyl 2,4-dimethyl-5-oxo-6-(3-((2-pyridin-4-yl)ethyl)carbamoyl)phenyl)-5,6-dihydrobenzo[c] [2,7] Naphthyridine-1-carboxylate (compound 23b)

[Chem. 42]

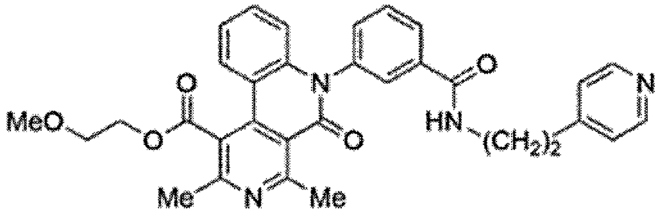

A reaction similar to that of Example 24 was conducted using compound 22 prepared in Example 27-1 (140mg, 0.31 mmol), 4-(aminoethyl)pyridine (38 mg, 0.31 mmol), EDC/HCl (65 mg, 0.34 mmol), HOBt/$H_2O$ (57mg, 0.37 mmol), TEA (62 mg, 0.62 mmol), and DMF (15 mL) to provide compound 23b (108 mg, 63%).

$^1H$ NMR (500 MHz, $CDCl_3$): δ 2.72 (3H, s), 2.95 (3H, s), 3.07 (3H, s), 3.37 (3H, s), 3.70-3.76 (4H, m), 4.62 (2H, t), 6.28 (1H, s), 6.58 (1H, d), 7.17-7.20 (3H, m), 7.35 (1H, t), 7.47 (1H, d), 7.66 (1H, s), 7.70 (1H, t), 7.89 (1H, d), 8.05 (1H, d), 8.58 (2H, d). MS (ESI, pos): m/z 573 $(M + Na)^+$.

Example 29

2-Methoxyethyl 2,4-dimethyl-5-oxo-6-(3-((2-pyridin-4-yl)propyl)carbamoyl)phenyl)-5,6-dihydrobenzo[c][2,7] Naphthyridine-1-carboxylate (compound 23c)

[Chem. 43]

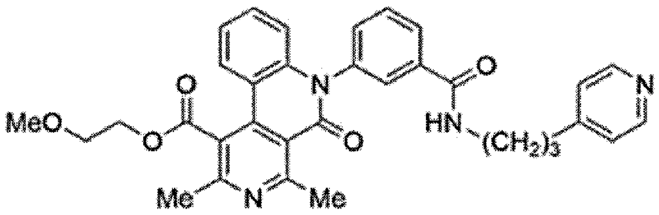

A reaction similar to that of Example 24 was conducted using compound 22 prepared in Example 27-1 (100 mg, 0.22 mmol), 4-(aminopropyl)pyridine (30 mg, 0.22 mmol), EDC/HCl (46 mg, 0.24 mmol), HOBt/$H_2O$ (40 mg, 0.26 mmol), TEA (44mg, 0.44 mmol) and DMF (10 mL) to provide compound 23c (99 mg, 80%).

$^1H$ NMR (500 MHz, $CDCl_3$) : δ 1.96 (2H, q), 2.71 (5H, m), 3.07 (3H, s), 3.37 (3H, s), 3.49 (2H, m), 3.71 (2H, t), 4.62 (2H, t), 6.27 (1H, t), 6.58 (1H, d), 7.13 (2H, d), 7.18 (1H, t), 7.35 (1H, t), 7.47 (1H, d), 7.65 (1H, s), 7.71 (1H, t), 7.93 (1H, d), 8.04 (1H, d), 8.48 (1H, d). MS (ESI, pos): m/z 587 $(M + Na)^+$.

Example 30

2-Methoxyethoxy 5-((3-(tert-butoxycarbonylcarbonyl)phenyl) carbamoyl)-2,6-dimethyl-4-phenylnicotinate (compound 24)

[Chem. 44]

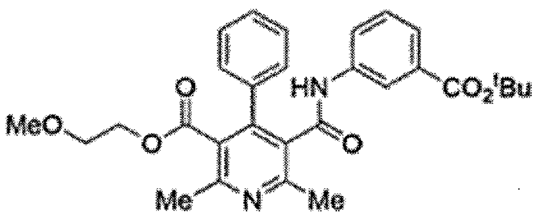

Example 31

3-(5-((2-methoxyethoxy)carbonyl)-2,6-dimethyl-4-phenylnicotinamido)benzoic acid (compound 25)

[Chem. 45]

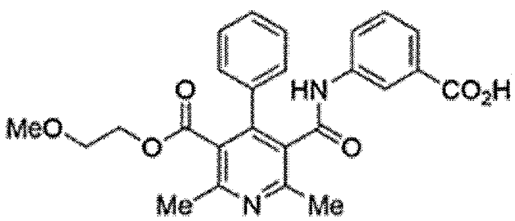

A solution of compound 16c prepared in Example 20-1 (100 mg, 0.20 mmol) in dioxane (1 mL) was cooled to 0°C under an argon atmosphere, and then a 4M hydrochloride dioxane solution (1 mL) was added dropwise thereto. The mixture was stirred at 0°C for 30 minutes and at room temperature for 50 minutes. A solution of 4M hydrochloride dioxane (1 mL) was added dropwise thereto, and the mixture was stirred for an additional 90 minutes. After concentration under reduced pressure and drying in vacuo, the residue was purified by medium pressure column chromatography

(Presep, 8 g, chloroform: methanol = 100:0 → 90:10) to provide compound 24 (26 mg, 26%) as a yellow gum. Also, compound 25 (28 mg, 31%) was obtained as a yellow solid.

Compound 24 $^1H$ NMR (500 MHz, $CDCl_3$): δ 1.57 (9H, s), 2.63 (3H, s), 2.70 (3H, s), 3.23 (3H, s), 4.11 (2H, t, J = 4.8 Hz), 7.29 (1H, t, J = 7.9 Hz), 7.33-7.39 (5H, m), 7.45-7.47 (1H, m), 7.51-7.56 (1H, m), 7.67-7.71 (1H, m). MS (ESI, pos): m/z 505 $(M + H)^+$.

Compound 25 $^1H$ NMR (500 MHz, DMSO): δ 2.53 (3H, s), 2.54 (3H, s), 3,16 (3H, s), 3.26 (2H, t, J = 4.7 Hz), 4.07 (2H, t, J = 4.7 Hz), 7.25-7.40 (6H, m), 7.54-7.63 (2H, m), 8.04-8.06 (1H, m), 10.50 (1H, s). MS (ESI, neg): m/z 447 $(M - H)^+$.

Example 32

2-Methoxyethoxy 5-((3-(methoxycarbonyl)phenyl)carbamoyl)-2,6-dimethyl-4-phenylnicotinate (compound 26)

[Chem. 46]

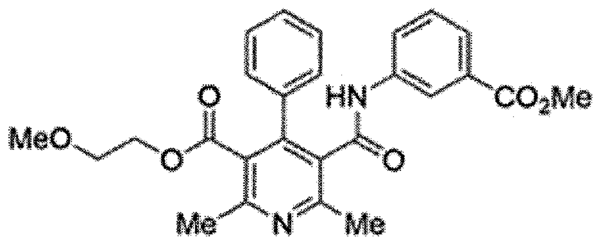

A solution of compound 25 prepared in Example 31 (100 mg, 0.20 mmol) in THF (2 mL) was cooled to 0°C under an argon atmosphere, then oxalyl chloride (TCI, 95 μL, 1.1 mmol) and DMF (1 drop) were added dropwise thereto and the mixture was stirred at room temperature for 30 minutes. Methanol (1 mL) was added thereto and the mixture was stirred for 3 hours. Further, methanol (1 mL) was added thereto and the mixture was stirred for 1 hour. After concentration under reduced pressure, ethyl acetate (15 mL) and saturated aqueous sodium bicarbonate (15 mL) were added thereto and the mixture was stirred and separated. The aqueous layer was extracted with ethyl acetate, and the organic layers were combined, washed with saturated brine, dried over magnesium sulfate, and concentrated under reduced pressure. The residue was purified by medium pressure column chromatography (Presep, 8 g, chloroform: methanol = 100:0 → 99:1) to provide compound 26 (61 mg, 60%) as a yellow amorphous.

$^1H$ NMR (500 MHz, $CDCl_3$): δ 2.62 (3H, s), 2.70 (3H, s), 3.23 (3H, s), 3.23 (2H, t, J = 4.8 Hz), 3.88 (3H, s), 4.11 (2H, t, J = 5.0 Hz), 6.99 (1H, br), 7.31 (1H, t, J = 7.9 Hz), 7.34-7.40 (1H, m), 7.46-7.50 (1H, m), 7.61-7.64 (1H, m), 7.73-7.76 (1H, m). MS (ESI, pos): m/z 463 $(M + H)^+$.

Example 33

3-(5-((2-methoxyethoxy)carbonyl)-2,6-dimethyl-4-phenyl-1,4-dihydropyridine-3-carboxamido)benzoic acid (compound 31)

Scheme 7

[Chem. 47]

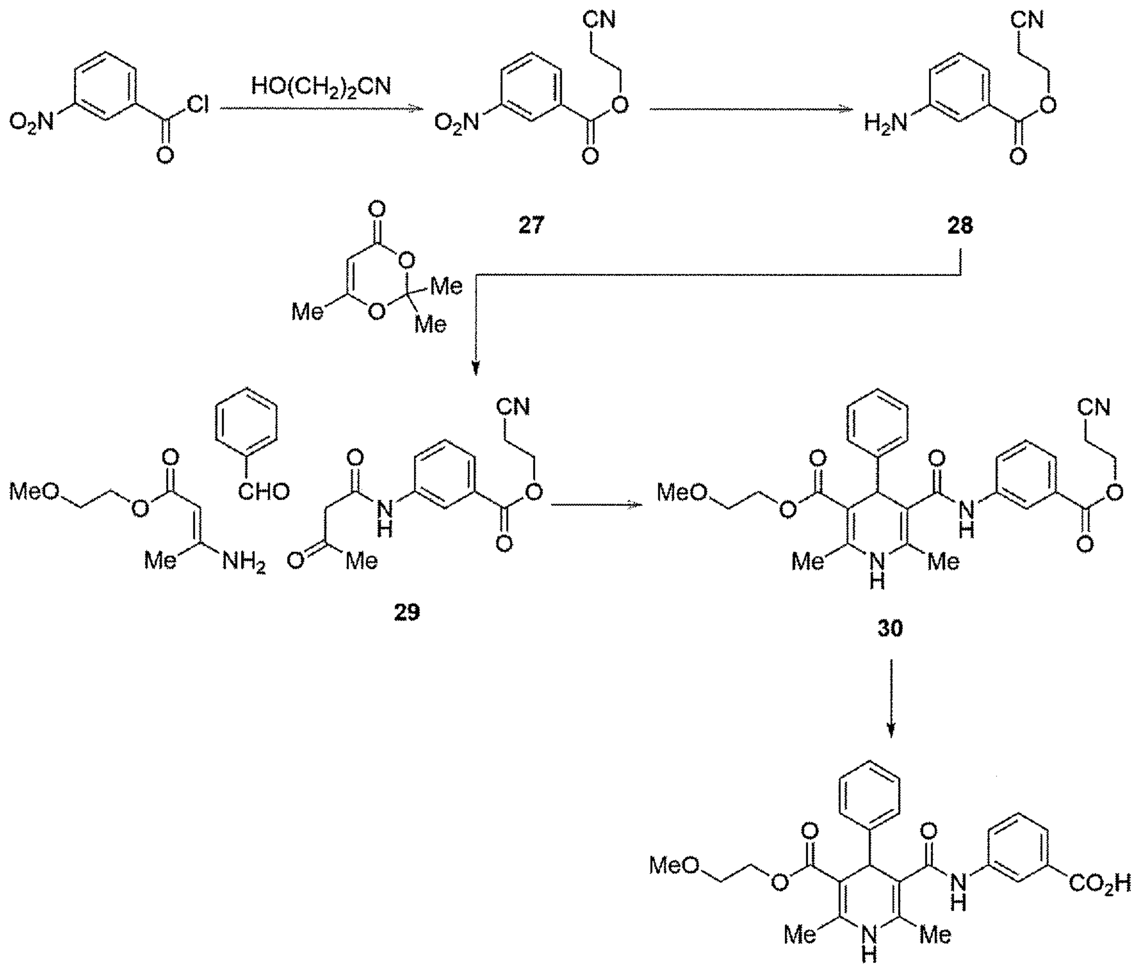


27

28

29

30

31

33-1: 2-Cyanoethyl 3-nitrobenzoate (compound 27)

[Chem. 48]

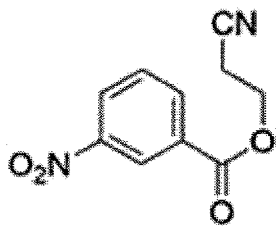

After a solution of 3-nitrobenzoyl chloride (1.85 g, 10 mmol) in dichloromethane (10 mL) was cooled to 0°C under an argon atmosphere, 2-cyanoethanol (745 ul, 11 mmol) was added thereto, and the mixture was stirred at room temperature for 1.5 hours. Triethylamine (1 mL) was added thereto, the mixture was stirred for 0.5 hours, and separated with ethyl acetate (100 mL) and water (100 mL). The aqueous layer was extracted with ethyl acetate, and the organic

layers were combined, washed with saturated brine, dried over magnesium sulfate, and concentrated under reduced pressure. The concentrated residue was dried in vacuo to provide compound 27 (2.17 g, 99%) as a colorless solid.

$^1H$ NMR (500 MHz, DMSO-$d_6$) : δ 3.09 (2H, dd), 4.54 (2H, dd), 7.88 (1H, dd), 8.39 (1H, ddd), 8.51-8.56 (1H, m), 8.66 (1H, dd). MS (ESI, pos) : m/z 191 $(M + H - NO)^+$

33-2: 2-Cyanoethyl 3-aminobenzoate (compound 28)

[Chem. 49]

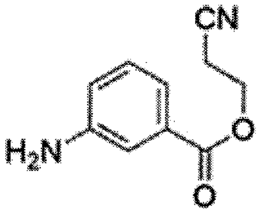

To a solution of compound 27 (2.17 g, 9.86 mmol) in methanol (30 mL) and AcOEt (10 mL), palladium-activated carbon (100 mg, 0.09 mmol) was added under an argon atmosphere, and the mixture was stirred at room temperature under a hydrogen atmosphere for 2 hours. Additional palladium-activated carbon (100 mg, 0.09 mmol) was added thereto under an argon atmosphere, and the mixture was stirred at room temperature under a hydrogen atmosphere for 1 hour. The mixture was filtered under reduced pressure and concentrated under reduced pressure. The concentrated residue was dried in vacuo to provide compound 28 (1.84 g, 98%) as a white solid.

$^1H$ NMR (500 MHz, DMSO-$d_6$) : δ 3.00 (2H, dd), 4.40 (2H, dd), 5.35-5.45 (2H, br), 6.80-6.84 (1H, m), 7.11 (1H, ddd), 7.16 (1H, dd), 7.19 (1H, dd). MS (ESI, pos): m/z 191 $(M + H)^+$

33-3: 2-Cyanoethyl 3-(3-oxobutanamido)benzoate (compound 29)

[Chem. 50]

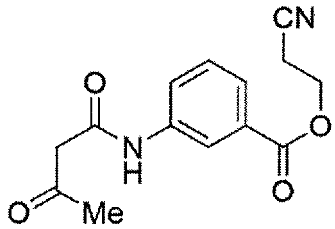

A solution of compound 28 (1.84 g, 9.86 mmol) and 2,2,6-trimethyl-1,3-dioxin-4-one (TCI, 1.51 g, 10.6 mmol) in toluene (20mL) and 1,4-dioxane (20mL) was heated at 110°C under an argon atmosphere, and the mixture was stirred for 2.5 hours. After concentration under reduced pressure, the residue was purified by medium pressure column chromatography (sfar silica HC D 50 g, chloroform: methanol = 100:0 → 99:1) to provide compound 29 (2.61 g, 98%) as a brown oil.

$^1H$ NMR (500 MHz, $CDCl_3$): δ 2.34 (3H, s), 2.85 (2H, dd), 3.62 (2H, s), 4.53 (2H, dd), 7.42 (1H, dd), 7.81 (1H, ddd), 7.88-7.94 (1H, m), 8.10 (1H, dd), 9.31 (1H, br). MS (ESI, pos): m/z 297 $(M + Na)^+$

33-4: 2-Methoxyethyl 5-((3-((2-cyanoethoxy)carbonyl)phenyl)carbamoyl)-2,6-dimethyl-4-phenyl-1,4-dihydropyridine-3-carboxylate (compound 30)

[Chem. 51]

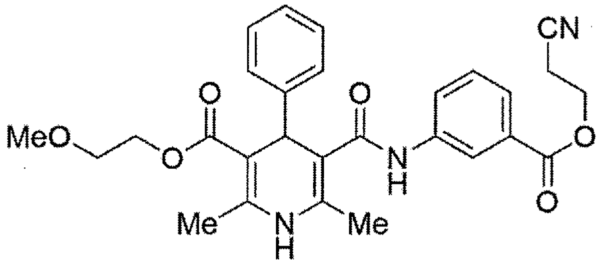

A solution of compound 29 (1.3 g, 4.7 mmol), 2-methoxyethyl-3-aminocrotonate (0.82 g, 5.2 mmol), and benzaldehyde (470 μl, 5.2 mmol) in isopropyl alcohol (15 mL) was stirred under an argon atmosphere at 70°C for 24 hours. After concentration under reduced pressure, the residue was purified by medium pressure column chromatography (Presep, 115 g, hexane:ethyl acetate = 84:16 → 0:100). Fractions containing the desired product were collected and further purified by medium pressure column chromatography (Presep, 28 g, hexane:ethyl acetate = 85:15 → 30:70) to provide compound 30 (893 mg, 37%) as a yellow amorphous.

$^1$H NMR (500 MHz, $CDCl_3$): δ 2.32 (3H, s), 2.36 (3H, s), 2.83 (2H, dd), 3.37 (3H, s), 3.56-3.60 (2H, m), 4.16-4.30 (2H, m), 4.50 (2H, dd), 4.86 (1H, s), 5.61 (1H, s), 7.27-7.30 (2H, m), 7.33 (1H, dd), 7.37 (2H, dd), 7.45-7.48 (1H, m), 7.59-7.62 (1H, m), 7.71 (1H, ddd), 7.80 (1H, dd). MS (ESI, pos): m/z 526 $(M + Na)^+$

33-5: 3-(5-((2-methoxyethoxy)carbonyl)-2,6-dimethyl-4-phenyl-1,4-dihydropyridine-3-carboxamido)benzoic acid (compound 31)

[Chem. 52]

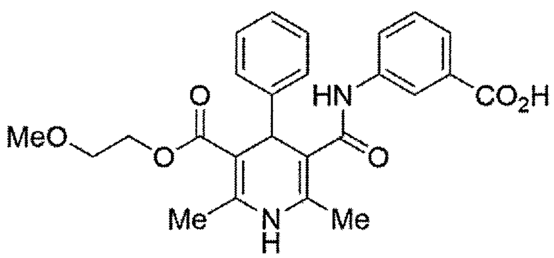

To a solution of compound 30 (0.44 g, 0.87 mmol) in tetrahydrofuran (2 mL), 1.0 M TBAF in TFA (1ml, 1 mmol) was added, and the mixture was stirred at room temperature for 20 hours under an argon atmosphere. Ethyl acetate (20 mL) and water (20 mL) were added thereto, and the mixture was stirred and separated. The aqueous layer was extracted with ethyl acetate, and the organic layers were combined, washed with saturated brine, dried over magnesium sulfate, and concentrated under reduced pressure. The residue was purified by medium pressure column chromatography (Presep, 13 g, chloroform: methanol = 100:0 → 90:10) to provide compound 31 (260 mg, 66%) as a yellow amorphous.

$^1$H NMR (500 MHz, $CDCl_3$) : δ 2.32 (3H, s), 2.35 (3H, s), 3.38 (3H, s), 3.57-3.62 (2H, m), 4.18-4.30 (2H, m), 4.90 (1H, s), 5.63 (1H, s), 7.27-7.40 (5H, m), 7.46 (2H, d), 7.62-7.65 (1H, m), 7.75 (1H, ddd), 7.87 (1H, dd, J = 1.7Hz).

$^1$H NMR (500 MHz, DMSO-$d_6$): δ 2.05 (3H, s), 2.27 (3H, s), 3.21 (3H, s), 3.49-3.49 (2H, m), 3.97-4.10 (2H, m), 4.90 (1H, s), 5.63 (1H, s, $D_2O$ exchangeable), 7.06-7.22 (5H, m), 7.35 (1H, dd), 7.56 (1H, ddd), 7.76-7.80 (1H, m), 8.20 (1H, dd), 8.49 (1H, s), 9.69 (1H, s). MS not detectable.

Example 34

N-benzyl-2,4-dimethyl-5-oxo-5,6-dihydrobenzo[c] [2,7] naphthyridine-1-carboxamide (compound 35)

Scheme 8

[Chem. 53]

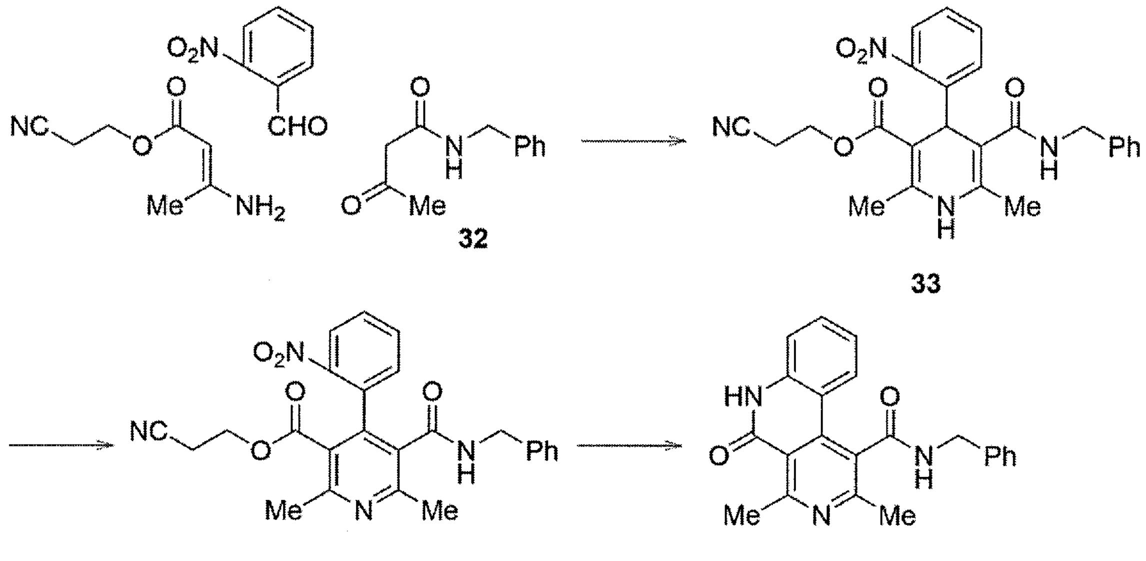
32
33
34
35

34-1: 2-Cyanoethyl-3-aminocrotonate

[Chem. 54]

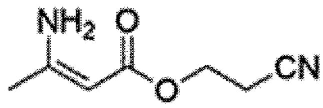

A method similar to that of Example 1-1 was used to provide 2-cyanoethyl-3-aminocrotonate (746.8 mg, 97%) as a crude product from 2-cyanoethyl 3-oxobutanoate (775.7 mg, 5.0 mmol), ammonium acetate (1.927 g, 25 mmol), and 2-propanol.

$^1$H NMR (500 MHz, $CDCl_3$): δ 1.93 (3H, s), 2.69 (2H, t, J = 6.4 Hz), 4.26 (2H, t, J = 6.4 Hz), 4.56 (1H, s). MS (ESI, pos): m/z 177 $(M + Na)^+$.

34-2: N-benzyl-3-oxobutanamide (compound 32)

[Chem. 55]

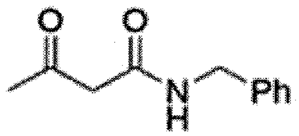

A method similar to that of Example 19-1 was used to provide compound 32 (2.25 g, 79%) as a yellow solid from benzylamine (1.607 g, 15 mmol), 2,2,6-trimethyl-1,3-dioxin-4-one (1.99 ml, 15 mmol), and toluene (15 mL).

$^1$H NMR (500 MHz, $CDCl_3$): δ 2.26 (3H, s), 3.45 (2H, s), 4.46 (2H, d, J = 5.8 Hz), 7.20-7.36 (6H, m). MS (ESI,

pos): m/z 214 $(M + Na)^+$.

34-3: 2-Cyanoethyl 5-(benzylcarbamoyl)-2,6-dimethyl-4-(2-nitrophenyl)-1,4-dihydropyridine-3-carboxylate (compound 33)

[Chem. 56]

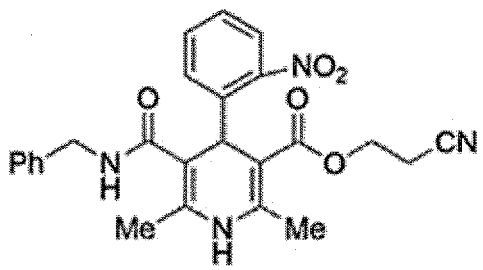

A method similar to that of Example 19-3 was used to provide compound 33 (506 mg, 23%) as a crude product from compound 32 (926mg, 4.84 mmol), 2-nitrobenzaldehyde (732 mg, 4.84 mmol), 2-cyanoethyl-3-aminocrotonate (746.8 mg, 4.85 mmol) and 2-propanol. NMR and MS were not measured.

34-4: 2-Cyanoethyl 5-(benzylcarbamoyl)-2,6-dimethyl-4-(2-nitrophenyl)nicotinate (compound 34)

[Chem. 57]

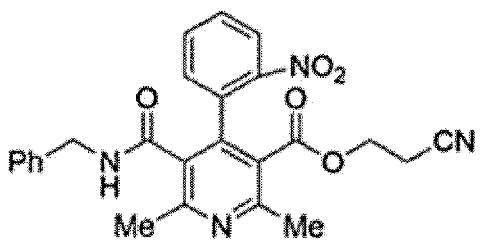

A method similar to that of Example 20-2 was used to provide compound 34 (154.9 mg, 31%) as a white solid from compound 33 (506mg, 1.10 mmol), potassium peroxodisulfate (891.8mg, 3.30 mmol), acetonitrile (5 mL), and water (5 mL).

$^1H$ NMR (500 MHz, $CDCl_3$): δ 2.35-2.52 (2H, m), 2.62 (3H, s), 4.02-4.11 (2H, m), 4.13-4.21 (1H, m), 4.47 (1H, dd, J = 14.5, 7.5 Hz), 6.40 (1H, t, J = 5.8 Hz), 6.80 (2H, d, J = 7.0 Hz), 7.13-7.23 (3H, m), 7.32 (1H, dd, J = 7.6, 1.3 Hz), 7.49 (1H, td, J = 7.9, 1.4 Hz), 7.63 (1H, td, J = 7.6, 1.2 Hz), 7.80 (1H, dd, J = 8.2, 1.1 Hz). MS (ESI, pos): m/z 459 $(M + H)^+$, 481 $(M + Na)^+$.

34-5: N-benzyl-2,4-dimethyl-5-oxo-5,6-dihydrobenzo[c][2,7] naphthyridine-1-carboxamide (compound 35)

[Chem. 58]

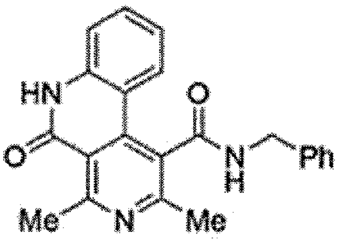

A solution of compound 34 (154.9 mg, 0.34 mmol) and palladium-activated carbon (10%, 15.49 mg) in ethanol (10 mL) was stirred under a hydrogen atmosphere at room temperature for 3 hours. Additional palladium-activated carbon (10%, 25.2 mg) was added thereto, the mixture was stirred at room temperature for 12 hours under a hydrogen atmosphere, and further palladium-activated carbon (10%, 21.5 mg) was added thereto. After 5 hours, the palladium was removed by filtration and the mixture was concentrated under reduced pressure. The residue was purified by medium pressure column chromatography (SNAP 25 $\mu$m, 10 g, chloroform: methanol = 100:0 $\rightarrow$ 99:1) to provide compound 35 (13.3 mg, 11%) as a white solid.

$^1$H NMR (500 MHz, DMSO-$d_6$): $\delta$ 2.99 (3H, s), 4.51 (2H, d, J = 5.9 Hz), 6.84-6.91 (1H, m), 7.27-7.34 (2H, m), 7.35-7.39 (4H, m), 7.46-7.52 (1H, m), 8.08 (1H, d, J = 8.3 Hz), 9.23 (1H, t, J = 5.9 Hz), 11.7 (1H, s). MS (ESI, pos): m/z 358 $(M + H)^+$, 380 $(M + Na)^+$.

Test Example 1

Construction of system for evaluating degree of mitochondrial division

A system for evaluating quantitatively the degree of mitochondrial division in cells was constructed. Specifically, cardiomyocytes derived from neonatal rats were cultured under either normoxia (20%) or hypoxia (1%) for 16 hours. Subsequently, the cells were stained with a fluorescent dye (MitoTracker Green FM, Thermo Fisher Scientific) that selectively stains mitochondria. Then, the mitochondria were observed under a fluorescence microscope, and the mitochondrial morphology was quantitatively measured by image analysis.
Figures 1(a) and (b) are representative fluorescence micrographs showing the morphology of mitochondria. Fig. 1(a) is a fluorescence micrograph of a cell cultured under normoxia, and Fig. 1(b) is a fluorescence micrograph of a cell cultured under hypoxia.

Figure 2(a) and (b) are each a micrograph and an image explaining a method for quantitatively measuring mitochondrial morphology by image analysis. Figure 2(a) is a representative fluorescence micrograph showing the general morphology of mitochondria. Figure 2(b) is an image obtained by filtering the photograph in Figure 2(a) and extracting the shape of the mitochondria. Image analysis was performed based on the image in Fig. 2(b) to quantify the lengths of mitochondrial fragments. For the quantification, in order to classify the mitochondria into vesicle-shaped, intermediate-shaped and tube-shaped, <3.5 um is set for vesicle-shaped, 3.6 um <length <4.2 um is set for intermediate-shaped, and >4.2 $\mu$m is set for tube-shaped, respectively, as thresholds.

Fig. 2(c) is a graph that illustrates the results of measurement of length of mitochondria from the cardiomyocytes of a neonatal rat cultured under normoxia or hypoxia. That is, the micrographs of Figure 1(a) and (b) were allocated to the micrograph of Figure 2(a) to create the image corresponding to Figure 2(b) for each micrograph, followed by performing image analysis to obtain the result of Figure 2(c). In Figure 2(c), "black bars" represent the results of culturing under normoxia, and "white bars" represent the results of culturing under hypoxia. As mentioned above, mitochondria of 3.5 um or smaller were classified as vesicle-shaped, mitochondria of 4.2 um or larger as tube-shaped, and mitochondria of 3.6 um or larger but smaller than 4.2 um as intermediate-shaped.

As a result, it was demonstrated that the cardiomyocytes from the neonatal rat, cultured under normoxia, produced large percentages of tube-shaped mitochondria, whereas the cardiomyocytes from the neonatal rat, cultured under hypoxia (1%), caused mitochondrial division, and produced large percentages of vesicle-shaped mitochondria. In addition, it was confirmed that the method of Test Example can quantitatively evaluate the morphology of mitochondria.

Test Example 2

Inhibitory effect on mitochondrial hyperfission by hypoxic stimulation

The system for evaluation constructed in Test Example 1 was used to evaluate the mitochondrial hyperfission

inhibitory effects of the compounds prepared in Examples.

Specifically, at first, each compound was added to the culture medium of cardiomyocytes derived from neonatal rats at a final concentration of 0.3 μM, and the mixture was incubated for 1 hour.

Then, each cells were cultured for an additional 18 hours in a hypoxic environment. Subsequently, mitochondria were stained with MitoTracker Green FM (Thermo Fisher Scientific) or anti-pyruvate dehydrogenase antibody (Abcam) and observed under a fluorescence microscope to quantitatively measure the morphology of mitochondria. For comparison, a group involving cilnidipine as a positive control and a group involving dimethyl sulfoxide (DMSO) as a negative control were also prepared.

The results are shown in Figures 3(a) and (b). Figure 3 is graphs that illustrate that the compounds prepared in Examples show the inhibitory effect on mitochondrial hyperfission as the results of measuring the percentage of cells with vesicle-shaped mitochondria. The results confirmed that compound 2 (Example 1), compound 4f (Example 10), compound 16b (Example 19), compound 18 (Example 22), compound 20a (Example 24), compound 6 (Example 12), Compound 17a (Example 20), Compound 14 (Example 18), Compound 10a (Example 14), and Compound 10b (Example 15), and Compound 16c (Example 20-1), Compound 17c (Example 21), compound 24 (Example 30), compound 25 (Example 31), compound 26 (Example 32), compound 30 (Example 33-4), and compound 31 (Example 33) showed mitochondrial hyperfission inhibitory effect that is the same as or more effect of cilnidipine.

Test Example 3

Calcium antagonism

The compounds prepared in Examples were evaluated for calcium channel blocking activity. Specifically, at first, Neuro-2a cells, mouse-derived neuroblastoma cell line, were cultured in a serum-free medium, and Fura 2-AM (Dojindo Laboratories) that is a calcium ion probe, was added to the medium so that it was taken up by the cells.

Subsequently, after replacing the medium with HEPES buffer (pH 7.4), each compound was each added at final concentrations of 0, 1, 10, 100 and 1,000 nM and the mixtures were incubated for 30 minutes.

Then, depolarization stimulation was applied with potassium chloride (final concentration 60 mM), and the influx of calcium ions into the cells was measured by fluorescence microscopy.

Figures 4(a) and (b) are graphs that illustrate the results of measurement of increase in intracellular calcium ion concentration induced by potassium chloride stimulation. Δratio on the vertical axis is expressed by formula (F1) below and represents a value that reflects the intracellular calcium ion concentration.

Formula (F1)

Δ ratio=(fluorescence intensity at fluorescence wavelength of 510 nm against an excitation wavelength of 340 nm)/(fluorescence intensity at fluorescence wavelength of 510 nm against an excitation wavelength of 380 nm)

The results demonstrated that compound 2, compound 4f, compound 16b, compound 18, compound 20a, compound 6, compound 14, compound 16c and compound 17a exhibit no Ca2+ antagonism.

Test Example 4

Inhibitory effect of hypoxic stimulation on againg of cardiomyocytes

The compounds prepared in Examples were investigated for effects on the aging of cardiomyocytes. The aging of cardiomyocytes was evaluated by detecting β-galactosidase, one of the aging markers.

Specifically, DOJINDO's cell aging detection kit-SPiDER-βGAL was used for detection according to the attached protocol. Each compound was added to the culture medium of cardiomyocytes derived from neonatal rats at a final concentration of 1 μM and the medium were incubated for 1 hour. Then, the medium was cultured for 16 hours under normoxia (20%) or hypoxia (1%). Subsequently, Bafilomycin A1, which inhibits endogenous β-galactosidase activity, was added and the medium was incubated for 1 hour. After washing the cells with a medium, SPiDER-βGAL was added

thereto and the mixture was incubated for 30 minutes. After removing the supernatant, HEPES buffer (pH 7.4) was added thereto and the cells were observed under a fluorescence microscope to detect β-galactosidase in the aging cells. For comparison, a group involving cilnidipine as a positive control and a group involving dimethylsulfoxide (DMSO) as a negative control were prepared.

The results are shown in Figure 5. Figure 5 shows the fluorescence of β-galactosidase, a marker of cellular aging accelerated by normoxia/hypoxia. Arrowheads indicate the β-galactosidase-positive cells.

The result demonstrated that compound 2 and compound 4f exhibit the suppressive effect on aging that is the same as cilnidipine.

Test Example 5

Cytoprotective effect against cytotoxicity induced by MeHg and hypotonic stimulation

Neonatal rat myocardial cells (NRCM) were seeded at 2.0 x $10^5$ cells/ml in Matrigel-coated 96-well plates. The NRCMs were pretreated by incubating for 1 hour at 37°C, 5% $CO_2$ with 1 μM of each compound 16c and cilnidipine as a positive control, 1 hour before hypotonic stimulation. To provide hypotonic stimulation, the NRCMs were incubated in isotonic solutions (100% DMEM) or 50% hypotonic solutions (50% DMEM and 50% water) in the presence or absence of 50 nM MeHg that a non-neurotoxic concentration, at 37°C and 5% $CO_2$ for 2 days. Two days later, cytotoxicity was measured by an LDH release assay using a cytotoxic LDH assay kit (Dojindo).

The results are shown in Figure 6. Compound 16c exhibited the cytoprotective effects against MeHg and hypotonic stimulation similar to those of cilnidipine.

**Claims**

1. A compound represented by formula (1):

[Chem. 1]

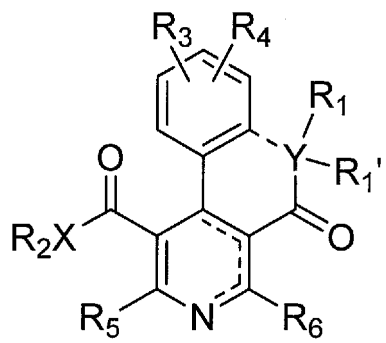

**(1)**

wherein

$R_1$ and $R_1$' are each independently hydrogen, an optionally substituted lower alkyl, an optionally substituted lower cycloalkyl, an optionally substituted lower heterocycloalkyl, an optionally substituted aryl, an optionally substituted arylalkyl, an optionally substituted heteroaryl, an optionally substituted heteroarylalkyl, an optionally substituted carbonyl, an optionally substituted carboxamide, or a covalent substituent;

$R_2$ is an optionally substituted lower alkyl, an optionally substituted lower cycloalkyl, an optionally substituted lower heterocycloalkyl, an optionally substituted aryl, an optionally substituted arylalkyl, an optionally substituted heteroaryl, an optionally substituted heteroarylalkyl, an optionally substituted carboxamide, an optionally substituted carbonyl, or a covalent substituent;

$R_3$ and $R_4$ are each independently hydrogen, halogen, hydroxy, nitro, cyano, an optionally substituted lower alkyl, an optionally substituted lower cycloalkyl, an optionally substituted lower heterocycloalkyl, an optionally substituted aryl, an optionally substituted arylalkyl, an optionally substituted heteroaryl, an optionally substituted heteroaryl, an optionally substituted heteroarylalkyl, an optionally substituted amino, an optionally substituted

lower alkoxy, an optionally substituted lower alkylthio, an optionally substituted carbonyl, an optionally substituted carboxamide, deuterium, or a covalent substituent;

$R_5$ and $R_6$ are each independently an optionally substituted lower alkyl, an optionally substituted lower cycloalkyl, an optionally substituted lower heterocycloalkyl, an optionally substituted aryl, an optionally substituted arylalkyl, an optionally substituted heteroaryl, an optionally substituted heteroarylalkyl, an optionally substituted carbonyl, an an optionally substituted carboxamide, or a covalent substituent;

X is nitrogen or oxygen;

Y is carbon, nitrogen or oxygen, and

A broken line represents the presence or absence of a bond;

provided that:

a broken line attached to Y represents the presence or absence of a bond, if Y is carbon or nitrogen, or the broken line represents the absence of a bond, if X is oxygen; and provided that the covalent substituents are selected from a group consisting of acrylamide, alkynylamide, chloroacetamide, chlorofluoroacetamide, vinylsulfone, vinylsulfonamide, sulfonylfluoride, sulfonyltriazole, sulfonyloxyfluoride, bicyclobutaneketone, bicyclobutanamide, bicyclobutanesulfone, and bicyclobutanesulfonamide, and

that the covalent substituent can be substituted with only one of the substituents of $R_1$ through $R_6$, when the covalent substituents are substituted;

or a pharmaceutically acceptable salt, a solvate, or a prodrug thereof.

2. A compound, or a pharmaceutically acceptable salt, a solvate, or a prodrug thereof, wherein the compound is represented by formula (2):

[Chem. 2]

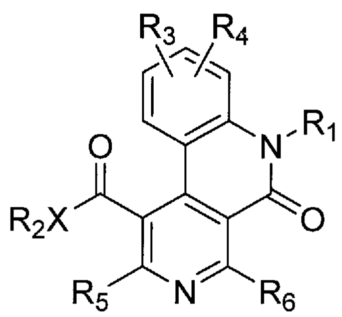

**(2)**

wherein $R_1$, $R_1$', $R_2$, $R_3$, $R_4$, $R_5$, $R_6$ and X are the same as defined in claim 1.

3. The compound of claim 2, or a pharmaceutically acceptable salt, a solvate, or a prodrug thereof, wherein the compound is represented by formula (2-2):

[Chem. 3]

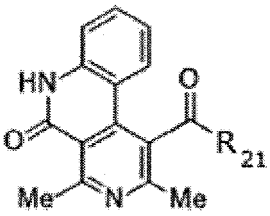

(2-2)

wherein $R_{21}$ is an optionally substituted lower alkyl, an optionally substituted arylalkyl, an optionally substituted heteroarylalkyl, an optionally substituted carboxamide, an optionally substituted carbonyl, or a covalent substituent; or

formula (2-3):

[Chem. 4]

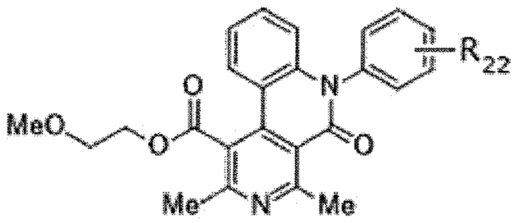

(2-3)

wherein $R_{22}$ is independently an optionally substituted carbonyl, preferably an optionally substituted carboxylic acid, an optionally substituted alkoxycarbonyl, in particular an optionally substituted methoxycarbonyl, or an optionally substituted tert-butoxycarbonyl, more preferably these substituents $R_{22}$ are located in the meta position.

**4.** The compound, or a pharmaceutically acceptable salt, a solvate, or a prodrug thereof, wherein the compound is represented by formula (3):

[Chem. 5]

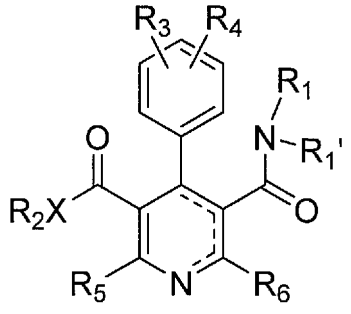

**(3)**

wherein $R_1$, $R_1$', $R_2$, $R_3$, $R_4$, $R_5$, $R_6$, X and a broken line are the same as defined in claim 1.

5. The compound of claim 4, or a pharmaceutically acceptable salt, a solvate, or a prodrug thereof, wherein the compound is represented by formula (3-2):

[Chem. 6]

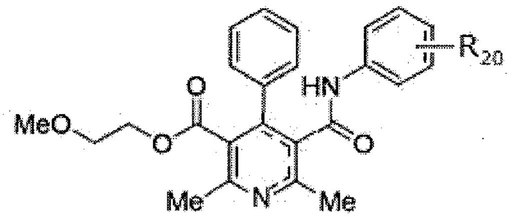

(3-2)

wherein $R_{20}$ is independently an optionally substituted carbonyl, preferably an optionally substituted carboxylic acid, an optionally substituted alkoxy carbonyl, in particular an optionally substituted methoxycarbonyl, or an optionally substituted tert-butoxycarbonyl, more preferably these substituents $R_{20}$ are located in the meta position.

6. The compound of any one of claims 1 to 5, or a pharmaceutically acceptable salt, a solvate, or a prodrug thereof, wherein $R_1$ or $R_1$' is each independently hydrogen or an optionally substituted lower alkyl.

7. The compound of any one of claims 1 to 6, or a pharmaceutically acceptable salt, a solvate, or a prodrug thereof, wherein $R_2$ is hydrogen or an optionally substituted lower alkyl.

8. The compound of any one of claims 1 to 7, or a pharmaceutically acceptable salt, a solvate, or a prodrug thereof, wherein $R_3$ or $R_4$ is each independently selected from the group consisting of hydrogen, an optionally substituted lower alkyl, amino, and nitro.

9. The compound of any one of claims 1 to 8, or a pharmaceutically acceptable salt, solvate, or a prodrug thereof, wherein $R_5$ or $R_6$ is each independently hydrogen, or an optionally substituted lower alkyl.

10. A pharmaceutical composition for treating or preventing a disease caused by mitochondrial hyperfission, which comprises the compound of any one of claims 1 to 9, or a pharmaceutically acceptable salt, a solvate, or a prodrug thereof.

11. The pharmaceutical composition of claim 10, wherein the disease caused by mitochondrial hyperfission is chronic

heart failure, amyotrophic lateral sclerosis, neurodegenerative diseases, inflammatory bowel diseases, or diabetes or diabetic complications.

Fig. 1

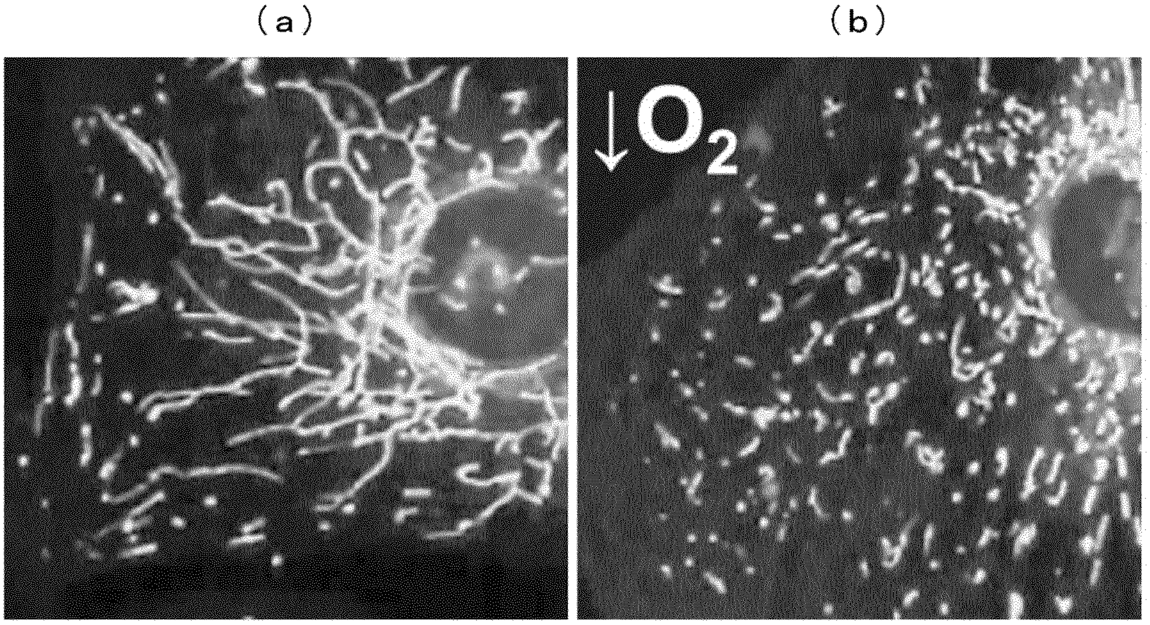

Fig. 2

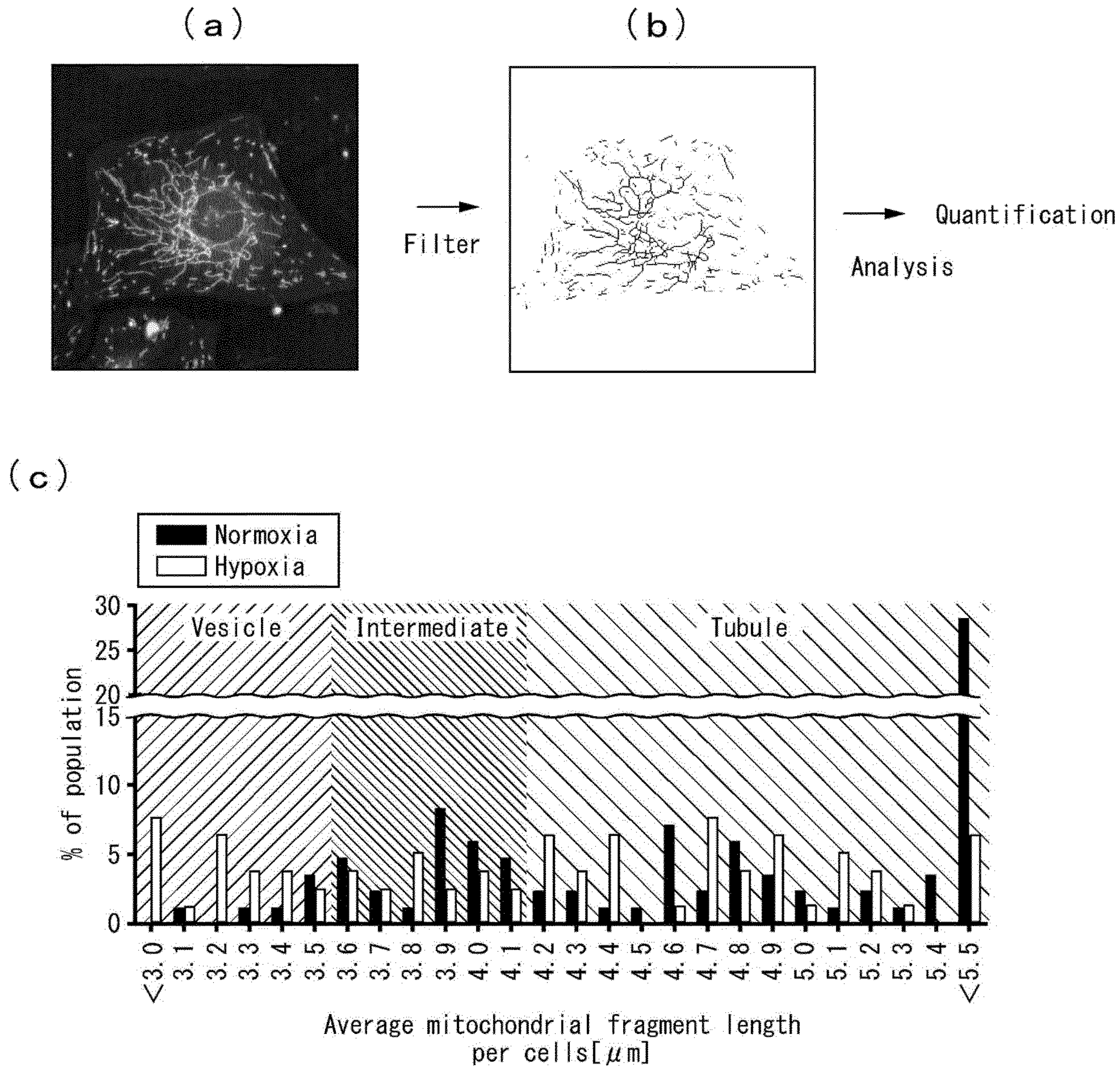
(a)
(b)
Filter
Quantification
Analysis
(c)
Normoxia
Hypoxia
Vesicle
Intermediate
Tubule
% of population
0
5
10
15
20
25
30
<3.0
3.1
3.2
3.3
3.4
3.5
3.6
3.7
3.8
3.9
4.0
4.1
4.2
4.3
4.4
4.5
4.6
4.7
4.8
4.9
5.0
5.1
5.2
5.3
5.4
<5.5
Average mitochondrial fragment length
per cells[μm]

Fig. 3

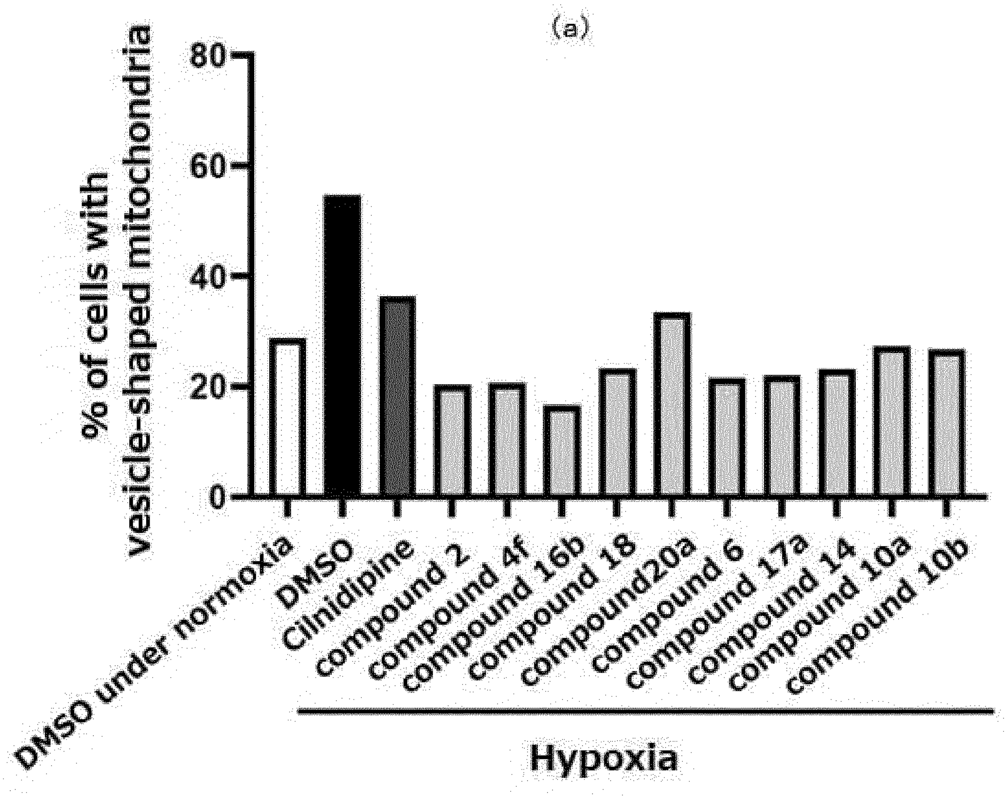

(a)
% of cells with vesicle-shaped mitochondria
80
60
40
20
0
DMSO under normoxia
DMSO
Cilnidipine
compound 2
compound 4f
compound 16b
compound 18
compound20a
compound 6
compound 17a
compound 14
compound 10a
compound 10b
Hypoxia


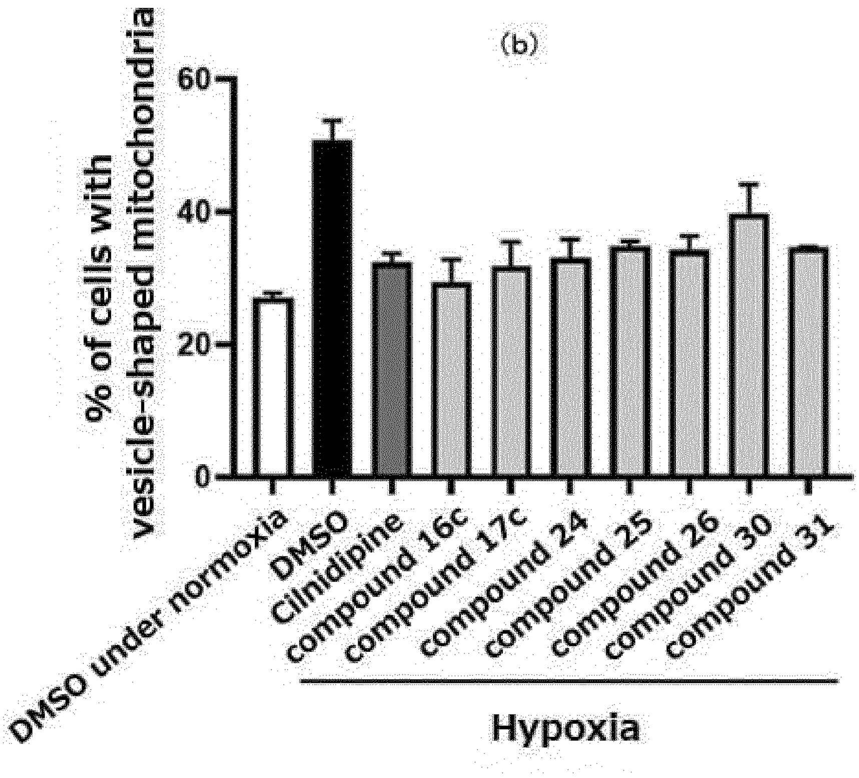

(b)
% of cells with vesicle-shaped mitochondria
60
40
20
0
DMSO under normoxia
DMSO
Cilnidipine
compound 16c
compound 17c
compound 24
compound 25
compound 26
compound 30
compound 31
Hypoxia

Fig. 4

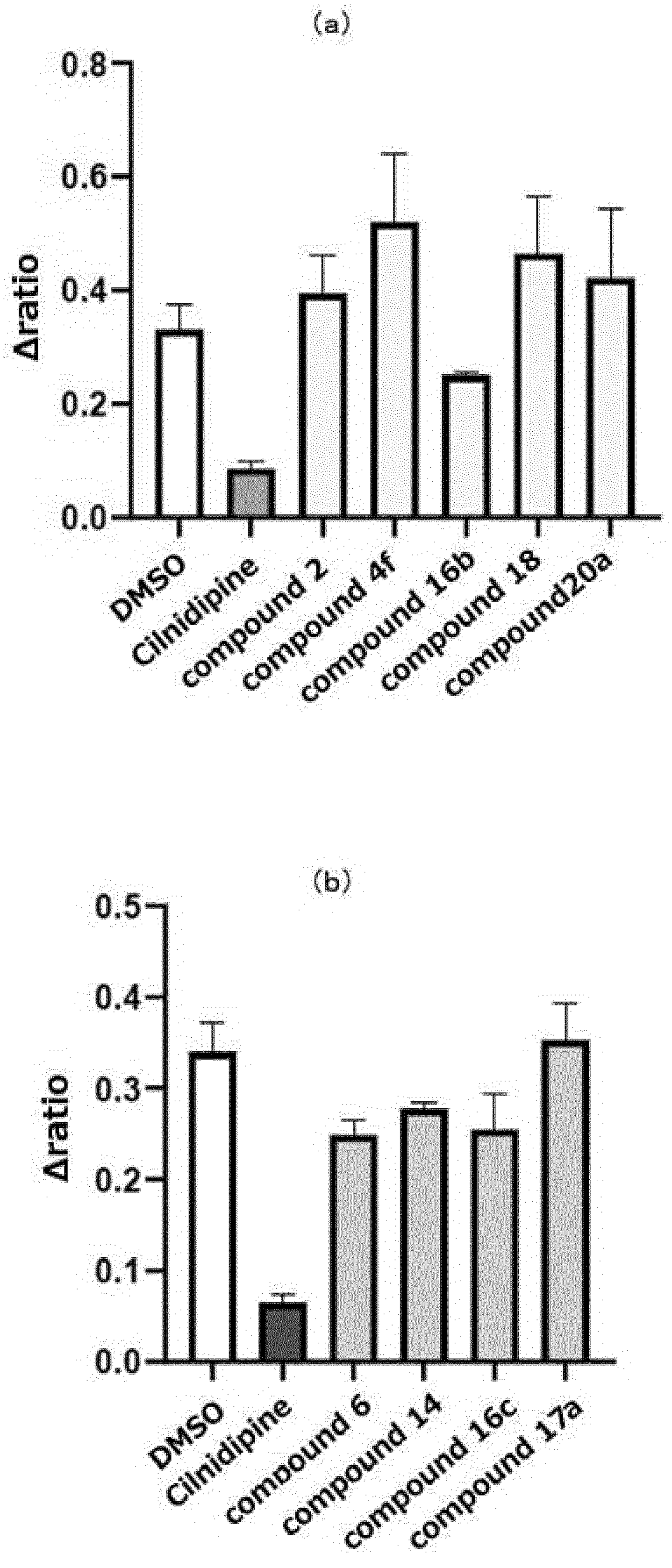
(a)
0.8
0.6
0.4
0.2
0.0
Δratio
DMSO
Cilnidipine
compound 2
compound 4f
compound 16b
compound 18
compound20a
(b)
0.5
0.4
0.3
0.2
0.1
0.0
Δratio
DMSO
Cilnidipine
compound 6
compound 14
compound 16c
compound 17a

Fig. 5

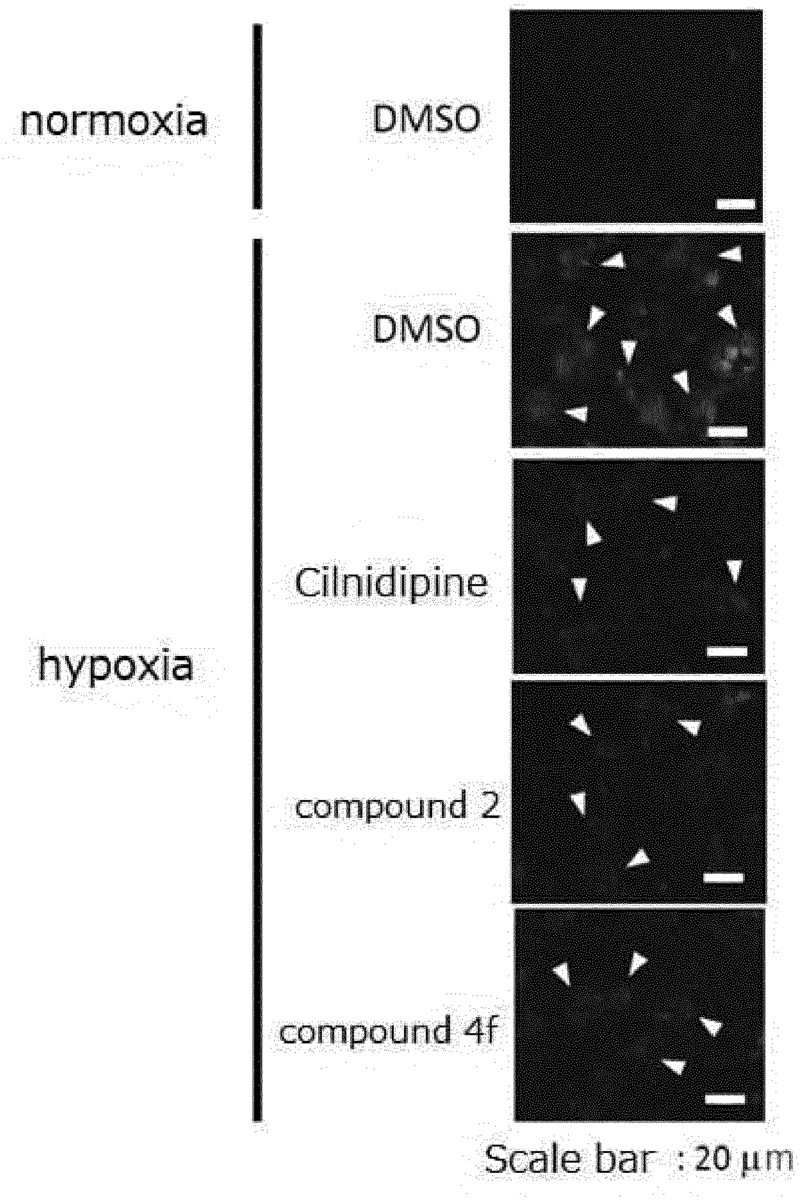
normoxia
DMSO
hypoxia
DMSO
Cilnidipine
compound 2
compound 4f
Scale bar : 20 μm

Fig. 6

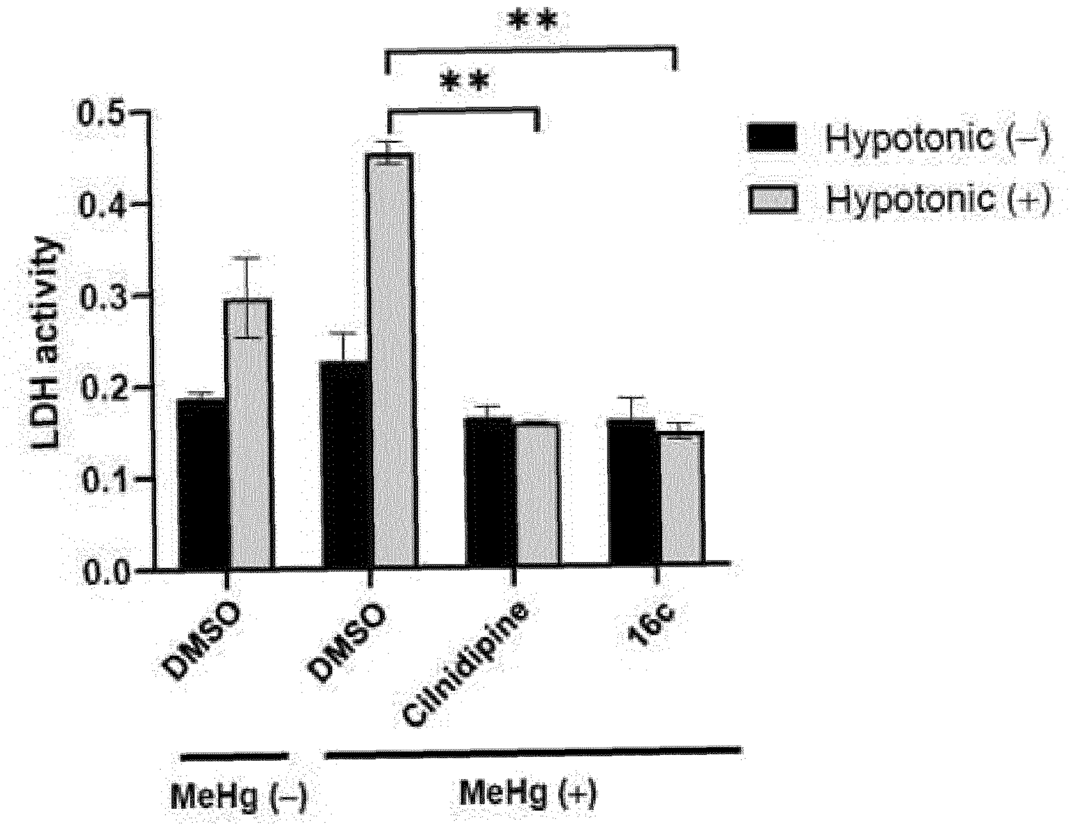

**
**
0.5
0.4
0.3
0.2
0.1
0.0
LDH activity
Hypotonic (–)
Hypotonic (+)
DMSO
DMSO
Cilnidipine
16c
MeHg (–)
MeHg (+)

**INTERNATIONAL SEARCH REPORT**

International application No.
**PCT/JP2021/043654**

**A. CLASSIFICATION OF SUBJECT MATTER**

***A61P 1/04***(2006.01)i; ***A61P 3/10***(2006.01)i; ***A61P 9/04***(2006.01)i; ***A61P 21/00***(2006.01)i; ***A61P 43/00***(2006.01)i; ***C07D 211/90***(2006.01)i; ***C07D 471/04***(2006.01)i; ***C07D 213/80***(2006.01)i; ***C07D 213/82***(2006.01)i; ***A61K 31/4418***(2006.01)i; ***A61K 31/4422***(2006.01)i; ***A61K 31/4745***(2006.01)i
FI: C07D211/90; A61P9/04; A61P21/00; A61P1/04; A61P3/10; A61P43/00 105; A61K31/4422; A61K31/4418; A61K31/4745; C07D213/80; C07D471/04 112A; C07D213/82 CSP

According to International Patent Classification (IPC) or to both national classification and IPC

**B. FIELDS SEARCHED**

Minimum documentation searched (classification system followed by classification symbols)

A61P1/04; A61P3/10; A61P9/04; A61P21/00; A61P43/00; C07D211/90; C07D471/04; C07D213/80; C07D213/82; A61K31/4418; A61K31/4422; A61K31/4745

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2016/080516 A1 (EA PHARMA CO., LTD.) 26 May 2016 (2016-05-26)<br>claims, examples | 1, 6-11<br>2-5 |
| X | CN 107216352 A (INSTITUTE OF RADIATION MEDICINE, CHINESE ACADEMY OF MEDICAL SCIENCES) 29 September 2017 (2017-09-29)<br>claims, example 2 | 1, 6-11 |
| X | WO 99/32446 A1 (AJINOMOTO CO., INC.) 01 July 1999 (1999-07-01)<br>claims, examples | 1, 4, 6-11 |
| X | JP 61-145180 A (BAYER AG) 02 July 1986 (1986-07-02)<br>claims, examples | 1, 4, 7-11 |
| X | JP 5-78319 A (FUJIREBIO INC.) 30 March 1993 (1993-03-30)<br>claims, paragraphs [0004], [0005], examples | 1, 4, 6-9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 January 2022** | **25 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/043654**

**C. DOCUMENTS CONSIDERED TO BE RELEVANT**

| Category* | Citation of document, with indication, where appropriate, of the relevant passages | Relevant to claim No. |
|---|---|---|
| X | US 2011/0118299 A1 (THE UNIVERSITY OF KENTUCKY RESEARCH FOUNDATION) 19 May 2011 (2011-05-19) claims, paragraphs [0138]-[0160], examples | 1-3, 6-11 |
| X | US 4478834 A (USV PHARMACEUTICAL CORP.) 23 October 1984 (1984-10-23) example | 1-3, 6-9 |
| X | LUO, L. et al, Synthesis of benzo[c][2,7]naphthyridine-6-ones via cascade aromatization/ C(sp2)-H amidation of 1,4-dihydropyridines, Tetrahedron Letters, 2017, vol. 58, no. 28, p. 2792-2795 schemes 1, 2, 3 | 1, 2, 4, 7-9 |
| X | GOERLITZER, K. et al, 9-Chloro-3,6-diazaphenanthrenes from nifedipine, Archiv der Pharmazie (Weinheim, Germany), 1985, vol. 318, no. 2, pp. 106-110 Compounds 1a, 1b, 4a, 4b | 1, 2, 6-9 |
| X | KAMETANI, T. et al, Syntheses of heterocyclic compounds. CCXCIX. Nitrene. 5. Reaction of 4-(2-nitrophenyl)-1,4-dihydropyridine derivatives with triethyl phosphite, Journal of the Chemical Society [Section] C: Organic, 1969, no. 12, pp. 1616-1619 Compound (IX) | 1, 2, 6, 7, 9 |
| X | KAMETANI,T. et al, Nitrenes. III. Reaction of 4-(2-nitrophenyl)pyridine derivatives with triethyl phosphite, Journal of the Chemical Society [Section] C: Organic, 1969, no. 1, pp. 138-140 compound (IX) | 1, 2, 6, 7, 9 |
| P, X | WO 2020/241638 A1 (KYUSHU UNIVERSITY, NATIONAL UNIVERSITY CORPORATION) 03 December 2020 (2020-12-03) claims, example | 1, 6-11 |

Form PCT/ISA/210 (second sheet) (January 2015)

**INTERNATIONAL SEARCH REPORT**
**Information on patent family members**

International application No.
**PCT/JP2021/043654**

| Patent document cited in search report | Publication date (day/month/year) | Patent family member(s) | Publication date (day/month/year) |
|---|---|---|---|
| WO 2016/080516 A1 | 26 May 2016 | (Family: none) | |
| CN 107216352 A | 29 September 2017 | (Family: none) | |
| WO 99/32446 A1 | 01 July 1999 | US 6350762 B1<br>claims, examples<br>EP 1043314 A1<br>KR 10-2001-0033442 A<br>CN 1283187 A | |
| JP 61-145180 A | 02 July 1986 | US 4707479 A<br>claims, examples<br>EP 185964 A2 | |
| JP 5-78319 A | 30 March 1993 | US 5367081 A<br>claims, column 1, line 44 to column 2, line 7, examples<br>WO 1984/002721 A1<br>EP 488345 A1<br>KR 10-1994-0005016 B | |
| US 2011/0118299 A1 | 19 May 2011 | JP 2013-526518 A<br>WO 2010/132671 A1<br>EP 2429992 A1<br>CN 102984938 A | |
| US 4478834 A | 23 October 1984 | (Family: none) | |
| WO 2020/241638 A1 | 03 December 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016080516 A
- WO 2020241638 A
- JP 4613496 B
- JP 2008044871 A
- WO 2013147137 A
- JP 2004359675 A
- WO 2008016171 A
- JP 2013014549 A


### Non-patent literature cited in the description


- **SONG, M ; DORN, G.W. 2ND.** Mitoconfusion: Non-canonical functioning of dynamism factors in static mitochondria of the heart. *Cell Metab,* 2015, vol. 21
- **DORN, G.W. 2ND.** Mitochondrial fission/fusion and cardiomyopathy. *Curr. Opin. Genet. Dev,* 2016, vol. 38, 38-44
- **HAGIR B SULIMAN ; CLAUDE A PIANTADOSI.** Mitochondrial Quality Control as a Therapeutic Target. *Pharmacol Rev,* January 2016, vol. 68 (1), 20-48
- **PICCA A ; MANKOWSKI RT ; BURMAN JL ; DONISI L ; KIM JS ; MARZETTI E ; LEEUWENBURGH C.** Mitochondrial quality control mechanisms as molecular targets in cardiac ageing. *Nat Rev Cardiol,* September 2018, vol. 15 (9), 543-554
- **AKIYUKI NISHIMURA 1 ; MOTOHIRO NISHIDA.** *Seikagaku,* 2019, vol. 91 (4), 519-522
- **NISHIMURA A et al.** Hypoxia-induced interaction of filamin with Drp1 causes mitochondrial hyperfission-associated myocardial aging. *Sci. Signal.,* 2018, vol. 11 (556), 5185
- **NAOYA SHINDO ; AKIO OJIDA.** Handbook of In Vivo Chemistry in Mice. From Lab to Living System. Wiley-VCH, 2020, 281-307
- **FANDI SUTANTO ; MARKELLA KONSTANTINIDOU ; ALEXANDER DOEMLING.** Covalent inhibitors: a rational approach to drug discovery. *RSC Medicinal Chemistry,* 2020, vol. 11, 876-884
- **KROGSGAARD-LARSEN et al.** Textbook of Drug design and Discovery. Taylor & Francis, April 2002